(19) Europäisches Patentamt
European Patent Office
Office européen des brevets

(11) **EP 3 521 448 A1**

(12) **EUROPEAN PATENT APPLICATION**
published in accordance with Art. 153(4) EPC

(43) Date of publication:
**07.08.2019 Bulletin 2019/32**

(21) Application number: **17856310.2**

(22) Date of filing: **28.09.2017**

(51) Int Cl.:
*C12Q 1/68* (2018.01)　　　*C12N 15/09* (2006.01)

(86) International application number:
**PCT/JP2017/035137**

(87) International publication number:
**WO 2018/062361 (05.04.2018 Gazette 2018/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.09.2016 PCT/JP2016/078810**
**31.03.2017 JP 2017072674**

(71) Applicants:
• **Hanumat Co., Ltd.**
**Kobe-shi, Hyogo 657-0025 (JP)**
• **EA Pharma Co., Ltd.**
**Tokyo 104-0042 (JP)**

(72) Inventors:
• **KUSUNOKI Masato**
**Kobe-shi**
**Hyogo 657-0025 (JP)**
• **TOIYAMA Yuji**
**Tsu-shi**
**Mie 514-8507 (JP)**
• **MITSUI Akira**
**Kawasaki-shi**
**Kanagawa 210-8681 (JP)**
• **TAKEHANA Kenji**
**Tokyo 104-0042 (JP)**
• **UMEZAWA Tsutomu**
**Tokyo 104-0042 (JP)**

(74) Representative: **Mewburn Ellis LLP**
**City Tower**
**40 Basinghall Street**
**London EC2V 5DE (GB)**

(54) **METHOD FOR DETERMINING ONSET RISK OF SPORADIC COLON CANCER**

(57)　The present invention provides a method for determining the likelihood of sporadic colorectal cancer development, the method including: a measurement step of measuring methylation rates of one or more CpG sites present in specific differentially methylated regions, in DNA recovered from a biological sample collected from a human subject; and a determination step of determining the likelihood of sporadic colorectal cancer development in the human subject, based on average methylation rates of the differentially methylated regions which are calculated based on the methylation rates measured and a preset reference value or a preset multivariate discrimination expression, in which the reference value is a value for identifying a sporadic colorectal cancer patient and a non-sporadic colorectal cancer patient, which is set for the average methylation rate of each differentially methylated region, and the multivariate discrimination expression includes, as variables, average methylation rates of one or more differentially methylated regions among the specific differentially methylated regions.

**Description**

Technical Field

The present invention relates to a method for determining the likelihood of sporadic colorectal cancer development in a human subject who does not have subjective symptoms of a large intestinal disease.

Priority is claimed on PCT International Application No. PCT/JP2016/078810, filed on September 29, 2016, and Japanese Patent Application No. 2017-072674, filed on March 31, 2017, the contents of which are incorporated herein by reference.

Background Art

Colorectal cancer has a high cure rate if properly treated at an early stage. However, there are often no subjective symptoms in an early stage. Thus, it is preferable to have a regular medical examination or the like to enable early detection. For colorectal cancer examination, a fecal occult blood examination is widely conducted. Due to using feces as a sample, the fecal occult blood examination is excellent from the viewpoint of being non-invasive. However, there is a problem in that it is not possible to distinguish colorectal cancer from other diseases, in which blood is mixed in feces, such as bacterial or viral enteritis, diverticular bleeding, and anal disease (hemorrhoids, anal fistula, or anal fissure).

As an examination for making a more accurate determination by distinguishing colorectal cancer from other diseases that become positive by the fecal occult blood examination, there is an endoscopic examination. However, detecting colorectal cancer at an early stage by visual recognition depends largely on an operator's skill and it is generally difficult to do so. In addition, the endoscopic examination has problems of being highly invasive and of also being a heavy burden on a subject.

As a method for achieving early detection of colorectal cancer which has developed in large intestinal mucosa and is based on ulcerative colitis in a more non-invasive manner than endoscopic examination, there is a method using DNA methylation as a biomarker. For example, PTL 1 reports that in ulcerative colitis patients, a methylation rate of five miRNA genes of miR-1, miR-9, miR-124, miR-137, and miR-34b/c in tumorous tissue is significantly higher than in non-tumorous ulcerative colitis tissue, and the methylation rate of the five miRNA genes in a biological sample collected from rectal mucosa which is a non-cancerous part can also be used as a marker for colorectal cancer development in ulcerative colitis patients.

Citation List

Patent Literature

[PTL 1] PCT International Publication No. WO 2014/151551

Summary of Invention

Problem to be solved by the Invention

An object of the present invention is to provide a method for determining the likelihood of sporadic colorectal cancer development in a human subject who does not have subjective symptoms of a large intestinal disease by a method which is less invasive than an endoscopic examination and places less burden on a subject.

Means to solve the Problem

As a result of intensive studies to solve the above problems, the present inventors comprehensively investigated methylation rates of CpG (cytosine-phosphodiester bond-guanine) sites in genomic DNAs of human subjects who do not have subjective symptoms of a large intestinal disease, and found 93 CpG sites with markedly different methylation rates in patients who had developed colorectal cancer and human subjects who had not developed sporadic colorectal cancer. In addition, the present inventors separately found 121 differentially methylated regions (referred to as "DMR" in some cases), and completed the present invention.

That is, the present invention provides the following [1] to [29], namely a method for determining the likelihood of sporadic colorectal cancer development, a marker for analyzing a DNA methylation rate, and a kit for collecting large intestinal mucosa.

[1] A method for determining the likelihood of sporadic colorectal cancer development, the method including:

a measurement step of measuring methylation rates of one or more CpG sites present in respective differentially methylated regions represented by differentially methylated region numbers 1 to 121 listed in Tables 1 to 7, in DNA recovered from a biological sample collected from a human subject; and

a determination step of determining the likelihood of sporadic colorectal cancer development in the human subject, based on average methylation rates of the differentially methylated regions which are calculated based on the methylation rates measured in the measurement step and a preset reference value or a preset multivariate discrimination expression,

in which the average methylation rate of the differentially methylated region is an average value of methylation rates of all CpG sites, for which the methylation rate is measured in the measurement step, among the CpG sites in the differentially methylated region,

the reference value is a value for identifying a sporadic colorectal cancer patient and a non-sporadic colorectal cancer patient, which is set for the average methylation rate of each differentially methylated region, and

the multivariate discrimination expression includes, as variables, average methylation rates of one or more differentially methylated regions among the differentially methylated regions represented by the differentially methylated region numbers 1 to 121.

[Table 1]

| DMR no. | Gene Symbol | Ensembl ID | Chromosome no. | DMR start | DMR end | Width | +I |
|---|---|---|---|---|---|---|---|
| 1 | | | 17 | 46827397 | 46827628 | 232 | + |
| 2 | | ENST00000561259.1 | 15 | 37180595 | 37181182 | 588 | + |
| 3 | FADS2 | | 11 | 61596200 | 61596511 | 312 | + |
| 4 | SHF | ENST00000560734.1;ENST00000560471.1; ENST00000560540.1;ENST00000561091.1; ENST00000560034.1 | 15 | 45479648 | 45479861 | 214 | + |
| 5 | TDH | ENST00000525867.1;ENST00000534302.1 | 8 | 11203722 | 11205353 | 1632 | + |
| 6 | MYF6 | ENST00000228641.3 | 12 | 81102475 | 81103021 | 547 | + |
| 7 | SOX21; SOX21-AS1 | ENST00000438290.1; ENST00000376945.2 | 13 | 95364512 | 95364619 | 108 | + |
| 8 | RANBP9 | ENST00000469916.1 | 6 | 13633257 | 13635423 | 2167 | − |
| 9 | | ENST00000390750.1 | 1 | 97366188 | 97369696 | 3509 | − |
| 10 | EHBP1 | ENST00000516627.1 | 2 | 62953601 | 62956283 | 2683 | − |
| 11 | HECTD1 | ENST00000384709.1 | 14 | 31610929 | 31613066 | 2138 | − |
| 12 | | ENST00000440936.1 | 11 | 27911088 | 27914543 | 3456 | − |
| 13 | ASH1L | ENST00000384405.1 | 1 | 155327687 | 155330111 | 2425 | − |
| 14 | | ENST00000401135.1 | 11 | 112115998 | 112119870 | 3873 | − |
| 15 | | ENST00000562976.1 | 16 | 32609347 | 32612783 | 3437 | − |
| 16 | HOXA2 | ENST00000222718.5 | 7 | 27142503 | 27143294 | 792 | + |
| 17 | GNAL | ENST00000535121.1;ENST00000269162.4; ENST00000423027.2;ENST00000540217.1 | 18 | 11751996 | 11752178 | 183 | + |
| 18 | ARHGEF4 | ENST00000428230.2;ENST00000525839.1; ENST00000326016.5 | 2 | 131674106 | 131674191 | 86 | + |
| 19 | PCDHA7; PCDHA12; PCDHA6; PCDHAC1; PCDHA10; PCDHA4; PCDHA11; PCDHA8; PCDHA1; PCDHA2; PCDHA9; PCDHA13; PCDHA5; PCDHA3 | ENST00000253807.2; ENST00000409700.3 | 5 | 140306074 | 140306355 | 282 | + |
| 20 | FLJ45983 | ENST00000458727.1; ENST00000355358.1; ENST00000418270.1 | 10 | 8094324 | 8094640 | 317 | + |

[Table 2]

| DMR no. | Gene Symbol | Ensembl ID | Chromosome no. | DMR start | DMR end | Width | +/- |
|---|---|---|---|---|---|---|---|
| 21 | ATF7IP2 | ENST00000396559.1;ENST00000561932.1; ENST00000543967.1 | 16 | 10479725 | 10480582 | 858 | + |
| 22 | | | 11 | 20617680 | 20618294 | 615 | + |
| 23 | DMRTA2 | ENST00000418121.1 | 1 | 50886813 | 50887075 | 263 | + |
| 24 | SEPT9 | ENST00000363781.1;ENST00000397613.4 | 17 | 75436513 | 75439186 | 2674 | + |
| 25 | TNFRSF25; PLEKHG5 | ENST00000348333.3;ENST00000377782.3; ENST00000356876.3;ENST00000400913.1; ENST00000489097.1 | 1 | 6525942 | 6526668 | 727 | + |
| 26 | FLJ32063 | ENST00000450728.1;ENST00000416200.1; ENST00000446911.1;ENST00000457245.1; ENST00000441234.1 | 2 | 200334170 | 200335332 | 1163 | + |
| 27 | DTX1 | ENST00000257600.3 | 12 | 113494374 | 113494471 | 98 | + |
| 28 | LYNX1 | ENST00000522906.1;ENST00000398906.1; ENST00000395192.2;ENST00000335822.5; ENST00000523332.1;ENST00000345173.6 | 8 | 143858547 | 143858706 | 160 | + |
| 29 | IZUMO1 | ENST00000332955.2 | 19 | 49250305 | 49250694 | 390 | + |
| 30 | | | 18 | 55095061 | 55095364 | 304 | + |
| 31 | AEBP2 | ENST00000360995.4;ENST00000541908.1 | 12 | 19593346 | 19593565 | 220 | + |
| 32 | | ENST00000406197.1 | 7 | 155284154 | 155284741 | 588 | + |
| 33 | ZNF542 | ENST00000490123.1 | 19 | 56879271 | 56879751 | 481 | + |
| 34 | LRRC43 | | 12 | 122651566 | 122651863 | 298 | + |
| 35 | ERCC6 | ENST00000374129.3;ENST00000539110.1; ENST00000542458.1 | 10 | 50696150 | 50698147 | 1998 | − |
| 36 | ACSM3 | ENST00000289416.5;ENST00000440284.2; ENST00000565498.1 | 16 | 20777186 | 20779229 | 2044 | − |
| 37 | WAPAL | ENST00000372075.1;ENST00000263070.7 | 10 | 88226215 | 88229444 | 3230 | − |
| 38 | HLA−E | ENST00000376630.4 | 6 | 30455709 | 30456000 | 292 | − |
| 39 | | ENST00000459557.1 | 6 | 114159118 | 114163406 | 4289 | − |
| 40 | | ENST00000486767.1 | 3 | 164402447 | 164406668 | 4222 | − |

[Table 3]

| DMR no. | Gene Symbol | Ensembl ID | Chromosome no. | DMR start | DMR end | Width | ± |
|---|---|---|---|---|---|---|---|
| 41 | BET1 | ENST00000471446.1;ENST00000426193.2;ENST00000426634.1 | 7 | 93625930 | 93628057 | 2128 | − |
| 42 | | | 6 | 14406829 | 14409842 | 3014 | − |
| 43 | ZNF323; ZKSCAN3 | ENST00000252211.2;ENST00000341464.5;ENST00000396838.2;ENST00000414429.1 | 6 | 28320486 | 28323328 | 2843 | − |
| 44 | MTMR3 | ENST00000384724.1;ENST00000401950.2;ENST00000333027.3;ENST00000323630.5;ENST00000351488.3;ENST00000415511.1 | 22 | 30295038 | 30296772 | 1735 | − |
| 45 | SH3YL1 | ENST00000403657.1;ENST00000468321.1;ENST00000403658.1 | 2 | 252349 | 255227 | 2879 | − |
| 46 | | ENST00000455502.1 | 7 | 93472562 | 93475664 | 3103 | − |
| 47 | | ENST00000555070.1 | 14 | 90167165 | 90167752 | 588 | − |
| 48 | | | 8 | 1404844 | 1405431 | 588 | − |
| 49 | TFDP2 | ENST00000383877.1;ENST00000489671.1;ENST00000464782.1;ENST00000317104.7;ENST00000467072.1;ENST00000499676.2 | 3 | 141863017 | 141865101 | 2085 | − |
| 50 | TMEM106B | | 7 | 12268344 | 12270783 | 2440 | − |
| 51 | | ENST00000364882.1 | 4 | 117758275 | 117761934 | 3660 | − |
| 52 | SLC20A2 | ENST00000520262.1;ENST00000520179.1;ENST00000342228.3 | 8 | 42357666 | 42360957 | 3292 | − |
| 53 | | | 1 | 47910065 | 47911801 | 1737 | + |
| 54 | STK32B | ENST00000282908.5 | 4 | 5053444 | 5053551 | 108 | + |
| 55 | SOX2OT; SOX2 | ENST00000498731.1;ENST00000431565.2;ENST00000325404.1 | 3 | 181427354 | 181428928 | 1575 | + |
| 56 | SOX2OT | ENST00000498731.1 | 3 | 181437890 | 181438559 | 670 | + |
| 57 | CLIP4 | ENST00000320081.5;ENST00000379543.5;ENST00000401605.1;ENST00000401617.2;ENST00000404424.1 | 2 | 29337848 | 29338142 | 295 | + |

[Table 4]

| DMR no. | Gene Symbol | Ensembl ID | Chromosome no. | DMR start | DMR end | Width | ± |
|---|---|---|---|---|---|---|---|
| 58 | | | 5 | 2038695 | 2039282 | 588 | + |
| 59 | SHISA9 | ENST00000423335.2;ENST00000482916.1;ENST00000558318.1;ENST00000424107.3 | 16 | 12995279 | 12995656 | 378 | + |
| 60 | | ENST00000364275.1 | 4 | 190938593 | 190938935 | 343 | + |
| 61 | | | 16 | 73096548 | 73097135 | 588 | + |
| 62 | TTYH1 | ENST00000391739.3;ENST00000376531.3;ENST00000301194.4;ENST00000376530.3 | 19 | 54926333 | 54927197 | 865 | + |
| 63 | PHACTR1 | ENST00000379350.1;ENST00000399446.2;ENST00000334971.6 | 6 | 13273152 | 13275352 | 2201 | + |
| 64 | DAB1 | ENST00000371236.1;ENST00000371234.4;ENST00000485760.1 | 1 | 58715419 | 58715632 | 214 | + |
| 65 | | ENST00000558382.1;ENST00000558499.1 | 15 | 96905928 | 96910011 | 4084 | + |
| 66 | ZNF382; ZNF529 | ENST00000423582.1;ENST00000460670.1;ENST00000292928.2;ENST00000439428.1 | 19 | 37096052 | 37096201 | 150 | + |
| 67 | SOX2OT; SOX2-OT | ENST00000498731.1 | 3 | 181440653 | 181444202 | 3550 | + |
| 68 | CPEB1; CPEB1-AS1 | ENST00000560650.1;ENST00000450751.2;ENST00000568757.1;ENST00000563519.1 | 15 | 83316116 | 83316484 | 369 | + |
| 69 | EVC2 | ENST00000344938.1;ENST00000310917.2 | 4 | 5710239 | 5710490 | 252 | + |
| 70 | C2orf74 | ENST00000426997.1;ENST00000420918.1 | 2 | 61372150 | 61372361 | 212 | + |
| 71 | DPYSL3 | ENST00000343218.5;ENST00000504965.1 | 5 | 146889149 | 146889390 | 242 | + |
| 72 | PENK; LOC101929415 | ENST00000518662.1;ENST00000523274.1;ENST00000523051.1;ENST00000518770.1;ENST00000539312.1;ENST00000451791.2;ENST00000314922.3 | 8 | 57358624 | 57358800 | 177 | + |

[Table 5]

| DMR no. | Gene Symbol | Ensembl ID | Chromosome no. | DMR start | DMR end | Width | +I |
|---|---|---|---|---|---|---|---|
| 73 | GJD2; LOC101928174 | ENST00000503496.1;ENST00000290374.4 | 15 | 35047146 | 35047453 | 308 | + |
| 74 | ADAMTS16 | ENST00000512155.1;ENST00000511368.1 | 5 | 5139810 | 5139920 | 111 | + |
| 75 | FAM159B | ENST00000512767.1 | 5 | 63986626 | 63986899 | 274 | + |
| 76 | KCNA4 | ENST00000526518.1;ENST00000328224.6 | 11 | 30038649 | 30038734 | 86 | + |
| 77 | IRX5 | ENST00000447390.2;ENST00000560487.1; ENST00000560154.1;ENST00000558597.1; ENST00000394636.4 | 16 | 54967579 | 54969439 | 1861 | + |
| 78 | BCAT1 | ENST00000538118.1;ENST00000544418.1; ENST00000539282.1 | 12 | 25055964 | 25056233 | 270 | + |
| 79 | SOX11 | ENST00000322002.3;ENST00000455579.1 | 2 | 5836177 | 5836284 | 108 | + |
| 80 | CHL1 | ENST00000452919.1;ENST00000444879.1; ENST00000489224.1;ENST00000256509.2; ENST00000397491.2 | 3 | 239108 | 239308 | 201 | + |
| 81 | FAM115A; TCAF1 | ENST00000392900.3;ENST00000355951.2; ENST00000479870.1 | 7 | 143578766 | 143581048 | 2283 | + |
| 82 | | ENST00000551875.1 | 12 | 115172454 | 115173299 | 846 | + |
| 83 | | | 17 | 46831196 | 46831783 | 588 | + |
| 84 | NR5A2 | | 1 | 200003863 | 200004690 | 828 | + |
| 85 | UTF1 | ENST00000304477.2 | 10 | 135043449 | 135043550 | 102 | + |
| 86 | ATP10A | ENST00000553577.1;ENST00000356865.6 | 15 | 26107150 | 26108725 | 1576 | + |
| 87 | LOC283999; TMEM235 | ENST00000374946.3;ENST00000550981.2 | 17 | 76227764 | 76228227 | 464 | + |
| 88 | ZNF177 | ENST00000343499.3;ENST00000541595.1; ENST00000446085.2 | 19 | 9473642 | 9473768 | 127 | + |
| 89 | | | 6 | 107809023 | 107809834 | 812 | + |
| 90 | NR2E1 | ENST00000368986.4 | 6 | 108492410 | 108493000 | 591 | + |
| 91 | CDO1 | ENST00000250535.4;ENST00000502631.1 | 5 | 115152332 | 115152439 | 108 | + |
| 92 | CASR | ENST00000498619.1;ENST00000490131.1 | 3 | 121902936 | 121903190 | 255 | + |

[Table 6]

| DMR no. | Gene Symbol | Ensembl ID | Chromosome no. | DMR start | DMR end | Width | +l |
|---|---|---|---|---|---|---|---|
| 93 | PCDHGA4; PCDHGA11; PCDHGA9; PCDHGA1; PCDHGB1; PCDHGB6; PCDHGA12; PCDHGB3; PCDHGB7; PCDHGA6; PCDHGA8; PCDHGA10; PCDHGA5; PCDHGB4; PCDHGA3; PCDHGA2; PCDHGB2; PCDHGA7; PCDHGB5 | ENST00000252085.3 | 5 | 140809819 | 140810664 | 846 | + |
| 94 | OCA2 | ENST00000353809.5;ENST00000354638.3 | 15 | 28344617 | 28344827 | 211 | + |
| 95 | LINC01248; SOX11 | ENST00000420221.1;ENST00000453678.1; ENST00000458264.1;ENST00000322002.3 | 2 | 5830853 | 5831440 | 588 | + |
| 96 | GDF7 | ENST00000272224.3 | 2 | 20871066 | 20871694 | 629 | + |
| 97 | SOX8 | ENST00000562570.1;ENST00000568394.1; ENST00000565467.1;ENST00000563863.1; ENST00000565069.1;ENST00000563837.1; ENST00000293894.3 | 16 | 1030543 | 1030628 | 86 | + |
| 98 | NEFM | ENST00000221166.5;ENST00000433454.2; ENST00000518131.1;ENST00000521540.1 | 8 | 24771213 | 24771326 | 114 | + |
| 99 | | ENST00000560487.1 | 16 | 54970835 | 54971133 | 299 | + |
| 100 | PTGFRN | ENST00000544471.1;ENST00000393203.2 | 1 | 117528415 | 117531212 | 2798 | + |
| 101 | STAC | ENST00000273183.3;ENST00000457375.2; ENST00000476388.1;ENST00000544687.1 | 3 | 36422165 | 36422637 | 473 | + |
| 102 | | | 12 | 81106709 | 81109314 | 2606 | + |
| 103 | HBQ1 | ENST00000199708.2 | 16 | 230287 | 230396 | 110 | + |
| 104 | | | 6 | 85484569 | 85485156 | 588 | + |

[Table 7]

| DMR no. | Gene Symbol | Ensembl ID | Chromosome no. | DMR start | DMR end | Width | +/- |
|---|---|---|---|---|---|---|---|
| 105 | NPR3 | ENST00000434067.2;ENST00000415685.2 | 5 | 32708777 | 32709689 | 913 | + |
| 106 | NMBR | ENST00000258042.1;ENST00000454401.1 | 6 | 142410081 | 142410276 | 196 | + |
| 107 | KCNIP1 | ENST00000411494.1;ENST00000328939.4; ENST00000390656.4;ENST00000520740.1 | 5 | 169931309 | 169931416 | 108 | + |
| 108 | ZNF835 | ENST00000537055.1 | 19 | 57183011 | 57183374 | 364 | + |
| 109 | SALL3 | ENST00000575722.1;ENST00000573860.1; ENST00000537592.2 | 18 | 76740075 | 76740337 | 263 | + |
| 110 | CCNA1 | ENST00000418263.1;ENST00000255465.4; ENST00000440264.1 | 13 | 37006053 | 37006793 | 741 | + |
| 111 | NR3C1 | ENST00000504336.1;ENST00000416954.2 | 5 | 142768792 | 142771780 | 2989 | − |
| 112 | STX19; ARL13B | ENST00000315099.2;ENST00000539730.1; ENST00000486562.1 | 3 | 93746411 | 93748870 | 2460 | − |
| 113 | NFIB | ENST00000493697.1 | 9 | 14307151 | 14309148 | 1998 | − |
| 114 | | ENST00000510419.1 | 4 | 75513579 | 75517080 | 3502 | − |
| 115 | TRIM9 | ENST00000554475.1 | 14 | 51554159 | 51556518 | 2360 | − |
| 116 | PIBF1 | ENST00000362511.1 | 13 | 73455494 | 73457491 | 1998 | − |
| 117 | | ENST00000468232.1 | 3 | 170126475 | 170129488 | 3014 | − |
| 118 | LOC101060498 | ENST00000510551.1 | 4 | 40316101 | 40318304 | 2204 | − |
| 119 | RNU6−2 | ENST00000384716.1 | 10 | 13257430 | 13260736 | 3307 | − |
| 120 | EFNB2 | | 13 | 107181847 | 107183783 | 1937 | − |
| 121 | ARG1 | ENST00000368087.3;ENST00000356962.2; ENST00000476845.1;ENST00000489091.1 | 6 | 131893339 | 131893636 | 298 | − |

[2] The method for determining the likelihood of sporadic colorectal cancer development according to [1], in which in the measurement step, in a case where one or more among the differentially methylated regions represented by differentially methylated region numbers 8 to 15, 35 to 52, and 111 to 121 have an average methylation rate of equal to or lower than the preset reference value, or one or more among the differentially methylated regions represented by differentially methylated region numbers 1 to 7, 16 to 34, and 53 to 110 have an average methylation rate of equal to or higher than the preset reference value, it is determined that there is a high likelihood of sporadic colorectal cancer development in the human subject.

[3] The method for determining the likelihood of sporadic colorectal cancer development according to [1], in which in the measurement step, the methylation rates of the one or more CpG sites present in the differentially methylated region, of which an average methylation rate is included as a variable in the multivariate discrimination expression, are measured, and

in the determination step, in a case where based on the average methylation rate of the differentially methylated region calculated based on the methylation rates measured in the measurement step, and the multivariate discrimination expression, a discrimination value which is a value of the multivariate discrimination expression is calculated, and the discrimination value is equal to or higher than a preset reference discrimination value, it is determined that there is a high likelihood of sporadic colorectal cancer development in the human subject.

[4] The method for determining the likelihood of sporadic colorectal cancer development according to [3], in which the multivariate discrimination expression includes, as variables, average methylation rates of two or more differentially methylated regions selected from the differentially methylated regions represented by the differentially methylated region numbers 1 to 121.

[5] The method for determining the likelihood of sporadic colorectal cancer development according to [3], in which the multivariate discrimination expression includes, as variables, average methylation rates of three or more differentially methylated regions selected from the differentially methylated regions represented by the differentially methylated region numbers 1 to 121.

[6] The method for determining the likelihood of sporadic colorectal cancer development according to [3], in which the multivariate discrimination expression includes, as variables, average methylation rates of one or more differentially methylated regions selected from the group consisting of the differentially methylated regions represented by the differentially methylated region numbers 1 to 52.

[7] The method for determining the likelihood of sporadic colorectal cancer development according to [3], in which the multivariate discrimination expression includes, as variables, average methylation rates of one or more differentially methylated regions selected from the group consisting of the differentially methylated regions represented by the differentially methylated region numbers 1 to 15.

[8] A method for determining the likelihood of sporadic colorectal cancer development, the method including:

a measurement step of measuring methylation rates of one or more CpG sites selected from the group consisting of CpG sites in base sequences represented by SEQ ID NOs: 1 to 93, in DNA recovered from a biological sample collected from a human subject; and

a determination step of determining the likelihood of sporadic colorectal cancer development in the human subject, based on the methylation rates measured in the measurement step and a preset reference value or a preset multivariate discrimination expression,

in which the reference value is a value for identifying a sporadic colorectal cancer patient and a non-sporadic colorectal cancer patient, which is set for the methylation rate of each CpG site, and

the multivariate discrimination expression includes, as variables, methylation rates of one or more CpG sites among the CpG sites in the base sequences represented by SEQ ID NOs: 1 to 93.

[9] The method for determining the likelihood of sporadic colorectal cancer development according to [8], in which in the measurement step, methylation rates of 2 to 10 CpG sites are measured.

[10] The method for determining the likelihood of sporadic colorectal cancer development according to [8] or [9], in which in the determination step, in a case where at least one among CpG sites in the base sequences represented by SEQ ID NOs: 1, 4, 6, 10, 11, 13, 14, 17 to 20, 23 to 27, 29, 30, 32, 33, 35, 36, 39, 41 to 48, 50 to 54, 59, 65 to 68, 70 to 77, 79 to 86, 90, and 91 has a methylation rate of equal to or lower than the preset reference value, or at least one among CpG sites in the base sequences represented by SEQ ID NOs: 2, 3, 5, 7 to 9, 12, 15, 16, 21, 22, 28, 31, 34, 37, 38, 40, 49, 55 to 58, 60 to 64, 69, 78, 87 to 89, 92, and 93 has a methylation rate of equal to or higher than the preset reference value, it is determined that there is a high likelihood of sporadic colorectal cancer development in the human subjuect.

[11] The method for determining the likelihood of sporadic colorectal cancer development according to any one of [8] to [10],

in which in the measurement step, methylation rates of CpG sites in the base sequences represented by SEQ ID NOs: 1 to 54 are measured, and

in the determination step, in a case where at least one among CpG sites in the base sequences represented by SEQ ID NOs: 1, 4, 6, 10, 11, 13, 14, 17 to 20, 23 to 27, 29, 30, 32, 33, 35, 36, 39, 41 to 48, and 50 to 54 has a methylation rate of equal to or lower than the preset reference value, or at least one among CpG sites in the base sequences represented by SEQ ID NOs: 2, 3, 5, 7 to 9, 12, 15, 16, 21, 22, 28, 31, 34, 37, 38, 40, and 49 has a methylation rate of equal to or higher than the preset reference value, it is determined that there is a high likelihood of sporadic colorectal cancer development in the human subject.

[12] The method for determining the likelihood of sporadic colorectal cancer development according to any one of [8] to [11],

in which in the determination step, in a case where a sum of the number of CpG sites having a methylation rate equal to or lower than the preset reference value among CpG sites in the base sequences represented by SEQ ID NOs: 1, 4, 6, 10, 11, 13, 14, 17 to 20, 23 to 27, 29, 30, 32, 33, 35, 36, 39, 41 to 48, and 50 to 54, and the number of CpG sites having a methylation rate equal to or higher than the preset reference value among CpG sites in the base sequences represented by SEQ ID NOs: 2, 3, 5, 7 to 9, 12, 15, 16, 21, 22, 28, 31, 34, 37, 38, 40, and 49 is three or more, it is determined that there is a high likelihood of sporadic colorectal cancer development in the human subject.

[13] The method for determining the likelihood of sporadic colorectal cancer development according to any one of [8] to [10],

in which in the measurement step, methylation rates of CpG sites in the base sequences represented by SEQ ID NOs: 1 to 8 are measured, and

in the determination step, in a case where at least one among CpG sites in the base sequences represented by SEQ ID NOs: 1, 4, and 6 has a methylation rate of equal to or lower than the preset reference value, or at least one among CpG sites in the base sequences represented by SEQ ID NOs: 2, 3, 5, 7, and 8 has a methylation rate of equal to or higher than the preset reference value, it is determined that there is a high likelihood of sporadic colorectal cancer development in the human subject.

[14] The method for determining the likelihood of sporadic colorectal cancer development according to any one of [8] to [10], and [13],

in which in the determination step, in a case where a sum of the number of CpG sites having a methylation rate equal to or lower than the preset reference value among CpG sites in the base sequences represented by SEQ ID NOs: 1, 4, and 6, and the number of CpG sites having a methylation rate equal to or higher than the preset reference value among CpG sites in the base sequences represented by SEQ ID NOs: 2, 3, 5, 7, and 8 is three or more, it is determined that there is a high likelihood of sporadic colorectal cancer development in the human subject.

[15] The method for determining the likelihood of sporadic colorectal cancer development according to any one of [8] to [10],

in which in the measurement step, methylation rates of CpG sites in the base sequences represented by SEQ ID NOs: 55 to 87 are measured, and

in the determination step, in a case where at least one among CpG sites in the base sequences represented by SEQ ID NOs: 59, 65 to 68, 70 to 77, and 79 to 86 has a methylation rate of equal to or lower than the preset reference value, or at least one among CpG sites in the base sequences represented by SEQ ID NOs: 55 to 58, 60 to 64, 69, 78, and 87 has a methylation rate of equal to or higher than the preset reference value, it is determined that there is a high likelihood of sporadic colorectal cancer development in the human subject.

[16] The method for determining the likelihood of sporadic colorectal cancer development according to any one of [8] to [10], and [15],

in which in the determination step, in a case where a sum of the number of CpG sites having a methylation rate equal to or lower than the preset reference value among CpG sites in the base sequences represented by SEQ ID NOs: 59, 65 to 68, 70 to 77, and 79 to 86, and the number of CpG sites having a methylation rate equal to or higher than the preset reference value among CpG sites in the base sequences represented by SEQ ID NOs: 55 to 58, 60 to 64, 69, 78, and 87 is two or more, it is determined that there is a high likelihood of sporadic colorectal cancer development in the human subject.

[17] The method for determining the likelihood of sporadic colorectal cancer development according to any one of [8] to [10],

in which in the measurement step, methylation rates of CpG sites in the base sequences represented by SEQ ID NOs: 88 to 93 are measured, and

in the determination step, in a case where at least one among CpG sites in the base sequences represented by SEQ ID NOs: 90 and 91 has a methylation rate of equal to or lower than the preset reference value, or at least one among CpG sites in the base sequences represented by SEQ ID NOs: 88, 89, 92, and 93 has a methylation rate of equal to or higher than the preset reference value, it is determined that there is a high likelihood of sporadic colorectal cancer development in the human subject.

[18] The method for determining the likelihood of sporadic colorectal cancer development according to any one of [8] to [10], and [17],

in which in the determination step, in a case where a sum of the number of CpG sites having a methylation rate equal to or lower than the preset reference value among CpG sites in the base sequences represented by SEQ ID NOs: 90 and 91, and the number of CpG sites having a methylation rate equal to or higher than the preset reference value among CpG sites in the base sequences represented by SEQ ID NOs: 88, 89, 92, and 93 is two or more, it is determined that there is a high likelihood of sporadic colorectal cancer development in the human subject.

[19] The method for determining the likelihood of sporadic colorectal cancer development according to [12], [14], [16], or [18],

in which in a case where the sum is five or more, it is determined that there is a high likelihood of sporadic colorectal cancer development in the human subject.

[20] The method for determining the likelihood of sporadic colorectal cancer development according to [8] or [9],

in which the multivariate discrimination expression includes, as variables, methylation rates of one or more CpG sites selected from the group consisting of CpG sites in the base sequences represented by SEQ ID NOs: 55 to 87,

in the measurement step, a methylation rate of the CpG site which is included as a variable in the multivariate discrimination expression is measured, and

in the determination step, in a case where based on the methylation rate measured in the measurement step, and the multivariate discrimination expression, a discrimination value which is a value of the multivariate discrimination expression is calculated, and the discrimination value is equal to or higher than a preset reference discrimination value, it is determined that there is a high likelihood of colorectal cancer development in the human subject.

[21] The method for determining the likelihood of sporadic colorectal cancer development according to [8] or [9],

in which the multivariate discrimination expression includes, as variables, methylation rates of one or more CpG sites selected from the group consisting of CpG sites in the base sequences represented by SEQ ID NOs: 88 to 93,

in the measurement step, a methylation rate of the CpG site which is included as a variable in the multivariate discrimination expression is measured, and

in the determination step, in a case where based on the methylation rate measured in the measurement step, and the multivariate discrimination expression, a discrimination value which is a value of the multivariate discrimination expression is calculated, and the discrimination value is equal to or higher than a preset reference discrimination value, it is determined that there is a high likelihood of sporadic colorectal cancer development in the human subject.

[22] The method for determining the likelihood of sporadic colorectal cancer development according to any one of [8] to [21],

in which the multivariate discrimination expression is a logistic regression expression, a linear discrimination expression, an expression created by Naive Bayes classifier, or an expression created by Support Vector Machine.

[23] The method for determining the likelihood of sporadic colorectal cancer development according to any one of [8] to [22],

in which the biological sample is intestinal tract tissue.

[24] The method for determining the likelihood of sporadic colorectal cancer development according to any one of [8] to [23],

in which the biological sample is rectal mucosal tissue.

[25] The method for determining the likelihood of sporadic colorectal cancer development according to [24],

in which the rectal mucosal tissue is collected by a kit for collecting large intestinal mucosa which includes a collection tool and a collection auxiliary tool,

the collection tool includes a first clamping piece and a second clamping piece which are a pair of plate-like bodies, each of the first clamping piece and the second clamping piece is configured to have a clamping portion, a gripping portion, a spring portion, and a fixing portion, and

the collection auxiliary tool has

    a truncated cone-shaped collection tool introduction portion having a slit on a side wall, and
    a rod-like gripping portion,

one end of the gripping portion is connected in the vicinity of a side edge portion having a larger outer diameter of the collection tool introduction portion,

the slit is provided from a side edge portion having a smaller outer diameter of the collection tool introduction portion toward the side edge portion having a larger outer diameter,

a width of the slit is wider than a width in a state in which the first clamping piece and the second clamping piece are bonded to each other at end portions on a side of the clamping portions, and

the collection tool introduction portion has a larger outer diameter of 30 to 70 mm and a length in a rotation axis direction of 50 to 150 mm.

[26] The method for determining the likelihood of sporadic colorectal cancer development according to [25],

in which a recess is provided on at least one of an end portion of a surface, in the clamping portion of the first clamping piece, opposed to the second clamping piece, and an end portion of a surface, in the clamping portion of the second clamping piece, opposed to the first clamping piece.

[27] A kit for collecting large intestinal mucosa, including:

    a collection tool; and
    a collection auxiliary tool,
    in which the collection tool includes
    a first clamping piece and a second clamping piece which are a pair of plate-like bodies,
    each of the first clamping piece and the second clamping piece is configured to have a clamping portion, a gripping portion, a spring portion, and a fixing portion, and
    the collection auxiliary tool has

        a truncated cone-shaped collection tool introduction portion having a slit on a side wall, and
        a rod-like gripping portion,

one end of the gripping portion is connected in the vicinity of a side edge portion having a larger outer diameter of the collection tool introduction portion,

the slit is provided from a side edge portion having a smaller outer diameter of the collection tool introduction portion toward the side edge portion having a larger outer diameter,

a width of the slit is wider than a width in a state in which the first clamping piece and the second clamping piece are bonded to each other at end portions on a side of the clamping portions, and

the collection tool introduction portion has a larger outer diameter of 30 to 70 mm and a length in a rotation axis direction of 50 to 150 mm.

[28] The kit for collecting large intestinal mucosa according to [27], in which a recess is provided on at least one of an end portion of a surface, in the clamping portion of the first clamping piece, opposed to the second clamping piece, and an end portion of a surface, in the clamping portion of the second clamping piece, opposed to the first clamping piece.

[29] A marker for analyzing a DNA methylation rate, including:

a DNA fragment having a partial base sequence containing one or more CpG sites selected from the group consisting of CpG sites in base sequences represented by SEQ ID NOs: 1 to 93, in which the marker is used to determine the likelihood of sporadic colorectal cancer development in a human subject.

Advantageous Effects of the Invention

According to the method for determining the likelihood of sporadic colorectal cancer development according to the present invention, for a biological sample collected from a human subject, in particular, a human subject who does not have subjective symptoms of a large intestinal disease, it is possible to determine the likelihood of sporadic colorectal cancer development by investigating a methylation rate of a specific CpG site or an average methylation rate of a specific DMR in a genomic DNA. In addition, according to the kit for collecting rectal mucosa according to the present invention, it is possible to collect rectal mucosa from a patient's anus in a relatively safe and convenient manner.

Brief Description of the Drawings

FIG. 1 is an explanatory view of an embodiment of a collection tool 2.
FIG. 2 is an explanatory view of an embodiment of a collection auxiliary tool 11.
FIG. 3 is an explanatory view of a use mode of a kit for collecting rectal mucosa.
FIG. 4 is a cluster analysis based on methylation levels of CpG sites in 54 CpG sets chosen as a result of comprehensive DNA methylation analysis in Example 1.
FIG. 5 is a cluster analysis based on methylation levels of CpG sites in 8 CpG sets chosen as a result of comprehensive DNA methylation analysis in Example 1.
FIG. 6 is a principal component analysis based on methylation levels of CpG sites in 54 CpG sets chosen as a result of comprehensive DNA methylation analysis in Example 1.
FIG. 7 is a principal component analysis based on methylation levels of CpG sites in 8 CpG sets chosen as a result of comprehensive DNA methylation analysis in Example 1.
FIG. 8 is a cluster analysis based on methylation levels of CpG sites in 33 CpG sets chosen as a result of comprehensive DNA methylation analysis in Example 2.
FIG. 9 is a principal component analysis based on methylation levels of CpG sites in 33 CpG sets chosen as a result of comprehensive DNA methylation analysis in Example 2.
FIG. 10 is a ROC curve of examination for the presence or absence of sporadic colorectal cancer development in a case where methylation rates of the three CpG sites of a CpG site (cg01105403) in the base sequence represented by SEQ ID NO: 57, a CpG site (cg06829686) in the base sequence represented by SEQ ID NO: 63, and a CpG site (cg14629397) in the base sequence represented by SEQ ID NO: 77 are used as markers in Example 2.
FIG. 11 is cluster analysis based on methylation levels of CpG sites in 6 CpG sets chosen as a result of comprehensive DNA methylation analysis in Example 3.
FIG. 12 is a principal component analysis based on methylation levels of CpG sites in 6 CpG sets chosen as a result of comprehensive DNA methylation analysis in Example 3.
FIG. 13 is cluster analysis based on methylation rates of 121 DMR's (121 DMR sets) chosen as a result of comprehensive DNA methylation analysis in Example 4.
FIG. 14 is a principal component analysis based on methylation rates of 121 DMR sets chosen as a result of comprehensive DNA methylation analysis in Example 4.
FIG. 15 is a ROC curve of examination for the presence or absence of colorectal cancer development in sporadic ulcerative colitis patients in a case where average methylation rates of the three DMR's of DMR represented by DMR no. 11, DMR represented by DMR no. 24, and DMR represented by DMR no. 42 are used as markers in Example 4.

Description of Embodiments

A cytosine base of a CpG site in a genomic DNA can undergo a methylation modification at a C5 position thereof. In the present invention and the present specification, in a case where a methylated cytosine base (methylated cytosine) amount and a non-methylated cytosine base (non-methylated cytosine) amount among CpG sites in a biological sample collected from an individual organism are measured, a methylation rate of a CpG site means a proportion (%) of the methylated cytosine amount with respect to a sum of both amounts. In addition, in the present invention and the present specification, an average methylation rate of DMR means an additive average value (arithmetic average value) or synergistic average value (geometric average value) of methylation rates of a plurality of CpG sites present in DMR. However, an average value other than these may be used.

In the present invention and the present specification, "sporadic colorectal cancer" means colorectal cancer which develops by accumulation of accidental gene mutations due to environmental factors such as aging, diet, and lifestyle in an individual in whom an underlying causative disease is not clearly recognized and apparent hereditary colorectal cancer is also not recognized from a family history or genetic test, and which is also called sporadic colorectal cancer in some cases. That is, sporadic colorectal cancer includes all colorectal cancers except colorectal cancer that develops from a clear causative disease and hereditary colorectal cancer. For example, colorectal cancer that develops with progress of other inflammatory diseases of the large intestine such as ulcerative colitis is not included in sporadic colorectal cancer (Cellular and Molecular Life Sciences, 2014, vol. 71(18), pp. 3523 to 3535; Cancer Letters, 2014, vol. 345, pp. 235 to 241). In addition, hereditary colorectal cancer such as familial adenomatous polyposis (FAP) and Lynch syndrome is also not included in sporadic colorectal cancer (Cancer, 2015, 9:520).

<Method for determining the likelihood of sporadic colorectal cancer development>

The method for determining the likelihood of sporadic colorectal cancer development according to the present invention (hereinafter referred to as "determination method according to the present invention" in some cases) is a method for determining the likelihood of sporadic colorectal cancer development in a human subject in which the difference in methylation rate of CpG sites or DMR's in a genomic DNA between a healthy subject group which has not developed colorectal cancer and does not have subjective symptoms of other large intestinal diseases and a colorectal cancer patient group which has developed sporadic colorectal cancer is used as a marker. Using a methylation rate of a CpG site or an average methylation rate of DMR, both of which become these markers, as an index, it is determined whether the likelihood of colorectal cancer development in a human subject is high or low. By using a methylation rate of a specific CpG site or an average methylation rate of a specific DMR as a marker used for determining the likelihood of sporadic colorectal cancer development in a human subject, it is possible to detect sporadic colorectal cancer at an early stage, which is very difficult to make by visual discrimination, in a more objective and sensitive manner, and it is possible to expect early detection.

An average methylation rate of a CpG site or DMR used as a marker in the determination method according to the present invention can distinguish between a healthy subject and a subject who has developed sporadic colorectal cancer. Therefore, the determination method according to the present invention is suitable for determining the likelihood of sporadic colorectal cancer development in a human who does not have subjective symptoms of a large intestinal disease. In addition, the determination method according to the present invention is more non-invasive than an endoscopic examination and can determine the likelihood of sporadic colorectal cancer development in a more accurate manner than a fecal occult blood examination. Thus, the determination method according to the present invention is particularly useful for colorectal cancer screening examination such as large intestine inspection. For example, the determination method according to the present invention can be performed on a subject who is positive in a fecal occult blood examination.

Determination of the likelihood of sporadic colorectal cancer development based on a methylation rate of a CpG site used as a marker may be made based on the measured methylation rate value itself of the CpG site, or in a case where a multivariate discrimination expression that includes the methylation rate of the CpG site as a variable is used, the determination may be made based on a discrimination value obtained from the multivariate discrimination expression.

Determination of the likelihood of sporadic colorectal cancer development based on the average methylation rate of DMR used as a marker may be made based on an average methylation rate value itself of the DMR calculated from methylation rates of two or more CpG sites in the DMR, or in a case where a multivariate discrimination expression that includes the average methylation rate of the DMR as a variable is used, the determination may be made based on a discrimination value obtained from the multivariate discrimination expression.

For a CpG site and DMR which are used as markers in the present invention, it is preferable that a methylation rate thereof be largely different between a subject group which has not developed colorectal cancer and a sporadic colorectal cancer (hereinafter simply referred to as "colorectal cancer" in some cases) patient group. A larger difference between the two groups allows the presence or absence of sporadic colorectal cancer development to be detected in a more reliable manner. For the CpG site and the DMR which are used as markers in the present invention, a methylation rate thereof in colorectal cancer patients may be significantly higher than in subjects who have not developed colorectal cancer, that is, a higher methylation rate may be exhibited due to colorectal cancer development, or a methylation rate thereof in colorectal cancer patients may be significantly lower than in subjects who have not developed colorectal cancer, that is, a lower methylation rate may be exhibited due to sporadic colorectal cancer development.

For the CpG site and the DMR which are used as markers in the present invention, it is more preferable that the same colorectal cancer patient have a small difference in methylation rate between a non-cancerous site and a cancerous site in large intestine. By using such a methylation rate of a CpG site or such an average methylation rate of DMR as an index, even in a case where a biological sample collected from a non-cancerous site of a colorectal cancer patient is used, it is possible to determine the presence or absence of sporadic colorectal cancer development in a highly sensitive manner similar to a case where a biological sample collected from a cancerous site is used. For example, mucosa deep in the large intestine needs to be collected using an endoscope or the like, which places a heavy burden on a human subject. However, rectal mucosa in the vicinity of the anus can be collected in a comparatively easy manner. By using a CpG site or DMR having a small difference in methylation rate between a non-cancerous site and a cancerous site of the large intestine as a marker, irrespective of a location where the cancerous site is formed, it is possible to thoroughly detect a human subject who has developed sporadic colorectal cancer using rectal mucosa in the vicinity of the anus as a biological sample.

Among determination methods according to the present invention, the method for making a determination based on the measured methylation rate value itself of the CpG site is a method for determining the likelihood of sporadic colorectal cancer development in a human subject, the method including a measurement step of measuring methylation rates of a plurality of specific CpG sites to be used as markers in DNA recovered from a biological sample collected from the human subject, and a determination step of determining the likelihood of sporadic colorectal cancer development in the human subject based on the methylation rates measured in the measurement step and a reference value set previously with respect to each CpG site.

Specifically, a CpG site used as a marker in the present invention is one or more CpG sites selected from the group consisting of CpG sites in the base sequences represented by SEQ ID NOs: 1 to 93. The respective base sequences are shown in Tables 8 to 16. In the base sequences of the tables, CG in brackets is a CpG site detected by comprehensive DNA methylation analysis shown in Examples 1 to 3. A DNA fragment having a base sequence containing these CpG sites can be used as a DNA methylation rate analysis marker for determining the likelihood of sporadic colorectal cancer development in a human subject.

[Table 8]

| CpG ID | Base sequence | UCSC _REFGENE _NAME | ± | |
|---|---|---|---|---|
| cg07621697 | GAGTGTTCCATTTGCTCCCTTCCCAGCGGAAAGGCCCTCAT CTGCTCCCGCTGGACTGGG[CG]CTGCTCTGGTTCCTAGCCT GTGGCTTAGTAAGTGCTCAGGAGAAGTCAGTTGAATGAGTG | | − | 1 |
| cg16081854 | CCTGGGGGCCAGGGAGGCCAGTGCTGCCGATTGCGGCCAG GGCCACGTGGACTTCAGGAC[CG]GCCTGAAGTTATTTTTAG ATAAGCGACCTCTGGCGCCACGGACATCTTTTCCTAACCTT G | AHRR | + | 2 |
| cg01710670 | ACCTGTGCTCCGTCCCGCACGTGGCTTGGGAGCCTGGGACC CTTAAGGCTGGGCCGCAGG[CG]CAGCCGTTCACCCCGGGC TCCTCAGGCGGGGGGCTTCTGCCGAGCGGGTGGGGAGCAG GT | | + | 3 |
| cg22946888 | ACCTCCCAGGGCTCCTTGCCTTAGGTGGCTGTAGCATCCCT ACCACCCAGGACACTGGTG[CG]AATGACACAACTCAAGTTG GGAGGGGAACAGGGAAGGAAGGGATGGATGGGGGTGGTGT A | THG1L | − | 4 |
| cg00713204 | CCCGCTCCCCTGTCAATGTGGGCCGGCCTCCCGCTCCCCTG TGCTGCGAGCTCCACGGCC[CG]CTCTCAGTGGCTGCCTCAG TGCCACCCCTGCTGTCTCGAGCCTACCTCCCCCTTCCTTCT | BANP | + | 5 |
| cg12074150 | CTGATGTTGGGATGTGTTCGGCCTTCTGGTGGTTCGTGGTC TCGTGAGTGAAGCTCACAG[CG]GTGTGGGGAGGCTCAGGCA TGGGGGGCTGCAGGACCCAAGCCCTGCCCTGCGGGGAGGC A | | − | 6 |
| cg06758191 | ACCCCAGCGCCCGACCCTTTCCCCTTCATCTCCAGCATGAA TCCCTCAACCCGCTGGCTG[CG]GAGATCACAGACACTTCAG AAGGTGATGAGAGTCAAGGACTCCCTCCCACCCCCACCGCA | AFAP1 | + | 7 |
| cg12515659 | ATAAAACAGATAAGGAGAAGGCTGTATCTAGGCTGAATGGC TGGCCAATGTTTTCCTCTC[CG]TCAGTATAAATAAAATGGAT GGAAGAAAACACCCCTGGATACTATCAAATATGCCTTTCA | FAM134B | + | 8 |
| cg18172516 | AGAATTGAGTTACAATCAGTGACTCAACATTTTGACTTAGCA GATTGGCATTCCTTTTTA[CG]ATGGGACAAATTCTGTAAACT GCACATCGTATAGATCACACTTTTCAGCAAAATGCTCAA | RBMS1 | + | 9 |
| cg12280242 | GATCGGACCATCCTGGCTAACATGGTGAAGCCCCGTCTCTA CTAAAAATTCAAAAATGAG[CG]GACCAAGATGGCACACGCC TGTAGTCCCAGGTCCCAGCTACTCGGGAGCCTGAGGCAGGA | | − | 10 |
| cg27288829 | GAGCCCCAGGCTTGCCTCCCGGCTCCGGGGAAATCGGTTC CCTCCACTGGGGCCGGCATG[CG]CTCTGCATCCCCAGGCT GTCCTCCTCGGGCTTGGGGGGGTCTCCTGCTGTGCCTCTGT CT | RAX2 | − | 11 |
| cg14293674 | GCATGGACACATCATTATCACCCAAAGTCCATAGTTGACAT GGAAGTTCGCCCTTGGTGC[CG]TACATTCTATGGGTTTTAA CAAGAATATTCACCATTACAGTATTATACAAAAGAGGCTGG | | + | 12 |

[Table 9]

| CpG ID | Base sequence | UCSC _REFGENE _NAME | +I | |
|---|---|---|---|---|
| cg02507579 | TAAGAGTAAGATGATATCTCTCTCTGAATGCAAGATACAATTT TTTTCCATTGCAATTGG[CG]TAACCACAGAATGTTTTCTCTTG GCAACAATGGCATATGATCGCTATGTAGCCATATGCA | OR5H15 | — | 13 |
| cg19707653 | CCTGTGGGGATACTGAGGTTTATGTATGGTGCCAACCATGATT TAGGTCTCCTGTGGGGA[CG]GTTTGGAGGCCAAATGGGGAGG CGGAGGCGGAGCACTAAGGAATCCAGTCTCTGTACCAG | KIAA1671 | — | 14 |
| cg19285525 | TAGTTGGCACACACCCTCACCATGATCTAATAGACAGCTGTAT AATACTAAAGTGCCTAC[CG]CGTTGCATCATGATAAAGTGAC ATCATTGACTGGTACTGATGCTAAGTTTTGGGTGCTTC | RBMS1 | + | 15 |
| cg04131969 | GGCCCAATTCCCACTCCCCCAAACACACACAAGTACACACTG ACTAAGGCACAGCTAGGG[CG]GGGGCGGGCAGAAGGCCCCT TGGGAGGACGTGGCGCCACAGCTGCAATGGGTGTGGGGGT | MYADML | + | 16 |
| cg07227024 | TCTGGATCCAAGTCAAATTTTCAGTGATGGAAGAATCACACAT CACCTTGTGGATTTGAA[CG]GCTCCTCTTCAGTTGTCTCCCAC AGACTGCCATAATTTGCCCCAGAATAGAGTCCCTGAG | ALS2CR12 | — | 17 |
| cg00695177 | ACGTGTTCTCAGGACTTCCTGAGGGCTGTGTCACCGGCCATG GTCACTCATATTGGGATC[CG]ATTAAAATATTTCTTCAAATAT TTTAGAGTTTGACTTTTTTCATCAACATGATGAAGCCA | | — | 18 |
| cg03311906 | TGGGATTACAGGCGTGAGCCACCGTGCCCGGCCGTCTACTAC TTCTTAAAGGGTGAGAGG[CG]GAAGGATCACTTGAGCCCTGA AGTGTGCGACTGCAGTTAGCTTTTATCGTACCACTGCAC | | — | 19 |
| cg20536971 | GTTTACGTTCACACTCGCTAAAAGGGGTAGGAAGAATTGGAG AGCTTTTAAAATACTTAC[CG]CGCCCCCAAGTTTTAGGTGTGT AGGATTCATCAGTAAACAGAAAAAGGAGCTGCCCTCAT | PCCA | — | 20 |
| cg15828613 | ACCAAAGAAAATAGTTGCAGCTTAATGCCTCACTTGGGAGTTT GCAAAGTCTCTGCTCTC[CG]AAGGCCTTGGTGGGTGAAAAGC CTAAATCGTCCTTATTTCCCACCTTGCTTCTCTCCTTC | | + | 21 |
| cg24506221 | GCCCTCTCCCGGGCCTCCAGAATGGCGCCTTTCGGGTTGTGG CGGGCCGAGGGGCGGGGT[CG]CAGCAAGGCCCCGCCTGTCC CCTCTCCGGAGCTCTTATACTCTGAGCCCTGCTCGGTTTA | GSTM1 | + | 22 |
| cg27156510 | CCCAGCCTCAGCCTCCTAGAGTGCTGGGATTACAGGCGTGAG TCACCGCACCCAATCCCA[CG]TCTGTCTTTTAATCAAGGCAT GCTCTGCCTTCAAGTACACCCTCCATGATGTCTGCCAGA | | — | 23 |
| cg26077133 | TACCTTTAGAACCAGGGGAGGATCTGCTCTCAAGTTCACTGA GCCTTTCCAACCAGTGAG[CG]GTAGAGTGGATCCTCCCCCTA CCAAGCCTTCAGATGAGACCGCAGCCCAGCTGACACCTT | MSRA | — | 24 |

[Table 10]

| CpG ID | Base sequence | UCSC_REFGENE_NAME | ± | |
|---|---|---|---|---|
| cg24087071 | GTATCCTGTGTGTGTTTGATACCTCAGATTCAGCATCTACTACA GCACGAAGTGCTTATG[CG]TGTCCTGAATTATAGGAGAGTCGGA TTCACCACCCTGCCCAGAAACAGAAGCATTCCAGA | SERPINA10 | — | 25 |
| cg17662493 | TTTCTCCTTTTCACATCCCTTCCCCTATATCCACAAAGCAGTTTA AATTTTCAGGCTGGG[CG]CAGCAGCTCACACCTGTAATCCCAGC ACTTTGGGAGGCCGAGGCAGGAAGATCACCCGAG | SMC1B | — | 26 |
| cg12036633 | AGGAGGACATCACCTTAAAGTACCAGACTCTAGGGCCAGCCTGT GTTGGGAGAACCCCCC[CG]CCCCTTCTCTTGCAGCTTCCCCCG GGGGGGACAGATCTTCATGGGGACACAAGGGAGAGT | | — | 27 |
| cg11251367 | ATGAATGGCTGGCCGACTGAACTATGTATTCACTGGGCCTTATT CTGCTCTCTCTAGAAC[CG]CACAGATAAATCCAATCCTTTGTTC CATGTAATAAATCTGATATTTAAGGTTCGCTATGA | FMN2 | + | 28 |
| cg14181874 | GAGCCCTGCCCGAGGAGAGGTGGCTGAGGCCCAGCAAGAATTC GAGCGGCATTGGTGGGC[CG]GTAGTGCTGGGGGACCCGGTGCA CCCTCCACAGCTGCTGGCCCAGGTGCTAAACCCCTCA | | — | 29 |
| cg21164300 | TCAGCTTGGCTCACTGGTGACGACGTATCCAAAATGCCGTATTT AACACATTGGCTTGAG[CG]GTAGAGCAGCTCTCAGATGGCTTCC AGGACTGGCTGAGCTGGTGTTGAGGCCTCATTCAC | | — | 30 |
| cg19405842 | TGGTGTGCAGTTCTCTGTCTCGTGATTCGTGTAACAGTGAGTGC TGCCTGCACCAACAGC[CG]GCTGCCTTCCGTGGCTGTGTGGGC TCCTGTGCGGAGGCCGCCCCTCTCCCTGGCCAAGCA | PRKCZ | + | 31 |
| cg21114725 | GCTGTGCGAGGCGCTCGCGGACTGGTGCAGGTTCTGGGTGGGC GCCAGCTAGGCAGGCCC[CG]CACTGGGCGCAGCCGGCCAGCG CCTGCTGGGCTTCATCCAGGGATGAGCTCCCTCTGGGC | | — | 32 |
| cg08433110 | TGACTTCACCGTGCTGTGTGAGCATCCGCTGAAGTCGTATGGAA ACACCAGGATGTGGGG[CG]GCTGGAAGTCTCCCGTGTTGCTGG TGGGAATGCAACAGGGCAGAGCGGTTGTGGAAAACA | GMDS | — | 33 |
| cg16051083 | TTACAGATGAGAAAACTCAGTGCCATATATCTTTGGAGTCTATT GTACAAAAATAGAATA[CG]TTGAACATGGAAAGTGGCTTTCTAT TTATTTATTTATTTTTGAGAGAGTCTCGCTCTGTC | ZDHHC14 | + | 34 |
| cg11454325 | CAGAGGTTATCGAATGCCGAGGAGCCCAGGATGCACTTCCGAG GCTCACTGGTGACTTTC[CG]GAGATACTTAGGCAAATGGACATA AATAGCTCTTGGATCCTAGCAGGAATTCTCAACCTC | GPR123 | — | 35 |
| cg12870217 | GCCTGATAAAGTAGGCGGTGGGCTGCTGGGTCCTAGATTGGTTA GTTTGCATATGAAAGG[CG]GCTAAGGAGTGAGTTTTTTGCTATG TCTAGAAATTGACTTGCCCTAGGAGGGTCAATCTC | | — | 36 |

[Table 11]

| CpG ID | Base sequence | UCSC _REFGENE _NAME | ± | |
|---|---|---|---|---|
| cg24208588 | GAGGTCTCGCAGGGGGACTGGTTGTCTTTTAGGAAATCAAGG GGCCAGCGCCCCCAGTGC[CG]GCTGGGAGATGCCTTCAGAGT TCGAAGAGAAAAGATGCGACCTTCAATCCGCTCCATTCT | | + | 37 |
| cg08429705 | GGCTGCTGGCATTCCCACCTTCTAGAGTGACTTTCACACTTCC TGATGAGTTTCCCATTC[CG]CTCAGCAGGCCCATAAATAGGAT TGTGCAGAGGTGCATATGCAAGCACTTTACCTGAAGA | GNG7 | + | 38 |
| cg24976563 | CTGATCTTTACTTACACAGACCAGACAATCCGACTCTATGACT GCCGATATGGCCGTTTC[CG]TAAATTCAAGAGCATCAAGGCC CGCGACGTAGGCTGGAGCGTCTTGGATGTGGCCTTCAC | DCAF11 | − | 39 |
| cg14323910 | TATTCTTCTGGGGAATATGAAGGGTTCAGTCTTTTTAGGAAAT TGGATGATATCTCTTCC[CG]ACCACTAGCAGCCTCTTTCAGTC ACTGGAAAATGCTTACAGGCAGTAGCCACCATCATGT | HLA−DQB1 | + | 40 |
| cg04212500 | CATCATCTTTCTCCCAGATCCCATCAAAGCAGAATGGTAGAAA CCTAAGGTCAGCCTGGG[CG]CAGTGGCTCACGTCTGTAATCC CAGCACTTTGGGAGGCCAAAGCAGGCGGATCACTTGAG | ERAL1 | − | 41 |
| cg00348031 | GGGATCCGCCTGTCCACGTGCAGCCGCCTCCGGGCGGCGTCG GCCATGCTGCTGCCCCAC[CG]TGGCTCTGTGGCTCCAGCCGG AATGGCAAAGCCTGGCTCCACAGCTGCCTGGGAGCGTGA | NFATC1 | − | 42 |
| cg02890235 | CCCCAGGTCTGGGTCCCGGCAGGGCTGGAAGGAGCCTGAGAG GGATGTGCGCAGCACCTC[CG]AGAGTCCCGCTTTAGAGAAAC ACGAATCAGATCATGAGAAAGCAGACCTCTGAGAAGTCA | | − | 43 |
| cg00525828 | CCCTTCTCCCTTTCCTGGGGACACCTGAGCAGCGCCACGGTG ATGGCAGGCTTGTGCACG[CG]TCATGCAGATACATCCTTATTT TCTTCCCACTCTTCGTCGTCCCCTGCCCGCCCACCCTC | BANP | − | 44 |
| cg02775404 | TGTTCTCTGGGAAATCCTTTTCAAGATAATTGAACTCTGCCTT TGAAACTCATCCTCTAA[CG]TAGATAGCGGGGCAGGGCTGATT ACAGAGGACGGAAGCCCAGGAGCCCCAGGGCCTGGCA | | − | 45 |
| cg23663942 | GACCTACCTGTACAGCTTGGTGTCACCACCTTGATTTGTGCTC AGGCACTAACAGTTTCA[CG]TGACCACCATAGATTTCTGTACC AATATGTAAATAATACAGTGAAAAAGGCAAATAACAT | | − | 46 |
| cg15115757 | CAGAAATGCCATCATCGTATGTGACACAGAATTTAGAAAAATG ACTTTGTGAAGAATGGC[CG]GAAGAGGGAAGCTAATGGTAGA GAAACCTCTCTGGTGATGGGATCATCTTAAGTCTATGA | TAP2 | − | 47 |
| cg03022891 | GCCACATGGGCACGTGTGGCCATGTGGGGGGTGCAGGACCCA AGAAGGAACAAGAGGGGC[CG]CGTAACCCTGCACAGCCTGGC CTGCTCGCTCCGCCGCCTCGGCCCTGCCCGCCCTCCTCT | TNNT3 | − | 48 |

[Table 12]

| CpG ID | Base sequence | UCSC _REFGENE _NAME | +/− | |
|---|---|---|---|---|
| cg22664298 | AAACTCCTGCAGCGTCCAGAACACAGAAAATAGACTCA TCTCCTAATTCGCCAGGGAGCT[CG]AGGGCTGCGGGGC CGCGGGGCTGCCTCCCCGCTCCTCCCCCAACCCGAC CCCACCCCAC | ADAMTS19 | + | 49 |
| cg06306564 | GGACAGAAAGCTGTTAGGCTGTGGGTTTAAAATAGGAT ATCCATGTAAACTGAAATAATG[CG]CTTACATGTTTAAA CAGCTAAGTGCCAGTTCAAAAGCAGTTTGATATTAGTTA TTTTCAT | HOPX | − | 50 |
| cg01647917 | TGGAGGAAAGCTCGGAGCTCCCATGCCCTCCCGGGGCA CCGCCTTCCAGGAACCTGCCTG[CG]TTCCGCTTCTGGG CACCCGGAAAGTCGCTCAGTGGCTGATTCAGGGTCGAG GAGCTGTGA | GZMM | − | 51 |
| cg16661157 | TTGCCTGTAGCCCATTGATCTACCCACTATGTATATTCA TTTTAATGCTGTTTTTGAGTC[CG]TTGACTACCCCGGGA AATCAAAGTTGACTACCACAGCCCTAGTCCTCAAGTGT CTTGCCT | PRKCA | − | 52 |
| cg17025908 | CATTGCTCCACACACCATCTCTCATTCATCCTCACCTCA CCCTGCTCGGACCAGTTCTAA[CG]GCAGTGGTTTATGG AGCACCTAGACATCAAATCGAGTGCCAGGCATCAGATG GAGGCTTC | | − | 53 |
| cg19455396 | AACACTTAGCATAGCTCCTACTCCCATTAAAACTCTATA AATGGTAGCTGTTACCAATGT[CG]CTATTAATACTGTTA ATCAGGGAACTGTTCTCTGTCCCTCCAGACCCTAGCTT CTTCAAA | TAP2 | − | 54 |

54 CpG sites in brackets in the base sequences represented by SEQ ID NOs: 1 to 54 (hereinafter collectively referred to as "54 CpG sets" in some cases) have a largely different methylation rate between a subject group which has not developed colorectal cancer and a colorectal cancer patient group in comprehensive DNA methylation analysis in Example 1 as described later. Among these, colorectal cancer patients have a much lower methylation rate than subjects who have not developed colorectal cancer at the CpG sites ("-" in the tables) in the base sequences represented by SEQ ID NOs: 1, 4, 6, 10, 11, 13, 14, 17 to 20, 23 to 27, 29, 30, 32, 33, 35, 36, 39, 41 to 48, and 50 to 54, and colorectal cancer patients have a much higher methylation rate than subjects who have not developed colorectal cancer at the CpG sites ("+" in the tables) in the base sequences represented by SEQ ID NOs: 2, 3, 5, 7 to 9, 12, 15, 16, 21, 22, 28, 31, 34, 37, 38, 40, and 49. The CpG site used as a marker is not limited to these 54 CpG sites and also includes other CpG sites in the base sequences represented by SEQ ID NOs: 1 to 54.

As the CpG site used as a marker in the present invention, only the CpG sites in the base sequences represented by SEQ ID NOs: 1 to 8 may be used. Among the 54 CpG sets, these 8 CpG sites (hereinafter collectively referred to as "8 CpG sets" in some cases) have a small difference in methylation rate between a non-cancerous site and a cancerous site of the large intestine in colorectal cancer patients.

[Table 13]

| CpG ID | Base sequence | UCSC_REFGENE _NAME | +| | |
|---|---|---|---|---|
| cg00853216 | TGTACTATAATTGTTTATGTATCTGTCTCATCTTCCTCTCCAGC CTACAAAATTCTTTGA[CG]TAAAAGGCCCTTTTCTATTTGATTT GTATCCTTAGCCCTTAGCAGAATACGTTGTTCATA | SOX6 | + | 55 |
| cg00866176 | CCTCCCTCCCCAACAACTCAAAAGCAGCGAGGCCTGTCCTTGA CCTGTCTGAGAATGGGC[CG]CTTCACCACCCTGCTTGGTTAAC TGAAGTCACCCGCACTGCAACACCCTGGTATCAGCCT | ST3GAL2 | + | 56 |
| cg01105403 | TGTCTACACCACGCTGGAACCATTTTCTGTCCCACCTCGGGAC TGGGTGGCACGTGAGAG[CG]GCCAGGGAGAGACCGCATCTGG GAAGGCACAGCTGGCTGCAGGGAACGGCCGCCCTGGAA | – | + | 57 |
| cg02078724 | ACTCAATTAGAAAAGCAGCGAAGCATGGTGGTTAAGAACACGG CTTCAGCAGACAGGCTG[CG]TTCAAAACTCAGTTCCCTCACAT ACTAGCTGTCGACTGGCTTTTCCAGTTTCGAAGAAAA | LSG1 | + | 58 |
| cg03057303 | TTGATTTATGCCCTTATTGTGGAATGAAAGTGCTTGTTACATAT TTCAAGAAAATGAATG[CG]CTCTTAGAAACAGATTGGAATGTA GGATGTATGCCAGCTTGTGGCAATGAGAATGCTTAA | SNHG16; SNHG16; SNHG16; SNHG16 | – | 59 |
| cg04234412 | CAGCACTGGGCGAGGGGAAGTTGGTGGGCCAGGGGTCCGGCC TTGTCCCTGCTCTGCCTC[CG]CAACAGCGACCCCGATCCCTTT CCCCAGGGACCACCCCCCACCCCATTCCGCAGGCCAAG | LOC391322 | + | 60 |
| cg04262140 | TGGTCGCAAAAGCAGCCCTTTCAATCGCACCGAATTTCCCCTG GTGTGAAAAGGCGCCAT[CG]CCAGCATTTTGCCGGGGGTTTATG CCTCAATCCCGCATTCCAGCCACTTCCACGAATTACT | – | + | 61 |
| cg04456492 | TCAATTTGGTAATGTGCTCATTACTGCTCCTAATTCATTCATAT TTTAGCAAACACTTAG[CG]TGGTGAGGCTTCTGATCCTCAGCA CTGGTAAAAATCTAACATTTATTGTATCTGTTCTAA | – | + | 62 |
| cg06829686 | GCAGGGGTCTCTACCCGGTGCCTTCCTCCCGGCACGCTAGCCT CCTCGCCGAAATTTCGT[CG]TCCCGGAGTCGGTAACCGAGTCC CAGGCTTTACTGCCACTCCACTCCCTGCTGGGTTATT | – | + | 63 |
| cg07684215 | AGGCTCTGGGCAGATGTCAGCTAAGGTCACGGCAGGAGGCTGA AGGGGAGGCTCCTGGCA[CG]TGACTCTGGATCGATGCCCCCC ATGTCTCCCCTGACCTCTGACTGTTCTAGATCCACAAT | TCERG1L | + | 64 |
| cg08421632 | TGAACTCCTGACCTCAGGTGATCCGCCTGCCGCGCGGCCTCCCAA AGTGCTGGGATTATAGA[CG]TGAGCCACCTCGGCAGGCCACCT GATGTTTTTTGGCACATAGCATAGTCTATGGTGTCAA | ANLN; ANLN; ANLN | – | 65 |

[Table 14]

| CpG ID | Base sequence | UCSC_REFGENE_NAME | ± | |
|---|---|---|---|---|
| cg10169393 | TTACACAGTAGGCTTCTTATTCAAGAAATCACAAAACTCAGGG ATTAACAGCCAGGATTT[CG]CAACTAGTTTTTGGGGTTCAAAT CTCAGCTCTACTGGTTACTAGCTGTGAATAAGCCCTG | – | — | 66 |
| cg10204409 | TTAATATCAGCAGTAGCTGGAATTAGAGTGCTGACTCTGCACC AAGCACTGTTCTAAACA[CG]TCATGTTTGTTGGCTCATTTTCA GTCTCACAGTAGCACAGTGGGGTGGAGATTCTTGTTA | SLC24A4; SLC24A4; SLC24A4 | — | 67 |
| cg10326673 | CTCCTGATCAGGGAACCTGGGTTCTATAACTGCTTCTACTACT GATTTGTCCTGTGACTT[CG]CGCACCAAATTTAGGCTTGTAAA TTAAACTCCCAGATTTCTGTTTTCCATTTTGCAGCTC | LCLAT1; LCLAT1; LCLAT1; LCLAT1 | — | 68 |
| cg10360725 | CAGCTGGCCTGACTGGGGGCCTGTGTCGGGTGCCATATGAGA GATTTCAACCAGCCCATG[CG]CAACCAGAGGGATGCGGCCCA CGGTGCGGGTGGTCTCAGCGTCGTCTCTGTCTGACCCTC | – | + | 69 |
| cg10530344 | TGCACTGCCAGGGCCTGTGAGCTGCCACACCAGGACACTGCC TGGCTTGCTTGGGGCTGG[CG]GGATCCCCTGAGCTGAGATCT GGTCTCCCTTTGGGAAGGGTGGGAGAATGGTGAGAGAAG | – | — | 70 |
| cg10690713 | ATGGCTGGGTTTTGGATATATTTTAAGTAGAGCCATCAGGATTT GTGAAAGGATCAGATG[CG]GATGTGGAAGAAAGAAAAATATCA AGCCTGACTCCTGGGCCATCGACAGTGGGAGGTGCC | – | — | 71 |
| cg10772532 | CACATATGTCTGCCTCCTATCATTTCTTCATGAGGTTCAGGGC AAAGGGCCTAGTCAAGC[CG]ATGATCTTTGGTTGCCCCTACAC TTTCCCCAAACCACCTACAAATAAACAAAACAAGGGG | C14orf145; C14orf145 | — | 72 |
| cg11044162 | GAGAGGGGGAGAAAAGTGAAGCGGGATAGATTTAGGGTAGAG ATGTTCAGGAGAGGCGGG[CG]ACCCATCTCAGATGAAATTCAG AAAAACTGACAACTGACTAGGGGTGGCAGGATGGCACA | ADAMTS9 | — | 73 |
| cg11141652 | CACTTGCCAGGTGGTGCTTGGCGAAGGCAAGCAGCTCCCACC CGCCCGGGGAATACAGCG[CG]ACCCCCGGCGGCATGCTCTTC AGCACCACCCCAGGAGGTACCAGGATCATCTACCACTGG | GSTTP1 | — | 74 |
| cg12219587 | GAGCCTAAGTGATCTGTTTAAATTGTAAATCTGATCACACCAC ACCTCTGCTTAAAACTC[CG]TAATGCTTTTGCATGGCCTTCAG GATAAATCTAAACTCCATAGCATCGCTTTGAAGACCC | – | — | 75 |
| cg12814117 | CAACCTACTTGACTCGCACCACTGACCCCCACACCTTGCATAG ACTGAGCAGATATATAA[CG]ATGGCCACCTCTCCATCTGATTC TAGACTGATTCTAGTTCCTAGAATCTCAGCATGATTC | – | — | 76 |

[Table 15]

| CpG ID | Base sequence | UCSC _REFGENE _NAME | +/- | |
|---|---|---|---|---|
| cg14629397 | TACCAGTCAGTAGTGGGTGACAAGGCCTTCCCACAGCATTTATC TTTAAGCTTCAGCATA[CG]TATTTGTACTCTTCATCCTATCTATT TGGAGTGGTCTCAAATTCCACAGGCTACTCCACG | – | — | 77 |
| cg16013720 | TCACTTCATTTCGTTCAATTTCGTTCAATTTCATTCCTTTTCATC CAGCGCCGGGAGGCC[CG]AGGCCACAAGGAAGGGGAGGGGGTC TTTCCGGGCGAATTTCCCTCATCTTGTAGATTTAC | – | + | 78 |
| cg16776298 | AGCCCCCACCTCTGGGCACCCCCTGGGTGGTTTGTCTCCATCGA CTGGCATTTACCATGA[CG]TCTCTCATATTATGGCCACTTGCACT TGCCCAGAGGTGGGCCTGCTCGCTCCTCCCCAGC | AJAP1; AJAP1 | — | 79 |
| cg17658874 | AAATATGAATTATGCAAATACATTTCTGCCCATTGAGATGATATT ACTCAACAGGGCCCT[CG]TAAGTGCCCAGTTCTGTTGGATGTTT AGACAGAAAACAAGCAAACTGTAGATACCGGCAA | RBMS3; RBMS3; RBMS3 | — | 80 |
| cg18285337 | TGCTCTTTGCTTGCCAACTGCGCAAAACCAGGCAGTGGGGCAGA TTTGGCCTGAGGGTCA[CG]GTTTGCCAACCCCTGCTCAAGCCTG CTCACTCTCAACGCTGGCTGCACGTTGCAATAATC | – | — | 81 |
| cg19236675 | TTGGCGTCACATGCCGAAGGAGTCTTCTAATGTCTCTCCCTCTC TGCGTGTCTGCTCTCA[CG]CCCGTGCAGGCATGACGAGTGTTCT GATGTCAGCCATTGGACTCCCTGTGTGTCTTAGCC | PMS2L11 | — | 82 |
| cg19631563 | CTGACAAAGGATGCTGGTGCTGAAATTCTTAATTCACTTAGCCT GTCAGCTTTGAAATTA[CG]ATTATAGAATTCTAAGAAACTTTGCA TGCTTTATATCAGATTTGTACACTTCTAATTTAT | EI24; EI24; EI24; EI24 | — | 83 |
| cg19919789 | CAGGAAGTTTTTTCCTGTGGTGGAAGCTTTTGTTCTCCAAGTCGA ATTTCCCTCAGCTGA[CG]TCAGCCCCAACTTAGGCCCAAGCCCA TTGAACCTGCAGTGGGGCTGAGGGAGGGCTGCCT | – | — | 84 |
| cg22109827 | AGCTGAACAGGCAAGGCTGTATGTTTGGAGAAGCTGGGACCCTA TCCGCTGCACTCAGAG[CG]GGGACCATCCGCCAAGGGAGACAG GGAAGGGTCTGTGCCACCTGCTGGAGGGAGGGCAGA | – | — | 85 |
| cg23231631 | GCAAGGTGGATGGATGATGATGATAGATAGATAGATAGATAGAT AGATAGATAGATAGAT[CG]ATCGATCTATCTCCACATCAGGGAG GCACATCAAGCCAGATGTTTAGGAACACAGTGTTT | GABRB1 | — | 86 |
| cg27351675 | TATGAGGAATTTGGGGCTCAGTTGAAAAGCCTAAACTGCCTCTC GGGAGGTTGGGCGCGG[CG]AACTACTTTCAGCGGCGCACGGAG ACGGCGTCTACGTGAGGGGTGATAAGTGACGCAACA | UBB | + | 87 |

33 CpG sites in brackets in the base sequences represented by SEQ ID NOs: 55 to 87 (hereinafter collectively referred to as "33 CpG sets" in some cases) have a largely different methylation rate between a subject group which has not developed colorectal cancer and a colorectal cancer patient group in comprehensive DNA methylation analysis in Example 2 as described later. Among these, colorectal cancer patients have a much lower methylation rate than subjects who have not developed colorectal cancer at the CpG sites ("-" in the tables) in the base sequences represented by SEQ ID NOs: 59, 65 to 68, 70 to 77, and 79 to 86, and colorectal cancer patients have a much higher methylation rate than subjects who have not developed colorectal cancer at the CpG sites ("+" in the tables) in the base sequences represented by SEQ ID NOs: 55 to 58, 60 to 64, 69, 78, and 87. The CpG site used as a marker is not limited to these 33 CpG sites and also includes other CpG sites in the base sequences represented by SEQ ID NOs: 55 to 87.

[Table 16]

| CpG ID | Base sequence | UCSC _REFGENE _NAME | +I | |
|--------|---------------|---------------------|-----|-----|
| cg01561758 | CCTCACTCTTGGATCACCATAAGAGTTGAGACAGCTGGG TCTGCAGGACATTGGAAAAGT[CG]GGTGTGCCTTCCTCT GTAGGGCCACCTGGGAAGGATACAGCTGTCTGCAAACCA TGATGT | – | + | 88 |
| cg06970370 | CGTCCTGCCCGCGGCACTGGCTGCGGGTGCCGGGCCAC CTGCGAGTGTGCGGAGGGATTC[CG]GACACCCGCGGCG GCGAGCTGAGGGAGCAGTCTCCACGAGAACTGAGGCGGA CCCTCTGG | LOC647121 | + | 89 |
| cg07973162 | GGATACCCAAGCAGCTCATTCCTGCCTGGCACCACAGTG ATCCTTTAGGAGGGTGGCCAG[CG]GAGCAGGGGGTTCAA AGATTCTTCTGGGGCCTGAAAGCTTGAAGGGATGAGTAA CTCCTC | UGT2B15; UGT2B17 | – | 90 |
| cg11792281 | AACACTGGCAGCACCTATTGAGGCCATGTTTCAGGATCA GACCATGCTGGTTTGAGCAGA[CG]CAGCAAGAGTGAGAA CCCCGGCCGAATTTTCATGGGTGGCTCTAGTAGAGCTGC TGGTGA | NLK | – | 91 |
| cg18500967 | AGCTGAAGAAACAGATGAGGAAGCACAGATAGTCTGGGA GGAGACACTCAAGCTTCCCAC[CG]GTGGCCACAGCACAC TCCATCCCTGGAAATACTGCAAACCAACCCCCCAGGAGC CCCGGG | – | + | 92 |
| cg23943944 | TATCCTCAACAAAACTGTAACAGGGAATCTATCTGTGTTC AGTGTTGCTCCCCTGAACAC[CG]TGCTCTTCACTCAGCC TTCACACCCCTCACATGGTATTCTATTTAAAAAAATAATA ATAA | – | + | 93 |

6 CpG sites in brackets in the base sequences represented by SEQ ID NOs: 88 to 93 (hereinafter collectively referred to as "6 CpG sets" in some cases) have a largely different methylation rate between a subject group which has not developed colorectal cancer and a colorectal cancer patient group in comprehensive DNA methylation analysis in Example 3 as described later. Among these, colorectal cancer patients have a much lower methylation rate than subjects who have not developed colorectal cancer at the CpG sites ("-" in the tables) in the base sequences represented by SEQ ID NOs: 90 and 91, and colorectal cancer patients have a much higher methylation rate than subjects who have not developed colorectal cancer at the CpG sites ("+" in the tables) in the base sequences represented by SEQ ID NOs: 88, 89, 92, and 93. The CpG site used as a marker is not limited to these 6 CpG sites and also includes other CpG sites in the base sequences represented by SEQ ID NOs: 88 to 93.

Regarding the respective CpG sites, reference values are previously set for identifying a colorectal cancer patient and a subject who has not developed colorectal cancer. For the CpG sites marked with "+" in Tables 8 to 12 among the 54 CpG sets, the CpG sites marked with "+" in Tables 13 to 15 among the 33 CpG sets, and the CpG sites marked with "+" in Table 16 among the 6 CpG sets, in a case where the measured methylation rate is equal to or higher than a preset reference value, it is determined that there is a high likelihood of sporadic colorectal cancer development in a human subject. For the CpG sites marked with "-" in Tables 8 to 12 among the 54 CpG sets, the CpG sites marked with "-" in Tables 13 to 15 among the 33 CpG sets, and the CpG sites marked with "+" in Table 16 among the 6 CpG sets, in a case where the measured methylation rate is equal to or lower than a preset reference value, it is determined that there is a high likelihood of sporadic colorectal cancer development in a human subject.

The reference value for each CpG site can be experimentally obtained as a threshold value capable of distinguishing between a colorectal cancer patient group and a subject group which has not developed colorectal cancer by measuring a methylation rate of the CpG site in both groups. Specifically, a reference value for methylation of any CpG site can be obtained by a general statistical technique. Examples thereof are shown below. However, ways of determining the reference value in the present invention are not limited to these.

As an example of a way of obtaining the reference value, for example, among human subjects, in patients (subjects who have not developed colorectal cancer) who are not diagnosed as having colorectal cancer by pathological examination using biopsy tissue in an endoscopic examination, DNA methylation of rectal mucosa is firstly measured for any CpG site. After performing measurement for a plurality of human subjects, a numerical value such as an average value or median value thereof which represents methylation of a group of these human subjects can be calculated and used as a reference value.

In addition, DNA methylation of rectal mucosa was measured for a plurality of subjects who have not developed colorectal cancer and a plurality of colorectal cancer patients, a numerical value such as an average value or a median value and a deviation which represent methylation of a colorectal cancer patient group and a subject group which has not developed colorectal cancer were calculated, respectively, and then a threshold value that distinguishes between both numerical values is obtained taking the deviations also into consideration, so that the threshold value can be used a reference value.

In the determination step, in a case where one or more among the CpG sites in the base sequences represented by SEQ ID NOs: 1, 4, 6, 10, 11, 13, 14, 17 to 20, 23 to 27, 29, 30, 32, 33, 35, 36, 39, 41 to 48, 50 to 54, 59, 65 to 68, 70 to 77, 79 to 86, 90, and 91 have a methylation rate of equal to or lower than a preset reference value, or one or more among the CpG sites in the base sequences represented by SEQ ID NOs: 2, 3, 5, 7 to 9, 12, 15, 16, 21, 22, 28, 31, 34, 37, 38, 40, 49, 55 to 58, 60 to 64, 69, 78, 87 to 89, 92, and 93 have a methylation rate of equal to or higher than a preset reference value, it is determined that there is a high likelihood of sporadic colorectal cancer development in the human subject. In the determination step according to the present invention, in a case where a sum of the number of CpG sites having a methylation rate equal to or lower than a preset reference value among the CpG sites in the base sequences represented by SEQ ID NOs: 1, 4, 6, 10, 11, 13, 14, 17 to 20, 23 to 27, 29, 30, 32, 33, 35, 36, 39, 41 to 48, 50 to 54, 59, 65 to 68, 70 to 77, 79 to 86, 90, and 91, and the number of CpG sites having a methylation rate equal to or higher than a preset reference value among the CpG sites in the base sequences represented by SEQ ID NOs: 2, 3, 5, 7 to 9, 12, 15, 16, 21, 22, 28, 31, 34, 37, 38, 40, 49, 55 to 58, 60 to 64, 69, 78, 87 to 89, 92, and 93 is two or more, preferably three or more, and more preferably five or more, it is determined that there is a high likelihood of sporadic colorectal cancer development in the human subject, which makes it possible to make a more accurate determination.

In a case of using the 54 CpG sets as markers in the present invention, that is, in a case of measuring methylation rates of the 54 CpG sets in the measurement step, in the determination step, in a case where one or more among the CpG sites in the base sequences represented by SEQ ID NOs: 1, 4, 6, 10, 11, 13, 14, 17 to 20, 23 to 27, 29, 30, 32, 33, 35, 36, 39, 41 to 48, and 50 to 54 have a methylation rate of equal to or lower than a preset reference value, or one or more among the CpG sites in the base sequences represented by SEQ ID NOs: 2, 3, 5, 7 to 9, 12, 15, 16, 21, 22, 28, 31, 34, 37, 38, 40, and 49 have a methylation rate of equal to or higher than a preset reference value, it is determined that there is a high likelihood of sporadic colorectal cancer development in the human subject. In the determination method according to the present invention, in a case where a sum of the number of CpG sites having a methylation rate equal to or lower than a preset reference value among the CpG sites in the base sequences represented by SEQ ID NOs: 1, 4, 6, 10, 11, 13, 14, 17 to 20, 23 to 27, 29, 30, 32, 33, 35, 36, 39, 41 to 48, and 50 to 54, and the number of CpG sites having a methylation rate equal to or higher than a preset reference value among the CpG sites in the base sequences represented by SEQ ID NOs: 2, 3, 5, 7 to 9, 12, 15, 16, 21, 22, 28, 31, 34, 37, 38, 40, and 49 is two or more, preferably three or more, and more preferably five or more, it is determined that there is a high likelihood of sporadic colorectal cancer development in the human subject, which makes it possible to make a more accurate determination.

In a case of using the 8 CpG sets as markers in the present invention, that is, in a case of measuring methylation rates of the 8 CpG sets in the measurement step, in the determination step, in a case where one or more among the CpG sites in the base sequences represented by SEQ ID NOs: 1, 4, and 6 have a methylation rate of equal to or lower than a preset reference value, or one or more among the CpG sites in the base sequences represented by SEQ ID NOs: 2, 3, 5, 7, and 8 have a methylation rate of equal to or higher than a preset reference value, it is determined that there is a high likelihood of sporadic colorectal cancer development in the human subject. In the determination method according to the present invention, in a case where a sum of the number of CpG sites having a methylation rate equal to or lower than a preset reference value among the CpG sites in the base sequences represented by SEQ ID NOs: 1, 4, and 6, and the number of CpG sites having a methylation rate equal to or higher than a preset reference value among the CpG sites in the base sequences represented by SEQ ID NOs: 2, 3, 5, 7, and 8 is two or more, preferably three or more, and more preferably five or more, it is determined that there is a high likelihood of sporadic colorectal cancer development in the human subject, which makes it possible to make a more accurate determination.

In a case of using the 33 CpG sets as markers in the present invention, that is, in a case of measuring methylation rates of the 33 CpG sets in the measurement step, in the determination step, in a case where one or more among the CpG sites in the base sequences represented by SEQ ID NOs: 59, 65 to 68, 70 to 77, and 79 to 86 have a methylation rate of equal to or lower than a preset reference value, or one or more among the CpG sites in the base sequences represented by SEQ ID NOs: 55 to 58, 60 to 64, 69, 78, and 87 have a methylation rate of equal to or higher than a preset reference value, it is determined that there is a high likelihood of sporadic colorectal cancer development in the human subject. In the determination method according to the present invention, in a case where a sum of the number of CpG sites having a methylation rate equal to or lower than a preset reference value among the CpG sites in the base sequences represented by SEQ ID NOs: 59, 65 to 68, 70 to 77, and 79 to 86, and the number of CpG sites having a methylation rate equal to or higher than a preset reference value among the CpG sites in the base sequences represented by SEQ ID NOs: 55 to 58, 60 to 64, 69, 78, and 87 is two or more, preferably three or more, and more preferably five or more, it is determined that there is a high likelihood of sporadic colorectal cancer development in the human subject, which makes it possible to make a more accurate determination.

In a case of using the 6 CpG sets as markers in the present invention, that is, in a case of measuring methylation rates of the 6 CpG sets in the measurement step, in the determination step, in a case where one or more among the CpG sites in the base sequences represented by SEQ ID NOs: 90 and 91 have a methylation rate of equal to or lower than a preset reference value, or one or more among the CpG sites in the base sequences represented by SEQ ID NOs: 88, 89, 92, and 93 have a methylation rate of equal to or higher than a preset reference value, it is determined that there is a high likelihood of sporadic colorectal cancer development in the human subject. In the determination method according to the present invention, in a case where a sum of the number of CpG sites having a methylation rate equal to or lower than a preset reference value among the CpG sites in the base sequences represented by SEQ ID NOs: 90 and 91, and the number of CpG sites having a methylation rate equal to or higher than a preset reference value among the CpG sites in the base sequences represented by SEQ ID NOs: 88, 89, 92, and 93 is two or more, preferably three or more, and more preferably five or more, it is determined that there is a high likelihood of sporadic colorectal cancer development in the human subject, which makes it possible to make a more accurate determination.

In the present invention, one or more CpG sites selected from the group consisting of CpG sites in the base sequences represented by SEQ ID NOs: 1 to 93 can be used as markers. As the CpG site used as a marker in the present invention, all 93 CpG sites (hereinafter collectively referred to as "93 CpG sets" in some cases) in brackets in the base sequences represented by SEQ ID NOs: 1 to 93 may be used, or the 54 CpG sets, the 8 CpG sets, the 33 CpG sets, or the 6 CpG sets may be used. The CpG site of the 54 CpG set and the CpG site of the 8 CpG set are excellent in that both sets show a small variance of methylation rate between a colorectal cancer patient group and a subject group which has not developed colorectal cancer and have a high ability to identify the colorectal cancer patient group and the subject group which has not developed colorectal cancer. On the other hand, the 33 CpG sets and the 6 CpG sets have somewhat lower specificity than the CpG sites of the 54 CpG sets and the CpG sites of the 8 CpG sets. However, the 33 CpG sets and the 6 CpG sets have very high sensitivity, and, for example, are very suitable for primary screening examination of sporadic colorectal cancer.

Among determination methods according to the present invention, the method for making a determination based on an average methylation rate value itself of a specific DMR is specifically a method for determining the likelihood of sporadic colorectal cancer development, the method including a measurement step of measuring methylation rates of one or more CpG sites present in the specific DMR used as markers in the present invention, in DNA recovered from a biological sample collected from the human subject, and a determination step of determining the likelihood of sporadic colorectal cancer development in the human subject based on an average methylation rate of the DMR calculated based on the methylation rates measured in the measurement step and a reference value previously set with respect to the average methylation rate of each DMR. The average methylation rate of each DMR is calculated as an average value of methylation rates of all CpG sites, for which a methylation rate has been measured in the measurement step, among the CpG sites in the DMR.

Specifically, the DMR used as a marker in the present invention is one or more DMR's selected from the group consisting of DMR's represented by DMR numbers 1 to 121. Chromosomal positions and corresponding genes of the respective DMR's are shown in Tables 17 to 23. Base positions of start and end points of DMR's in the tables are based on a data set "GRCh37/hg19" of the human genome sequence. A DNA fragment having a base sequence containing a CpG site present in these DMR's can be used as a DNA methylation rate analysis marker for determining the likelihood of sporadic colorectal cancer.

[Table 17]

| DMR no. | Gene Symbol | Ensembl ID | Chromosome no. | DMR start | DMR end | Width | ± |
|---|---|---|---|---|---|---|---|
| 1 | | | 17 | 46827397 | 46827628 | 232 | + |
| 2 | | ENST00000561259.1 | 15 | 37180595 | 37181182 | 588 | + |
| 3 | FADS2 | | 11 | 61596200 | 61596511 | 312 | + |
| 4 | SHF | ENST00000560734.1;ENST00000560471.1; ENST00000560540.1;ENST00000561091.1; ENST00000560034.1 | 15 | 45479648 | 45479861 | 214 | + |
| 5 | TDH | ENST00000525867.1;ENST00000534302.1 | 8 | 11203722 | 11205353 | 1632 | + |
| 6 | MYF6 | ENST00000228641.3 | 12 | 81102475 | 81103021 | 547 | + |
| 7 | SOX21; SOX21-AS1 | ENST00000438290.1; ENST00000376945.2 | 13 | 95364512 | 95364619 | 108 | + |
| 8 | RANBP9 | ENST00000469916.1 | 6 | 13633257 | 13635423 | 2167 | − |
| 9 | | ENST00000390750.1 | 1 | 97366188 | 97369696 | 3509 | − |
| 10 | EHBP1 | ENST00000516627.1 | 2 | 62953601 | 62956283 | 2683 | − |
| 11 | HECTD1 | ENST00000384709.1 | 14 | 31610929 | 31613066 | 2138 | − |
| 12 | | ENST00000440936.1 | 11 | 27911088 | 27914543 | 3456 | − |
| 13 | ASH1L | ENST00000384405.1 | 1 | 155327687 | 155330111 | 2425 | − |
| 14 | | ENST00000401135.1 | 11 | 112115998 | 112119870 | 3873 | − |
| 15 | | ENST00000562976.1 | 16 | 32609347 | 32612783 | 3437 | − |
| 16 | HOXA2 | ENST00000222718.5 | 7 | 27142503 | 27143294 | 792 | + |
| 17 | GNAL | ENST00000535121.1;ENST00000269162.4; ENST00000423027.2;ENST00000540217.1 | 18 | 11751996 | 11752178 | 183 | + |
| 18 | ARHGEF4 | ENST00000428230.2;ENST00000525839.1; ENST00000326016.5 | 2 | 131674106 | 131674191 | 86 | + |
| 19 | PCDHA7; PCDHA12; PCDHA6; PCDHAC1; PCDHA10; PCDHA4; PCDHA11; PCDHA8; PCDHA1; PCDHA2; PCDHA9; PCDHA13; PCDHA5; PCDHA3 | ENST00000253807.2; ENST00000409700.3 | 5 | 140306074 | 140306355 | 282 | + |
| 20 | FLJ45983 | ENST00000458727.1; ENST00000355358.1; ENST00000418270.1 | 10 | 8094324 | 8094640 | 317 | + |

[Table 18]

| DMR no. | Gene Symbol | Ensembl ID | Chromosome no. | DMR start | DMR end | Width | ± |
|---|---|---|---|---|---|---|---|
| 21 | ATF7IP2 | ENST00000396559.1;ENST00000561932.1; ENST00000543967.1 | 16 | 10479725 | 10480582 | 858 | + |
| 22 | | | 11 | 20617680 | 20618294 | 615 | + |
| 23 | DMRTA2 | ENST00000418121.1 | 1 | 50886813 | 50887075 | 263 | + |
| 24 | SEPT9 | ENST00000363781.1;ENST00000397613.4 | 17 | 75436513 | 75439186 | 2674 | + |
| 25 | TNFRSF25; PLEKHG5 | ENST00000348333.3;ENST00000377782.3; ENST00000356876.3;ENST00000400913.1; ENST00000489097.1 | 1 | 6525942 | 6526668 | 727 | + |
| 26 | FLJ32063 | ENST00000450728.1;ENST00000416200.1; ENST00000446911.1;ENST00000457245.1; ENST00000441234.1 | 2 | 200334170 | 200335332 | 1163 | + |
| 27 | DTX1 | ENST00000257600.3 | 12 | 113494374 | 113494471 | 98 | + |
| 28 | LYNX1 | ENST00000522906.1;ENST00000398906.1; ENST00000395192.2;ENST00000335822.5; ENST00000523332.1;ENST00000345173.6 | 8 | 143858547 | 143858706 | 160 | + |
| 29 | IZUMO1 | ENST00000332955.2 | 19 | 49250305 | 49250694 | 390 | + |
| 30 | | | 18 | 55095061 | 55095364 | 304 | + |
| 31 | AEBP2 | ENST00000360995.4;ENST00000541908.1 | 12 | 19593346 | 19593565 | 220 | + |
| 32 | | ENST00000406197.1 | 7 | 155284154 | 155284741 | 588 | + |
| 33 | ZNF542 | ENST00000490123.1 | 19 | 56879271 | 56879751 | 481 | + |
| 34 | LRRC43 | | 12 | 122651566 | 122651863 | 298 | + |
| 35 | ERCC6 | ENST00000374129.3;ENST00000539110.1; ENST00000542458.1 | 10 | 50696150 | 50698147 | 1998 | − |
| 36 | ACSM3 | ENST00000289416.5;ENST00000440284.2; ENST00000565498.1 | 16 | 20777186 | 20779229 | 2044 | − |
| 37 | WAPAL | ENST00000372075.1;ENST00000263070.7 | 10 | 88226215 | 88229444 | 3230 | − |
| 38 | HLA-E | ENST00000376630.4 | 6 | 30455709 | 30456000 | 292 | − |
| 39 | | ENST00000459557.1 | 6 | 114159118 | 114163406 | 4289 | − |
| 40 | | ENST00000486767.1 | 3 | 164402447 | 164406668 | 4222 | − |

[Table 19]

| DMR no. | Gene Symbol | Ensembl ID | Chromosome no. | DMR start | DMR end | Width | ± |
|---|---|---|---|---|---|---|---|
| 41 | BET1 | ENST00000471446.1;ENST00000426193.2; ENST00000426634.1 | 7 | 93625930 | 93628057 | 2128 | − |
| 42 | | | 6 | 14406829 | 14409842 | 3014 | − |
| 43 | ZNF323; ZKSCAN3 | ENST00000252211.2;ENST00000341464.5; ENST00000396838.2;ENST00000414429.1 | 6 | 28320486 | 28323328 | 2843 | − |
| 44 | MTMR3 | ENST00000384724.1;ENST00000401950.2; ENST00000333027.3;ENST00000323630.5; ENST00000351488.3;ENST00000415511.1 | 22 | 30295038 | 30296772 | 1735 | − |
| 45 | SH3YL1 | ENST00000403657.1;ENST00000468321.1; ENST00000403658.1 | 2 | 252349 | 255227 | 2879 | − |
| 46 | | ENST00000455502.1 | 7 | 93472562 | 93475664 | 3103 | − |
| 47 | | ENST00000555070.1 | 14 | 90167165 | 90167752 | 588 | − |
| 48 | | | 8 | 1404844 | 1405431 | 588 | − |
| 49 | TFDP2 | ENST00000383877.1;ENST00000489671.1; ENST00000464782.1;ENST00000317104.7; ENST00000467072.1;ENST00000499676.2 | 3 | 141863017 | 141865101 | 2085 | − |
| 50 | TMEM106B | | 7 | 12268344 | 12270783 | 2440 | − |
| 51 | | ENST00000364882.1 | 4 | 117758275 | 117761934 | 3660 | − |
| 52 | SLC20A2 | ENST00000520262.1;ENST00000520179.1; ENST00000342228.3 | 8 | 42357666 | 42360957 | 3292 | − |
| 53 | | | 1 | 47910065 | 47911801 | 1737 | + |
| 54 | STK32B | ENST00000282908.5 | 4 | 5053444 | 5053551 | 108 | + |
| 55 | SOX2OT; SOX2 | ENST00000498731.1;ENST00000431565.2; ENST00000325404.1 | 3 | 181427354 | 181428928 | 1575 | + |
| 56 | SOX2OT | ENST00000498731.1 | 3 | 181437890 | 181438559 | 670 | + |
| 57 | CLIP4 | ENST00000320081.5;ENST00000379543.5; ENST00000401605.1;ENST00000401617.2; ENST00000404424.1 | 2 | 29337848 | 29338142 | 295 | + |

[Table 20]

| DMR no. | Gene Symbol | Ensembl ID | Chromosome no. | DMR start | DMR end | Width | +/− |
|---|---|---|---|---|---|---|---|
| 58 | | | 5 | 2038695 | 2039282 | 588 | + |
| 59 | SHISA9 | ENST00000423335.2;ENST00000482916.1;ENST00000558318.1;ENST00000424107.3 | 16 | 12995279 | 12995656 | 378 | + |
| 60 | | ENST00000364275.1 | 4 | 190938593 | 190938935 | 343 | + |
| 61 | | | 16 | 73096548 | 73097135 | 588 | + |
| 62 | TTYH1 | ENST00000391739.3;ENST00000376531.3;ENST00000301194.4;ENST00000376530.3 | 19 | 54926333 | 54927197 | 865 | + |
| 63 | PHACTR1 | ENST00000379350.1;ENST00000399446.2;ENST00000334971.6 | 6 | 13273152 | 13275352 | 2201 | + |
| 64 | DAB1 | ENST00000371236.1;ENST00000371234.4;ENST00000485760.1 | 1 | 58715419 | 58715632 | 214 | + |
| 65 | | ENST00000558382.1;ENST00000558499.1 | 15 | 96905928 | 96910011 | 4084 | + |
| 66 | ZNF382; ZNF529 | ENST00000423582.1;ENST00000460670.1;ENST00000292928.2;ENST00000439428.1 | 19 | 37096052 | 37096201 | 150 | + |
| 67 | SOX2OT; SOX2-OT | ENST00000498731.1 | 3 | 181440653 | 181444202 | 3550 | + |
| 68 | CPEB1; CPEB1-AS1 | ENST00000560650.1;ENST00000450751.2;ENST00000568757.1;ENST00000563519.1 | 15 | 83316116 | 83316484 | 369 | + |
| 69 | EVC2 | ENST00000344938.1;ENST00000310917.2 | 4 | 5710239 | 5710490 | 252 | + |
| 70 | C2orf74 | ENST00000426997.1;ENST00000420918.1 | 2 | 61372150 | 61372361 | 212 | + |
| 71 | DPYSL3 | ENST00000343218.5;ENST00000504965.1 | 5 | 146889149 | 146889390 | 242 | + |
| 72 | PENK; LOC101929415 | ENST00000518662.1;ENST00000523274.1;ENST00000523051.1;ENST00000518770.1;ENST00000539312.1;ENST00000451791.2;ENST00000314922.3 | 8 | 57358624 | 57358800 | 177 | + |

[Table 21]

| DMR no. | Gene Symbol | Ensembl ID | Chromosome no. | DMR start | DMR end | Width | ± |
|---|---|---|---|---|---|---|---|
| 73 | GJD2; LOC101928174 | ENST00000503496.1;ENST00000290374.4 | 15 | 35047146 | 35047453 | 308 | + |
| 74 | ADAMTS16 | ENST00000512155.1;ENST00000511368.1 | 5 | 5139810 | 5139920 | 111 | + |
| 75 | FAM159B | ENST00000512767.1 | 5 | 63986626 | 63986899 | 274 | + |
| 76 | KCNA4 | ENST00000526518.1;ENST00000328224.6 | 11 | 30038649 | 30038734 | 86 | + |
| 77 | IRX5 | ENST00000447390.2;ENST00000560487.1; ENST00000560154.1;ENST00000558597.1; ENST00000394636.4 | 16 | 54967579 | 54969439 | 1861 | + |
| 78 | BCAT1 | ENST00000538118.1;ENST00000544418.1; ENST00000539282.1 | 12 | 25055964 | 25056233 | 270 | + |
| 79 | SOX11 | ENST00000322002.3;ENST00000455579.1 | 2 | 5836177 | 5836284 | 108 | + |
| 80 | CHL1 | ENST00000452919.1;ENST00000444879.1; ENST00000489224.1;ENST00000256509.2; ENST00000397491.2 | 3 | 239108 | 239308 | 201 | + |
| 81 | FAM115A; TCAF1 | ENST00000392900.3;ENST00000355951.2; ENST00000479870.1 | 7 | 143578766 | 143581048 | 2283 | + |
| 82 | | ENST00000551875.1 | 12 | 115172454 | 115173299 | 846 | + |
| 83 | | | 17 | 46831196 | 46831783 | 588 | + |
| 84 | NR5A2 | | 1 | 200003863 | 200004690 | 828 | + |
| 85 | UTF1 | ENST00000304477.2 | 10 | 135043449 | 135043550 | 102 | + |
| 86 | ATP10A | ENST00000553577.1;ENST00000356865.6 | 15 | 26107150 | 26108725 | 1576 | + |
| 87 | LOC283999; TMEM235 | ENST00000374946.3;ENST00000550981.2 | 17 | 76227764 | 76228227 | 464 | + |
| 88 | ZNF177 | ENST00000343499.3;ENST00000541595.1; ENST00000446085.2 | 19 | 9473642 | 9473768 | 127 | + |
| 89 | | | 6 | 107809023 | 107809834 | 812 | + |
| 90 | NR2E1 | ENST00000368986.4 | 6 | 108492410 | 108493000 | 591 | + |
| 91 | CDO1 | ENST00000250535.4;ENST00000502631.1 | 5 | 115152332 | 115152439 | 108 | + |
| 92 | CASR | ENST00000498619.1;ENST00000490131.1 | 3 | 121902936 | 121903190 | 255 | + |

[Table 22]

| DMR no. | Gene Symbol | Ensembl ID | Chromosome no. | DMR start | DMR end | Width | +I |
|---|---|---|---|---|---|---|---|
| 93 | PCDHGA4; PCDHGA11; PCDHGA9; PCDHGA1; PCDHGB1; PCDHGB6; PCDHGA12; PCDHGB3; PCDHGB7; PCDHGA6; PCDHGA8; PCDHGA10; PCDHGA5; PCDHGB4; PCDHGA3; PCDHGA2; PCDHGB2; PCDHGA7; PCDHGB5 | ENST00000252085.3 | 5 | 140809819 | 140810664 | 846 | + |
| 94 | OCA2 | ENST00000353809.5;ENST00000354638.3 | 15 | 28344617 | 28344827 | 211 | + |
| 95 | LINC01248; SOX11 | ENST00000420221.1;ENST00000453678.1; ENST00000458264.1;ENST00000322002.3 | 2 | 5830853 | 5831440 | 588 | + |
| 96 | GDF7 | ENST00000272224.3 | 2 | 20871066 | 20871694 | 629 | + |
| 97 | SOX8 | ENST00000562570.1;ENST00000568394.1; ENST00000565467.1;ENST00000563863.1; ENST00000565069.1;ENST00000563837.1; ENST00000293894.3 | 16 | 1030543 | 1030628 | 86 | + |
| 98 | NEFM | ENST00000221166.5;ENST00000433454.2; ENST00000518131.1;ENST00000521540.1 | 8 | 24771213 | 24771326 | 114 | + |
| 99 | | ENST00000560487.1 | 16 | 54970835 | 54971133 | 299 | + |
| 100 | PTGFRN | ENST00000544471.1;ENST00000393203.2 | 1 | 117528415 | 117531212 | 2798 | + |
| 101 | STAC | ENST00000273183.3;ENST00000457375.2; ENST00000476388.1;ENST00000544687.1 | 3 | 36422165 | 36422637 | 473 | + |
| 102 | | | 12 | 81106709 | 81109314 | 2606 | + |
| 103 | HBQ1 | ENST00000199708.2 | 16 | 230287 | 230396 | 110 | + |
| 104 | | | 6 | 85484569 | 85485156 | 588 | + |

[Table 23]

| DMR no. | Gene Symbol | Ensembl ID | Chromosome no. | DMR start | DMR end | Width | ± |
|---|---|---|---|---|---|---|---|
| 105 | NPR3 | ENST00000434067.2;ENST00000415685.2 | 5 | 32708777 | 32709689 | 913 | + |
| 106 | NMBR | ENST00000258042.1;ENST00000454401.1 | 6 | 142410081 | 142410276 | 196 | + |
| 107 | KCNIP1 | ENST00000411494.1;ENST00000328939.4; ENST00000390656.4;ENST00000520740.1 | 5 | 169931309 | 169931416 | 108 | + |
| 108 | ZNF835 | ENST00000537055.1 | 19 | 57183011 | 57183374 | 364 | + |
| 109 | SALL3 | ENST00000575722.1;ENST00000573860.1; ENST00000537592.2 | 18 | 76740075 | 76740337 | 263 | + |
| 110 | CCNA1 | ENST00000418263.1;ENST00000255465.4; ENST00000440264.1 | 13 | 37006053 | 37006793 | 741 | + |
| 111 | NR3C1 | ENST00000504336.1;ENST00000416954.2 | 5 | 142768792 | 142771780 | 2989 | − |
| 112 | STX19; ARL13B | ENST00000315099.2;ENST00000539730.1; ENST00000486562.1 | 3 | 93746411 | 93748870 | 2460 | − |
| 113 | NFIB | ENST00000493697.1 | 9 | 14307151 | 14309148 | 1998 | − |
| 114 | | ENST00000510419.1 | 4 | 75513579 | 75517080 | 3502 | − |
| 115 | TRIM9 | ENST00000554475.1 | 14 | 51554159 | 51556518 | 2360 | − |
| 116 | PIBF1 | ENST00000362511.1 | 13 | 73455494 | 73457491 | 1998 | − |
| 117 | | ENST00000468232.1 | 3 | 170126475 | 170129488 | 3014 | − |
| 118 | LOC101060498 | ENST00000510551.1 | 4 | 40316101 | 40318304 | 2204 | − |
| 119 | RNU6−2 | ENST00000384716.1 | 10 | 13257430 | 13260736 | 3307 | − |
| 120 | EFNB2 | | 13 | 107181847 | 107183783 | 1937 | − |
| 121 | ARG1 | ENST00000368087.3;ENST00000356962.2; ENST00000476845.1;ENST00000489091.1 | 6 | 131893339 | 131893636 | 298 | − |

DMR's represented by DMR numbers 1 to 121 (hereinafter collectively referred to as "121 DMR sets" in some cases) have a largely different methylation rate of a plurality of CpG sites contained in each region between a subject group which has not developed colorectal cancer and a colorectal cancer patient group. Among these, colorectal cancer patients have a much lower average methylation rate of DMR (average value of methylation rates of a plurality of CpG sites present in DMR) than subjects who have not developed colorectal cancer at DMR's ("-" in the tables) represented by DMR numbers 8 to 15, 35 to 52, and 111 to 121, and colorectal cancer patients have a much higher average methylation rate of DMR than subjects who have not developed colorectal cancer at DMR's ("+" in the tables) represented by DMR numbers 1 to 7, 16 to 34, and 53 to 110.

In the present invention, in a case where the average methylation rate of DMR is used as a marker, one of DMR's represented by DMR numbers 1 to 121 may be used as a marker, any two or more selected from the group consisting of DMR's represented by DMR nos. 1 to 121 may be used as markers, or all of the DMR's represented by DMR numbers 1 to 121 may be used as markers. In the present invention, from the viewpoint of further increasing determination accuracy, the number of DMR's used as a marker among DMR's represented by DMR numbers 1 to 121 is preferably two or more, more preferably three or more, even more preferably four or more, and still more preferably five or more.

From the viewpoint of obtaining further increased determination accuracy, the DMR whose methylation rate is used as a marker in the present invention is preferably one or more selected from the group consisting of DMR's represented by DMR numbers 1 to 52 (hereinafter collectively referred to as "52 DMR sets" in some cases), more preferably two or more selected from the 52 DMR sets, even more preferably three or more selected from the 52 DMR sets, still more preferably four or more selected from the 52 DMR sets, and particularly preferably five or more selected from the 52 DMR sets. Among these, one or more selected from the group consisting of DMR's represented by DMR numbers 1 to 15 (hereinafter collectively referred to as "15 DMR sets" in some cases) are preferable, two or more selected from 15 DMR sets are more preferable, three or more selected from the 15 DMR sets are even more preferable,

34

four or more selected from the 15 DMR sets is still more preferable, and five or more selected from the 15 DMR sets is particularly preferable.

An average methylation rate of each DMR may be an average value of methylation rates of all CpG sites contained in each DMR or may be an average value obtained by selecting, in a predetermined manner, at least one CpG site from all CpG sites contained in each DMR and averaging methylation rates of the selected CpG sites. A methylation rate of each CpG site can be measured in the same manner as the measurement of a methylation rate of a CpG site in the base sequences represented by SEQ ID NO: 1 and the like in Tables 8 to 16.

Regarding the average methylation rate of each DMR, a reference value is previously set for identifying a colorectal cancer patient and a subject who has not developed colorectal cancer. For the DMR's marked with "+" in Tables 17 to 23 among the 121 DMR sets, in a case where the measured average methylation rate of the DMR is equal to or higher than a preset reference value, it is determined that there is a high likelihood of sporadic colorectal cancer development in a human subject. For the DMR's marked with "-" in Tables 17 to 23 among the 121 DMR sets, in a case where the measured average methylation rate of the DMR is equal to or lower than a preset reference value, it is determined that there is a high likelihood of sporadic colorectal cancer development in a human subject.

The reference value for the average methylation rate of each DMR can be experimentally obtained as a threshold value capable of distinguishing between a subject group which has developed colorectal cancer and a non-colorectal cancer patient group by measuring an average methylation rate of the DMR in both groups. Specifically, a reference value for an average methylation rate of DMR can be obtained by a general statistical technique.

In a case where methylation rates of CpG sites such as the 93 CpG sets are used as markers, in the determination method according to the present invention, it is possible to determine the likelihood of sporadic colorectal cancer development in the human subject based on the methylation rates measured in the measurement step and a preset multivariate discrimination expression, in the determination step. The multivariate discrimination expression includes, as variables, methylation rates of one or more CpG sites among CpG sites in the base sequences represented by SEQ ID NOs: 1 to 93.

In a case where average methylation rates of one or more DMR's selected from the group consisting of the 121 DMR sets are used as markers, in the determination method according to the present invention, it is possible to determine the likelihood of sporadic colorectal cancer development in the human subject based on an average methylation rate of DMR calculated based on the methylation rates measured in the measurement step and a preset multivariate discrimination expression, in the determination step. The multivariate discrimination expression includes, as variables, methylation rates of one or more CpG sites among CpG sites in the 121 DMR sets.

The multivariate discrimination expression used in the present invention can be obtained by a general technique used for discriminating between two groups. As the multivariate discrimination expression, a logistic regression expression, a linear discrimination expression, an expression created by Naive Bayes classifier, or an expression created by Support Vector Machine are mentioned, but not limited thereto. For example, these multivariate discrimination expressions can be created using an ordinary method by measuring a methylation rate of one CpG site or two or more CpG sites among CpG sites in the base sequences represented by SEQ ID NOs: 1 to 93 with respect to a colorectal cancer patient group and a subject group which has not developed colorectal cancer, and using the obtained methylation rate as a variable. In addition, these multivariate discrimination expressions can be created using an ordinary method by measuring an average methylation rate of one DMR or two or more DMR's among the DMR's in the 121 DMR sets with respect to a colorectal cancer patient group and a non-colorectal cancer patient, and using the obtained methylation rate as a variable.

In the multivariate discrimination expression used in the present invention, a reference discrimination value for identifying a colorectal cancer patient and a subject who has not developed colorectal cancer is previously set. The reference discrimination value can be experimentally obtained as a threshold value capable of distinguishing between a colorectal cancer patient group and a subject group which has not developed colorectal cancer by obtaining a discrimination value which is a value of a multivariate discrimination expression used with respect to both groups and making a comparison for the discrimination value of the colorectal cancer patient group and the discrimination value of the subject group which has not developed colorectal cancer.

In a case of making a determination using a multivariate discrimination expression, specifically, in the measurement step, a methylation rate of a CpG site or an average methylation rate of DMR which is included as a variable in the multivariate discrimination expression used is measured, and in the determination step, a discrimination value which is a value of the multivariate discrimination expression is calculated based on the methylation rate measured in the measurement step and the multivariate discrimination expression, and, based on the discrimination value and a preset reference discrimination value, it is determined whether the likelihood of sporadic colorectal cancer development in a human subject in whom the methylation rate of the CpG site or the average methylation rate of the DMR is measured is high or low. In a case where the discrimination value is equal to or higher than the preset reference discrimination value, it is determined that the likelihood of sporadic colorectal cancer development in a human subject is high.

The multivariate discrimination expression used in the present invention is preferably an expression including, as variables, methylation rates of one or more CpG sites selected from the group consisting of the 33 CpG sites, more preferably an expression including, as variables, only methylation rates of one or more CpG sites selected from the group consisting of the 33 CpG sites, even more preferably an expression including, as variables, only methylation rates of 2 to 10 CpG sites optionally selected from the group consisting of the 33 CpG sites, and still more preferably an expression including, as variables, only methylation rates of 2 to 5 CpG sites optionally selected from the group consisting of the 33 CpG sites.

The multivariate discrimination expression used in the present invention is preferably an expression including, as variables, methylation rates of one or more CpG sites selected from the group consisting of the 6 CpG sites, more preferably an expression including, as variables, only methylation rates of one or more CpG sites selected from the group consisting of the 6 CpG sites, even more preferably an expression including, as variables, only methylation rates of 2 to 6 CpG sites optionally selected from the group consisting of the 6 CpG sites, and still more preferably an expression including, as variables, only methylation rates of 2 to 5 CpG sites optionally selected from the group consisting of the 6 CpG sites.

For CpG sites constituting the 33 CpG sets and the 6 CpG sets, even in a case where 2 to 10 (2 to 6 in a case of the 6 CpG sets), and preferably 2 to 5 CpG sites are optionally selected from these sets and only the selected CpG sites are used, it is possible to determine the likelihood of sporadic colorectal cancer development with sufficient sensitivity and specificity. For example, as shown in Example 2 as described later, in a case where among the 33 CpG sets, the three CpG sites of the CpG site in the base sequence represented by SEQ ID NO: 57, the CpG site in the base sequence represented by SEQ ID NO: 63, and the CpG site in the base sequence represented by SEQ ID NO: 77 are used as markers, and a multivariate discrimination expression created by logistic regression using methylation rates of the three CpG sites as variables is used, it is possible to determine the likelihood of sporadic colorectal cancer development with sensitivity of about 95% and specificity of about 96%. In a case where the number of CpG sites for which a methylation rate is measured is large in a clinical examination or the like, labor and cost may be excessive. By choosing a CpG site used as a marker from CpG sites constituting the 33 CpG sets and the 6 CpG sets, it is possible to accurately determine the likelihood of sporadic colorectal cancer development using a reasonable number of CpG sites of 1 or 2 to 10 which are measurable in a clinical examination.

The multivariate discrimination expression used in the present invention is preferably an expression including, as variables, average methylation rates of one or more DMR's selected from the group consisting of the 121 DMR sets as described above, more preferably an expression including, as variables, only average methylation rates of two or more DMR's selected from the group consisting of the 121 DMR sets as described above, even more preferably an expression including, as variables, only average methylation rates of three or more DMR's optionally selected from the group consisting of the 121 DMR sets as described above, still more preferably an expression including, as variables, only average methylation rates of four or more DMR's optionally selected from the group consisting of the 121 DMR sets as described above, and particularly preferably an expression including, as variables, only average methylation rates of five or more DMR's optionally selected from the group consisting of the 121 DMR sets as described above. Among these, an expression including, as variables, average methylation rates of one or more DMR's selected from the group consisting of the 52 DMR sets as described above is preferable, an expression including, as variables, only average methylation rates of two or more DMR's selected from the group consisting of the 52 DMR sets as described above is more preferable, an expression including, as variables, only average methylation rates of 2 to 10 DMR's optionally selected from the group consisting of the 52 DMR sets as described above is even more preferable, an expression including, as variables, only average methylation rates of 3 to 10 DMR's optionally selected from the group consisting of the 52 DMR sets as described above is still more preferable, and an expression including, as variables, only average methylation rates of 5 to 10 DMR's optionally selected from the group consisting of the 52 DMR sets as described above is particularly preferable. More preferably, an expression including, as variables, average methylation rates of one or more DMR's selected from the group consisting of the 15 DMR sets as described above is preferable, an expression including, as variables, only average methylation rates of two or more DMR's selected from the group consisting of the 15 DMR sets as described above is more preferable, an expression including, as variables, only average methylation rates of 2 to 10 DMR's optionally selected from the group consisting of the 15 DMR sets as described above is even more preferable, an expression including, as variables, only average methylation rates of 3 to 10 DMR's optionally selected from the group consisting of the 15 DMR sets as described above is still more preferable, and an expression including, as variables, only average methylation rates of 5 to 10 DMR's optionally selected from the group consisting of the 15 DMR sets as described above is particularly preferable.

A biological sample to be subjected to the determination method according to the present invention is not particularly limited as long as the biological sample is collected from a human subject and contains a genomic DNA of the subject. The biological sample may be blood, plasma, serum, tears, saliva, or the like, or may be mucosa of the gastrointestinal tract or a piece of tissue collected from other tissue such as the liver. As the biological sample to be subjected to the determination method according to the present invention, large intestinal mucosa is preferable from the viewpoint of strongly reflecting a state of the large intestine, and rectal mucosa is more preferable from the viewpoint of being collectible in a relatively less invasive manner. In a case where the biological sample is collected from body fluid such as the blood, the piece of tissue, large intestine mucosa, or rectal mucosa, collection may be achieved by using a collection tool corresponding to each biological sample.

In addition, it is sufficient that the biological sample is in a state in which DNA can be extracted. The biological sample may be a biological sample that has been subjected to various pretreatments. For example, the biological sample may be formalin-fixed paraffin-embedded (FFPE) tissue. Extraction of DNA from the biological sample can be carried out by an ordinary method, and various commercially available DNA extraction/purification kits can also be used.

A method for measuring a methylation rate of a CpG site is not particularly limited as long as the method is capable of distinguishing and quantifying a methylated cytosine base and a non-methylated cytosine base with respect to a specific CpG site. A methylation rate of a CpG site can be measured using a method known in the art as it is or with appropriate modification as necessary. As the method for measuring a methylation rate of a CpG site, for example, a bisulfite sequencing method, a combined bisulfite restriction analysis (COBRA) method, a quantitative analysis of DNA methylation using real-time PCR (qAMP) method, and the like are mentioned. Alternatively, the method may be performed using a microarray-based integrated analysis of methylation by isoschizomers (MIAM) method.

<Kit for collecting large intestinal mucosa >

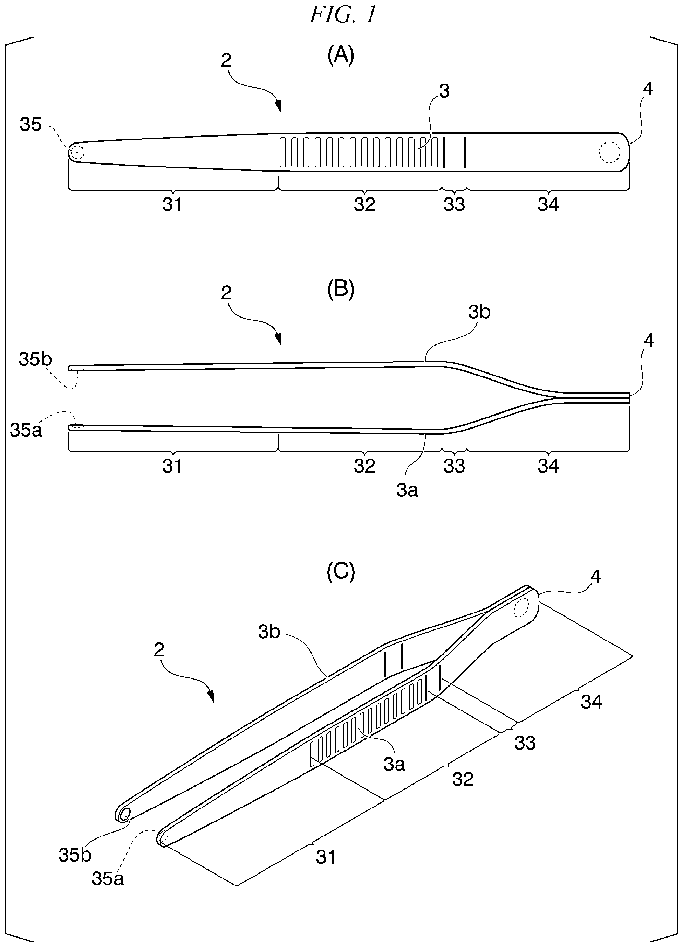

A kit for collecting large intestinal mucosa according to the present invention includes a collection tool for clamping and collecting rectal mucosa and a collection auxiliary tool for expanding the anus and allowing the collection tool to reach a surface of large intestinal mucosa from the anus. Hereinafter, referring to FIGS. 1 to 3, the kit for collecting large intestinal mucosa according to the present invention will be described.

FIGS. 1(A) to 1(C) are explanatory views of an embodiment of a collection tool 2 of a kit 1 for collecting large intestinal mucosa. FIG. 1(A) is a front view showing a state in which force is not applied to a first clamping piece 3a and a second clamping piece 3b of the collection tool 2, FIG. 1(B) is a plan view showing a state in which force is applied to the first clamping piece 3a and the second clamping piece 3b of the collection tool 2, and FIG. 1(C) is a perspective view showing a state in which force is not applied to the first clamping piece 3a and the second clamping piece 3b of the collection tool 2. As shown in FIG. 1, the collection tool 2 includes the first clamping piece 3a and the second clamping piece 3b which are a pair of elastic plate-like bodies. The first clamping piece 3a is configured to have a clamping portion 31a, a gripping portion 32a, a spring portion 33a, and a fixing portion 34a, and the second clamping piece 3b is configured to have a clamping portion 31b, a gripping portion 32b, a spring portion 33b, and a fixing portion 34b. A shape of the first clamping piece 3a and the second clamping piece 3b may be a rod shape in addition to a plate shape, and there is no limitation on the shape as long as the shape has a certain length for clamping and collecting rectal mucosa. In addition, a material is also not particularly limited as long as the material is an elastic body, and the material may be a metal such as stainless steel or a resin. The collection tool 2 is preferably a metal from the viewpoint that overlapping of the first clamping piece 3a and the second clamping piece 3b in a state in which force is applied is stabilized, and large intestinal mucosa is more easily collected.

The first clamping piece 3a and the second clamping piece 3b are connected and fixed to each other in a mutually opposed state on the fixing portion 34a and the fixing portion 34b. A method of performing the connection and fixing is not particularly limited, and for example, both clamping pieces can be connected and fixed to each other by welding ends of the fixing portion 34a and the fixing portion 34b so that the first clamping piece 3a and the second clamping piece 3b overlap with each other.

A length of the fixing portion 34a and the fixing portion 34b is not particularly limited, and is preferably 20 to 50 mm and more preferably 30 to 40 mm. In a case where the length of the fixing portion is within the above-mentioned range, it is easy to connect and fix both clamping pieces, and it is possible to impart sufficient strength against application of force.

In the first clamping piece 3a, a spring portion 33a having elasticity is provided between the gripping portion 32a and the fixing portion 34a. In the second clamping piece 3b, a spring portion 33b having elasticity is provided between the gripping portion 32b and the fixing portion 34b. In a case where force is applied by the spring portion 33a and the spring portion 33b so that the first clamping piece 3a and the second clamping piece 3b get closer to each other, an end of the clamping portion 31a and an end of the clamping portion 31b can be bonded to each other.

A length of the spring portion 33a and the spring portion 33b is not particularly limited, and is preferably 2 to 10 mm and more preferably 3 to 7 mm. In a case where the length of the spring portion is within the above-mentioned range, sufficient elasticity can be easily applied to both clamping pieces.

In the first clamping piece 3a, there is the gripping portion 32a between the clamping portion 31a and the spring portion 33a. In the second clamping piece 3b, there is the gripping portion 32b between the clamping portion 31b and the spring portion 33b. Back surfaces (surfaces to be gripped by a person who collects large intestinal mucosa) of a surface of the gripping portion 32a against the gripping portion 32b and a surface of the gripping portion 32b against the gripping portion 32a may be subjected to anti-slipping processing so that no slipping occurs in a case of being gripped by a person (a person who collects large intestinal mucosa). The anti-slipping processing is not particularly limited, and, for example, a resin-like anti-slipping portion may be separately attached to a metallic gripping portion, or applying a rough pattern or the like such as jagged pattern, a wedge-like pattern, or a rough surface of sandpaper can be mentioned. As the anti-slipping processing, as shown in FIG. 1(A), processing of providing a plurality of protrusions or recesses substantially parallel to each other in a width direction so as to form a jagged pattern is performed.

A length of the gripping portion 32a and the gripping portion 32b is preferably 20 to 50 mm, and more preferably 30 to 40 mm. In a case where the length of the gripping portion is within the above-mentioned range, it becomes easier to achieve gripping and apply force to both clamping pieces.

In the first clamping piece 3a, a clamping surface 35a for clamping large intestinal mucosa is formed on an end portion of a surface of the clamping portion 31a facing the second clamping piece 3b. In the second clamping piece 3b, a clamping surface 35b for clamping large intestinal mucosa is formed on an end portion of a surface of the clamping portion 31b facing the first clamping piece 3a. The clamping surface 35a and the clamping surface 35b are provided so as to be in close contact with each other on least at side edge portions thereof in a state in which an end portion of the clamping portion 31a and an end portion of the clamping portion 31b are bonded to each other due to application of force to the first clamping piece 3a and the second clamping piece 3b.

Due to application of force to the first clamping piece 3a and the second clamping piece 3b, the two pieces come close to each other. Therefore, in a state in which the clamping surface 35a and the clamping surface 35b of the collection tool 2 are in contact with large intestinal mucosa, by applying force to the first clamping piece 3a and the second clamping piece 3b, it is possible to clamp the large intestinal mucosa with the clamping surface 35a and the clamping surface 35b. More specifically, a side edge portion of the clamping surface 35a and a side edge portion of the clamping surface 35b come into contact with each other in a state in which the large intestinal mucosa is clamped therebetween. By separating the collection tool 2 from the large intestinal mucosa in this state, the large intestinal mucosa clamped between the clamping surface 35a and the clamping surface 35b is torn off and collected.

At least one of the clamping surface 35a and the clamping surface 35b is preferably provided with a recess in order to collect the large intestinal mucosa in a state in which damage to tissue is relatively small. Due to being a case where at least one of both surfaces is cup-shaped, a space is formed inside in a case where a side edge portion of the clamping surface 35a and a side edge portion of the clamping surface 35b come into contact with each other. Among the large intestinal mucosa clamped between the clamping surface 35a and the clamping surface 35b, a portion housed in the space is not subjected to much load in a case where the large intestinal mucosa is torn off, so that destruction of tissue can be suppressed. A shape of the recess is not particularly limited, and the recess may be, for example, cup-shaped (hemisphere-shaped). Both clamping surface 35a and clamping surface 35b are provided with the recess, which makes it easier to collect the large intestinal mucosa and makes it possible to suppress destruction of tissue.

In a case where the recess is formed in the clamping surface 35a and the clamping surface 35b, an inner diameter of the recess may be set to such a size that a necessary amount of large intestinal mucosa can be collected. In a case of large intestinal mucosa to be subjected to the determination method according to the present invention, it is sufficient to have a size such that a small amount of mucosa can be collected. For example, by setting an inner diameter of the recess of the clamping surface 35a and the clamping surface 35b to 1 to 5 mm and preferably 2 to 3 mm, it is possible to collect a sufficient amount of large intestinal mucosa without excessively damaging the large intestinal mucosa.

It is sufficient that the side edge portion of the clamping surface 35a and the side edge portion of the clamping surface 35b can come into close contact with each other. The side edge portions may be flat or serrated. In a case of being serrated, the large intestinal mucosa can be cut and collected with a relatively weak force by being clamped between the side edge portion of the clamping surface 35a and the side edge portion of the clamping surface 35b.

A width of the first clamping piece 3a and the second clamping piece 3b is such that, in order to easily achieve gripping, a width of a part from the gripping portion to the fixing portion is preferably 5 to 15 mm, and more preferably 6 to 10 mm. On the other hand, a width of the clamping portions in the first clamping piece 3a and the second clamping piece 3b is preferably narrowed toward the end portions where the clamping surfaces are provided, from the viewpoint that large intestinal mucosa can be collected with a smaller force. A width of the end portions of the first clamping piece 3a and the second clamping piece 3b can be, for example, 2 to 6 mm, and preferably 3 to 4 mm, while being made larger than the above-mentioned recess.

A length of the clamping portion 31a and the clamping portion 31b is preferably 20 to 60 mm, and more preferably 30 to 50 mm. By setting the clamping portion to be within the above-mentioned range, it becomes easier to collect mucosa in a state of penetrating a slit 13 of the collection auxiliary tool 11.

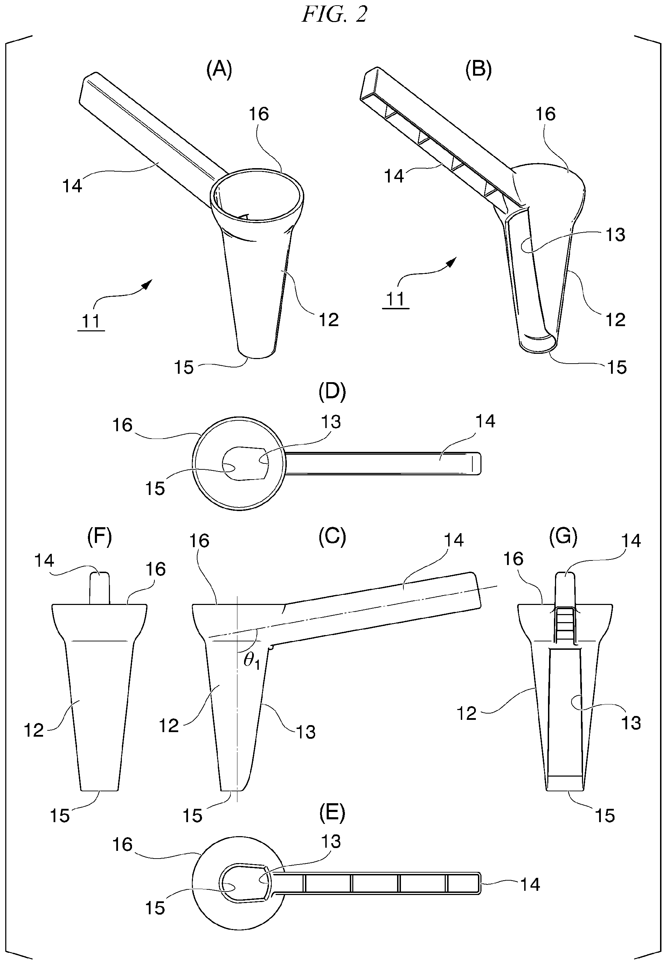

FIG. 2 is an explanatory view of an embodiment of the collection auxiliary tool 11. FIG. 2(A) is a perspective view as seen from an upper side of the collection auxiliary tool 11, and FIG. 2(B) is a perspective view as seen from a lower side thereof. In addition, FIGS. 2(C) to 2(G) are a front view, a plan view, a bottom view, a left side view, and a right side view of the collection auxiliary tool 11, respectively. As shown in FIG. 2, the collection auxiliary tool 11 has a collection tool introduction portion 12, a slit 13, and a gripping portion 14.

The collection tool introduction portion 12 is a truncated cone-shaped member having a slit 13 on a side wall.

In the collection tool introduction portion 12, insertion into the anus is done from a tip end side edge portion 15 having a smaller outer diameter, and the collection tool 2 is inserted from a proximal side edge portion 16 having a larger outer diameter. The collection tool introduction portion 12 may have a through-hole in a rotation axis direction. From the viewpoint of ease of insertion into the anus, an outer diameter of the proximal side edge portion 16 is preferably 30 to 70 mm, and more preferably 40 to 60 mm. In addition, from the viewpoint of ease of introduction of the collection tool 2 into a surface of large intestinal mucosa, an outer diameter of the tip end side edge portion 15 is preferably 10 to 30 mm, and more preferably 15 to 25 mm. Similarly, a length of the collection tool introduction portion 12 in a rotation axis direction is preferably 50 to 150 mm, more preferably 70 to 130 mm, and even more preferably 80 to 120 mm.

The slit 13 is provided from the tip end side edge portion 15 of the collection tool introduction portion 12 toward the proximal side edge portion 16. Presence of the slit 13 reaching the tip end side edge portion 15 on a part of a side wall of the collection tool introduction portion 12 increases a degree of freedom of movement of the tip end of the collection tool 2 in the intestinal tract, which makes it possible to more easily collect large intestinal mucosa in the rectum, the internal structure of which is complicated. The slit 13 may be set at any position of the collection tool introduction portion 12. For example, as shown in FIG. 2(B), the slit 13 is preferably located on a side close to the gripping portion 14. In addition, the number of the slit 13 provided in the collection tool introduction portion 12 may be one, or two or more.

In order to cause the collection tool 2 to penetrate the slit 13 and reach a surface of large intestinal mucosa, a width of the slit 13 is designed to be wider than a width of the first clamping piece 3a and the second clamping piece 3b of the collection tool 2 in a state in which the clamping surface 35a and the clamping surface 35b are in contact with each other. For example, in a state in which the clamping surface 35a and the clamping surface 35b are in contact with each other, in a case where a width $L_1$ of the end portions of the first clamping piece 3a and the second clamping piece 3b of the collection tool 2 is 2 to 5 mm, a width $L_2$ on a side of the tip end side edge portion 15 of the slit 13 is preferably 7 to 25 mm, and preferably 15 to 20 mm. In addition, the width of the slit 13 may be constant or may be narrowed toward either direction. Two or more slits may be formed on a wall surface of the collection tool introduction portion 12.

One end of the gripping portion 14 is connected in the vicinity of the proximal side edge portion 16 of the collection tool introduction portion 12 in a direction away from the collection tool introduction portion 12. The gripping portion 14 may be a hollow rod shape of which a lower side is open and which is reinforced by ribs. A length of the gripping portion 14 is preferably 50 to 150 mm, and more preferably 70 to 130 mm, from the viewpoint of ease of grasping by hand or the like. In addition, from the viewpoint of ease of grasping by hand or the like, a width of the gripping portion 14 is preferably 5 to 20 mm, and more preferably 8 to 13 mm, and a thickness of the gripping portion 14 is preferably 10 to 30 mm, and more preferably 15 to 25 mm. A shape of the gripping portion 14 may be any shape as long as the shape is easy to grasp, and may be, for example, a plate shape, a rod shape, or any other shape.

The gripping portion 14 may be vertically connected to a center axis of a truncated cone shape of the collection tool introduction portion 12 in the vicinity of a proximal side edge portion 16 of the collection tool introduction portion 12. However, from the viewpoint of causing the collection tool 2 to easily reach large intestinal mucosa, an angle $\theta_1$ (see FIG. 2 (C)) between a rotation axis direction of the collection tool introduction portion 12 and a center axis direction of the collection tool introduction portion 12 is preferably greater than 90° and equal to or less than 120°, more preferably 95° to 110°, and even more preferably 95° to 105°.

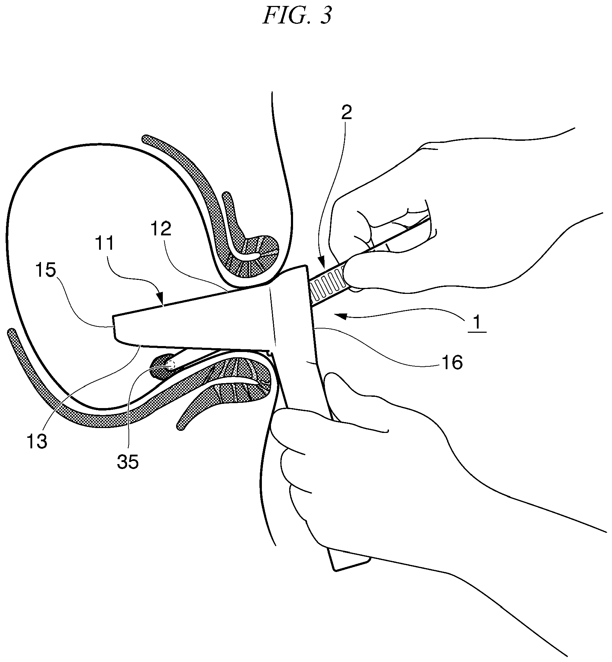

FIG. 3 is an explanatory view showing a mode of use of the kit 1 for collecting large intestinal mucosa according to the present invention. First, the collection auxiliary tool 11 is inserted from the tip end side edge portion 15 into the anus of a subject whose large intestinal mucosa is to be collected. In a state in which the gripping portion 14 is held with one hand and is stabilized, the collection tool 2 is introduced from an opening part on a side of the proximal side edge portion 16. The introduced collection tool 2 is caused to penetrate through the slit 13 from the tip end and reach a surface of the large intestinal mucosa. The collection tool 2 is pulled out from the slit 13 in a state where the large intestinal mucosa is clamped between the clamping surface 35a and the clamping surface 35b of the collection tool 2, so that the large intestinal mucosa can be collected.

Examples

Next, the present invention will be described in more detail by showing examples and the like. However, the present invention is not limited thereto.

[Example 1]

With respect to DNA in large intestinal mucosa collected from 8 healthy subjects (5 males and 3 females), and 6 colorectal cancer patients (3 males and 3 females) who had not developed other inflammatory diseases of the large intestine such as ulcerative colitis and had been diagnosed as having sporadic colorectal cancer by pathological diagnosis using biopsy tissue in an endoscopic examination, comprehensive analysis for a methylation rate of a CpG site was conducted.

<Comprehensive analysis of methylation level of CpG site>

(1) Biopsy and DNA extraction

Mucosal tissue was collected from 3 locations in the large intestine of the same subject, and frozen and stored at -80°C. The collected sites were cecum, transverse colon, rectum, and cancerous part for the colorectal cancer patients, and were cecum, transverse colon, and rectum for the healthy subjects. The collected tissue was finely cut and DNA was extracted using QIAmp DNA kit (manufactured by Qiagen).

(2) Quality evaluation of DNA sample

The concentration of the obtained DNA was obtained as follows. That is, a fluorescence intensity of each sample was measured using Quant-iT PicoGreen ds DNA Assay Kit (manufactured by Life Technologies), and the concentration thereof was calculated using a calibration curve of $\lambda$-DNA attached to the kit.

Next, each sample was diluted to 1 ng/$\mu$L with TE (pH 8.0), real-time PCR was carried out using Illumina FFPE QC Kit (manufactured by Illumina) and Fast SYBR Green Master Mix (manufactured by Life Technologies), so that a Ct value was obtained. A difference in Ct value (hereinafter referred to as $\Delta$Ct value) between the sample and a positive control was calculated for each sample, and quality was evaluated. Samples with a $\Delta$Ct value less than 5 were determined to have good quality and subjected to subsequent steps.

(3) Bisulfite treatment

Bisulfite treatment was performed on the DNA samples using EZ DNA Methylation Kit (manufactured by ZYMO RESEARCH). Thereafter, Infinium HD FFPE Restore Kit (manufactured by Illumina) was used to restore the degraded DNA.

(4) Whole genome amplification

The restored DNA was alkali-denatured and neutralized. To the resultant were added enzymes and primers for amplification of the whole genome of Human Methylation 450 DNA Analysis Kit (manufactured by Illumina), and isothermal reaction was allowed to proceed in Incubation Oven (manufactured by Illumina) at 37°C for 20 hours or longer, so that the whole genome was amplified.

(5) Fragmentation and purification of whole genome-amplified DNA

To the whole genome-amplified DNA was added an enzyme for fragmentation of Human Methylation 450 DNA Analysis Kit (manufactured by Illumina Co.), and reaction was allowed to proceed in Microsample Incubator (SciGene) at 37°C for 1 hour. To the fragmented DNA were added a coprecipitant and 2-propanol, and the resultant was centrifuged to precipitate DNA.

(6) Hybridization

To the precipitated DNA was added a hybridization buffer, and reaction was allowed to proceed in Hybridization Oven (manufactured by Illumina) at 48°C for 1 hour, so that the DNA was dissolved. The dissolved DNA was incubated in Microsample Incubator (manufactured by SciGene) at 95°C for 20 minutes to denature into single strands, and then dispensed onto the BeadChip of Human Methylation 450 DNA Analysis Kit (manufactured by Illumina). The resultant was allowed to react in Hybridization Oven at 48°C for 16 hours or longer to hybridize probes on the BeadChip with the single-stranded DNA.

(7) Labeling reaction and scanning

The probes on the BeadChip after the hybridization were subjected to elongation reaction to bind fluorescent dyes. Subsequently, the BeadChip was scanned with the iSCAN system (manufactured by Illumina), and methylated fluorescence intensity and non-methylated fluorescence intensity were measured. At the end of the experiment, it was confirmed that all of the scanned data was complete and that scanning was normally done.

(8) Quantification and comparative analysis of DNA methylation level

The scanned data was analyzed using the DNA methylation analysis software GenomeStudio (Version: V2011.1). A DNA methylation level (β value) was calculated by the following expression.

$$[\beta \text{ value}] = [\text{Methylated fluorescence intensity}] \div ([\text{Methylated fluorescence intensity}] +$$

$$[\text{Non-methylated fluorescence intensity}] + 100)$$

In a case where the methylation level is high, the β value approaches 1, and in a case where the methylation level is low, the β value approaches 0. DiffScore calculated by GenomeStudio was used for comparative analysis of the DNA methylation level of the colorectal cancer patient rectal sample group (n=6) with respect to the healthy subject rectal sample group (n=8). In a case where the DNA methylation levels of both groups are close to each other, DiffScore approaches 0. In a case where the level is higher in the colorectal cancer patients, a positive value is exhibited, and in a case where the level is lower in the colorectal cancer patients, a negative value is exhibited. The greater a difference in methylation level between both groups, the greater an absolute value of DiffScore. In addition, a value (Δβ value) obtained by subtracting an average β value of the healthy subject rectal sample group (n=8) from an average β value of the colorectal cancer patient rectal sample group (n=6) was also used for the comparative analysis.

GenomeStudio and the software Methylation Module (Version: 1.9.0) were used for DNA methylation quantification and DNA methylation level comparative analysis. Setting conditions for GenomeStudio are as follows.

DNA methylation quantification;

Normalization: Yes (Controls)

Subtract Background: Yes

Content Descriptor: HumanMethylation450_15017482_v. 1.2. bpm

DNA methylation level comparative analysis;

Normalization: Yes (Controls)

Subtract Background: Yes

Content Descriptor: HumanMethylation450_15017482_v. 1.2. bpm

Ref Group: Comparative analysis 4. Group-3

Error Model: Illumina custom

Compute False Discovery Rate: No

(9) Multivariate analysis

Using the results obtained by the DNA methylation level quantification and comparative analysis, DiffScore was calculated with the statistical analysis software R (Version: 3.0.1, 64 bit, Windows (registered trademark)), and cluster analysis and principal component analysis were performed.

R script of cluster analysis:

```
> data.dist<- as.dist (1-
cor (data. frame, use="pairwise.complete.obs",method="p"))>
hclust(data.dist, method="complete")
        # data. frame: data frame composed of CpG (row) x sample (column)
        # 1-Pearson correlation coefficient defined as distance, implemented by
complete linkage method
```

R script of principal component analysis:

```
> prcomp(t(data.frame), scale = T)
 # data.frame: data frame composed of CpG (row) x sample (column)
```

<Selection of CpG biomarker>

(1) Extraction of CpG biomarker candidates

As means for selecting CpG biomarker candidates from comprehensive DNA methylation analysis data, narrowing-down based on DiffScore and $\Delta\beta$ value has been reported (BMC Med genomics vol. 4, p. 50, 2011; Sex Dev vol. 5, p. 70, 2011). Biomarker candidates are extracted by setting an absolute value of DiffScore to higher than 30 and an absolute value of $\Delta\beta$ value to higher than 0.2 for the former report, and by setting an absolute value of DiffScore to higher than 30 and an absolute value of $\Delta\beta$ value to higher than 0.3 for the latter report. According to these methods, biomarker candidates were extracted from 485,577 CpG sites loaded on the BeadChip.

Specifically, firstly, 54 CpG sites with an absolute value of DiffScore higher than 30 and with an absolute value of $\Delta\beta$ value higher than 0.3 were selected from the 485,577 CpG sites. Hereinafter, these 54 CpG sites are collectively referred to as "54 CpG sets". Furthermore, for the purpose of discriminating cancer patients who had developed sporadic colorectal cancer without missing, the cancer patient samples were narrowed-down to samples with less fluctuation in the DNA methylation level. That is, an unbiased variance var of $\beta$ values of 23 cancer patient samples (4 sites $\times$ 6 or 7 samples per each site) was obtained, and narrowing-down to 8 CpG sites with a value of unbiased variance var lower than 0.02 was performed. Hereinafter, these 8 CpG sites are collectively referred to as "8 CpG sets".

The results of the respective CpG sites of the 54 CpG sets are shown in Tables 24 and 25. In the tables, the CpG site with # in the "8 CpG" column shows a CpG site included in the 8 CpG sets.

[Table 24]

| CpG ID | Average β value (cancer rectal) n=6 | Average β value (non-cancerous rectal) n=8 | DiffScore | Δβ value | unbiased variance (cancer) n=23 | 54 CpG | 8 CpG |
|---|---|---|---|---|---|---|---|
| cg07621697 | 0.04 ± 0.01 | 0.37 ± 0.31 | −371 | −0.33 | 0.000 | # | # |
| cg16081854 | 0.74 ± 0.01 | 0.40 ± 0.27 | 374 | 0.33 | 0.001 | # | # |
| cg01710670 | 0.74 ± 0.05 | 0.41 ± 0.29 | 374 | 0.33 | 0.003 | # | # |
| cg22946888 | 0.12 ± 0.06 | 0.57 ± 0.41 | −371 | −0.43 | 0.004 | # | # |
| cg00713204 | 0.62 ± 0.11 | 0.28 ± 0.31 | 374 | 0.33 | 0.012 | # | # |
| cg12074150 | 0.09 ± 0.14 | 0.46 ± 0.43 | −371 | −0.36 | 0.013 | # | # |
| cg06758191 | 0.77 ± 0.14 | 0.33 ± 0.27 | 374 | 0.44 | 0.017 | # | # |
| cg12515659 | 0.61 ± 0.15 | 0.26 ± 0.32 | 374 | 0.35 | 0.018 | # | # |
| cg18172516 | 0.58 ± 0.14 | 0.24 ± 0.24 | 374 | 0.34 | 0.020 | # | |
| cg12280242 | 0.24 ± 0.10 | 0.58 ± 0.35 | −360 | −0.32 | 0.023 | # | |
| cg27288829 | 0.13 ± 0.17 | 0.44 ± 0.25 | −371 | −0.31 | 0.025 | # | |
| cg14293674 | 0.74 ± 0.16 | 0.43 ± 0.30 | 374 | 0.31 | 0.029 | # | |
| cg02507579 | 0.13 ± 0.19 | 0.46 ± 0.27 | −371 | −0.33 | 0.031 | # | |
| cg19707653 | 0.18 ± 0.18 | 0.50 ± 0.16 | −371 | −0.32 | 0.032 | # | |
| cg19285525 | 0.60 ± 0.17 | 0.23 ± 0.26 | 374 | 0.37 | 0.034 | # | |
| cg04131969 | 0.61 ± 0.20 | 0.31 ± 0.23 | 374 | 0.30 | 0.034 | # | |
| cg07227024 | 0.11 ± 0.20 | 0.45 ± 0.30 | −371 | −0.34 | 0.035 | # | |
| cg00695177 | 0.13 ± 0.20 | 0.51 ± 0.41 | −371 | −0.38 | 0.038 | # | |
| cg03311906 | 0.42 ± 0.23 | 0.79 ± 0.18 | −371 | −0.36 | 0.038 | # | |
| cg20536971 | 0.45 ± 0.20 | 0.80 ± 0.15 | −371 | −0.35 | 0.039 | # | |
| cg15828613 | 0.68 ± 0.22 | 0.35 ± 0.30 | 374 | 0.33 | 0.041 | # | |
| cg24506221 | 0.78 ± 0.28 | 0.44 ± 0.34 | 374 | 0.35 | 0.041 | # | |
| cg27156510 | 0.28 ± 0.21 | 0.65 ± 0.24 | −371 | −0.36 | 0.049 | # | |
| cg26077133 | 0.18 ± 0.23 | 0.58 ± 0.30 | −371 | −0.39 | 0.052 | # | |
| cg24087071 | 0.36 ± 0.25 | 0.66 ± 0.19 | −314 | −0.30 | 0.053 | # | |
| cg17662493 | 0.30 ± 0.23 | 0.71 ± 0.29 | −371 | −0.41 | 0.058 | # | |
| cg12036633 | 0.55 ± 0.28 | 0.90 ± 0.03 | −371 | −0.35 | 0.066 | # | |
| cg11251367 | 0.51 ± 0.27 | 0.15 ± 0.31 | 374 | 0.37 | 0.069 | # | |
| cg14181874 | 0.46 ± 0.28 | 0.80 ± 0.29 | −371 | −0.33 | 0.069 | # | |
| cg21164300 | 0.40 ± 0.35 | 0.81 ± 0.18 | −371 | −0.42 | 0.073 | # | |
| cg19405842 | 0.57 ± 0.31 | 0.26 ± 0.23 | 374 | 0.31 | 0.078 | # | |
| cg21114725 | 0.32 ± 0.29 | 0.75 ± 0.31 | −371 | −0.42 | 0.078 | # | |
| cg08433110 | 0.49 ± 0.31 | 0.89 ± 0.03 | −371 | −0.38 | 0.079 | # | |
| cg16051083 | 0.43 ± 0.31 | 0.09 ± 0.12 | 374 | 0.34 | 0.081 | # | |
| cg11454325 | 0.28 ± 0.30 | 0.72 ± 0.29 | −371 | −0.43 | 0.084 | # | |
| cg12870217 | 0.24 ± 0.32 | 0.60 ± 0.22 | −371 | −0.36 | 0.084 | # | |

[Table 25]

| CpG ID | Average β value (cancer rectal) n=6 | | Average β value (non-cancerous rectal) n=8 | | DiffScore | Δβ value | unbiased variance (cancer) n=23 | 54 CpG | 8 CpG |
|---|---|---|---|---|---|---|---|---|---|
| cg24208588 | 0.52 ± 0.33 | | 0.11 ± 0.13 | | 374 | 0.41 | 0.092 | # | |
| cg08429705 | 0.69 ± 0.32 | | 0.38 ± 0.38 | | 374 | 0.31 | 0.101 | # | |
| cg24976563 | 0.41 ± 0.34 | | 0.77 ± 0.27 | | −371 | −0.36 | 0.102 | # | |
| cg14323910 | 0.53 ± 0.34 | | 0.20 ± 0.33 | | 374 | 0.33 | 0.103 | # | |
| cg04212500 | 0.41 ± 0.37 | | 0.72 ± 0.30 | | −344 | −0.31 | 0.104 | # | |
| cg00348031 | 0.46 ± 0.33 | | 0.78 ± 0.02 | | −365 | −0.31 | 0.107 | # | |
| cg02890235 | 0.34 ± 0.35 | | 0.72 ± 0.28 | | −371 | −0.38 | 0.108 | # | |
| cg00525828 | 0.65 ± 0.36 | | 0.98 ± 0.00 | | −371 | −0.33 | 0.110 | # | |
| cg02775404 | 0.38 ± 0.38 | | 0.78 ± 0.04 | | −371 | −0.38 | 0.111 | # | |
| cg23663942 | 0.49 ± 0.31 | | 0.80 ± 0.04 | | −347 | −0.30 | 0.113 | # | |
| cg15115757 | 0.55 ± 0.38 | | 0.88 ± 0.02 | | −371 | −0.32 | 0.114 | # | |
| cg03022891 | 0.51 ± 0.35 | | 0.83 ± 0.07 | | −371 | −0.32 | 0.117 | # | |
| cg22664298 | 0.58 ± 0.38 | | 0.18 ± 0.13 | | 374 | 0.40 | 0.123 | # | |
| cg06306564 | 0.36 ± 0.40 | | 0.86 ± 0.12 | | −371 | −0.50 | 0.125 | # | |
| cg01647917 | 0.43 ± 0.40 | | 0.78 ± 0.33 | | −371 | −0.34 | 0.137 | # | |
| cg16661157 | 0.33 ± 0.42 | | 0.66 ± 0.41 | | −344 | −0.32 | 0.146 | # | |
| cg17025908 | 0.49 ± 0.43 | | 0.84 ± 0.19 | | −371 | −0.34 | 0.158 | # | |
| cg19455396 | 0.46 ± 0.45 | | 0.88 ± 0.08 | | −371 | −0.42 | 0.174 | # | |

(2) Multivariate analysis of clinical samples using CpG biomarker candidates

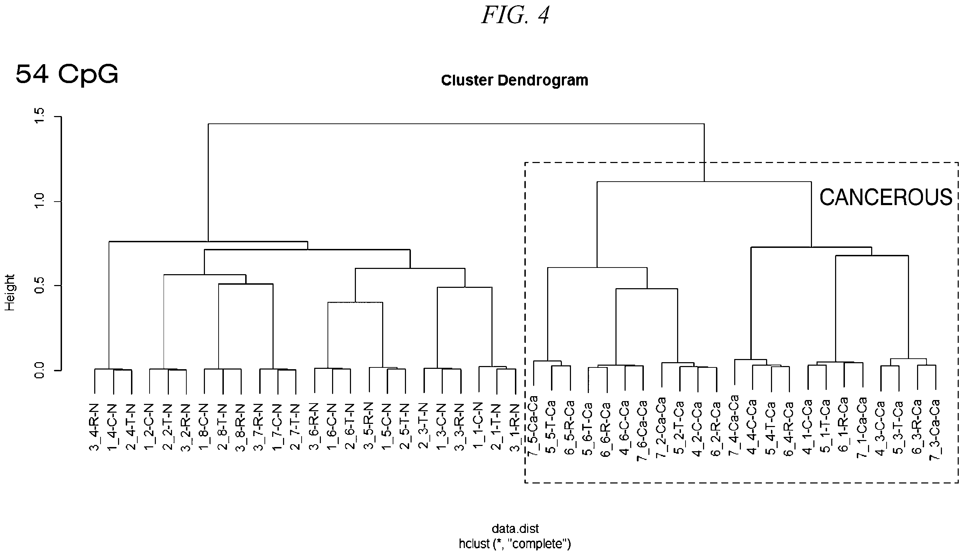

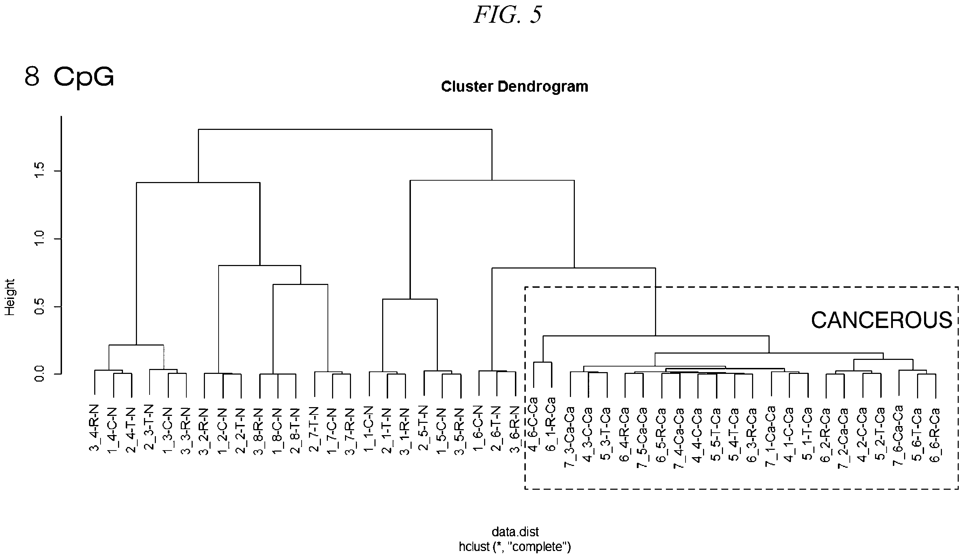

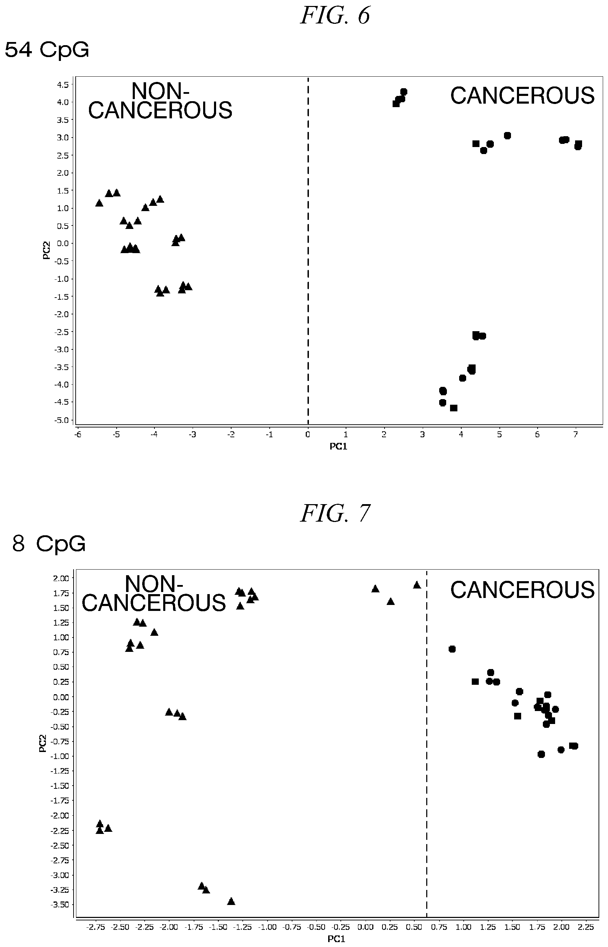

Cluster analysis and principal component analysis for all 23 samples were performed using the 54 CpG sets or 8 CpG sets, and as shown in FIGS. 4 and 5, in the cluster analysis, all colorectal cancer patient samples accumulated in the same cluster (within a frame, in the drawings) in any of the CpG sets. In addition, as shown in FIGS. 6 and 7, in the principal component analysis (the vertical axis is a second principal component), colorectal cancer patient samples (black circles are samples collected from non-cancerous sites, and black squares are samples collected cancerous sites) and healthy subject (non-cancerous) samples (black triangles) each formed independent clusters in a first principal component (horizontal axis) direction. That is, in any of the CpG sets, it was possible to clearly distinguish between the colorectal cancer patient samples and the healthy subject samples. From these results, 54 CpG's listed in Tables 24 and 25 are extremely useful as biomarkers of sporadic colorectal cancer development in a human subject, and it is apparent that these CpG's can be used to determine the presence or absence of sporadic colorectal cancer development in a human subject, in particular, a subject who does not have subjective symptoms of a large intestinal disease, with high sensitivity and specificity.

[Example 2]

With respect to DNA in large intestinal mucosa collected from 28 healthy subjects and 20 colorectal cancer patients who had not developed other inflammatory diseases of the large intestine such as ulcerative colitis and had been diagnosed as having sporadic colorectal cancer by pathological diagnosis using biopsy tissue in an endoscopic examination, comprehensive analysis for a methylation rate of a CpG site was conducted.

For the DNA to be subjected to analysis of a methylation rate of a CpG site, DNA was extracted from mucosal tissue of the rectum of each subject in the same manner as in Example 1, the whole genome was amplified, and quantification and comparative analysis of the DNA methylation level of the CpG site were performed. The results were used to calculate DiffScore, and cluster analysis and principal component analysis were performed. Infinium Methylation EPIC BeadChip (manufactured by Illumina) was used for BeadChip. In addition, setting conditions for GenomeStudio were the same as in Example 1 except that "MethylationEPIC_v-1-0_B2.bpm" was used for "Content Descriptor".

(1) Extraction of CpG biomarker candidates

Subsequently, CpG biomarker candidates were extracted from comprehensive DNA methylation analysis data. Specifically, firstly, 142 CpG sites with an absolute value of $\Delta\beta$ higher than 0.15 were extracted from 866,895 CpG sites.
Next, the following two types of logistic regression models were created.

[Model 1] 10,011 logistic regression models based on all combinations of 2 CpG sites selected from 142 CpG sites.
[Model 2] 467,180 logistic regression models based on all combinations of 3 CpG's selected from 142 CpG sites.

Regarding discrimination expressions of both logistic regression models, a CpG site that satisfies each of the following two criteria was selected. In addition, for [Model 2], a frequency of the appearance of CpG sites was also calculated so that a CpG site with a frequency of three or more was selected.

[Criterion 1] Sensitivity of higher than 90%, specificity of higher than 90%, coefficient p value of discrimination expression of lower than 0.05, and Akaike's information criterion (AIC) of lower than 30.
[Criterion 2] Sensitivity of higher than 95%, specificity of higher than 85%, coefficient p value of discrimination expression of lower than 0.05, and Akaike's information criterion (AIC) of lower than 30.

CpG sites appearing in the discrimination expression were selected for each of the two criteria, and 33 CpG sites (33 CpG sets) listed in Tables 13 to 15 were chosen. The results of the respective CpG sites are shown in Table 26.

[Table 26]

| CpG ID | Average $\beta$ value (cancer rectal) n=20 | Average $\beta$ value (non-cancerous rectal) n=28 | $\Delta\beta$ value |
|---|---|---|---|
| cg00853216 | 0.55 ± 0.30 | 0.37 ± 0.25 | 0.18 |
| cg00866176 | 0.74 ± 0.20 | 0.52 ± 0.32 | 0.22 |
| cg01105403 | 0.71 ± 0.26 | 0.49 ± 0.35 | 0.22 |
| cg02078724 | 0.44 ± 0.21 | 0.27 ± 0.13 | 0.17 |
| cg03057303 | 0.36 ± 0.24 | 0.51 ± 0.26 | -0.15 |
| cg04234412 | 0.69 ± 0.31 | 0.49 ± 0.32 | 0.20 |
| cg04262140 | 0.45 ± 0.12 | 0.28 ± 0.10 | 0.17 |
| cg04456492 | 0.64 ± 0.17 | 0.46 ± 0.27 | 0.19 |
| cg06829686 | 0.33 ± 0.16 | 0.13 ± 0.05 | 0.20 |
| cg07684215 | 0.55 ± 0.27 | 0.37 ± 0.29 | 0.18 |
| cg08421632 | 0.61 ± 0.24 | 0.80 ± 0.03 | -0.19 |
| cg10169393 | 0.49 ± 0.07 | 0.65 ± 0.05 | -0.16 |
| cg10204409 | 0.44 ± 0.20 | 0.59 ± 0.13 | -0.15 |
| cg10326673 | 0.34 ± 0.32 | 0.50 ± 0.25 | -0.16 |
| cg10360725 | 0.73 ± 0.24 | 0.57 ± 0.33 | 0.16 |
| cg10530344 | 0.47 ± 0.18 | 0.62 ± 0.10 | -0.15 |
| cg10690713 | 0.46 ± 0.25 | 0.61 ± 0.18 | -0.15 |
| cg10772532 | 0.46 ± 0.33 | 0.63 ± 0.33 | -0.17 |
| cg11044162 | 0.56 ± 0.39 | 0.71 ± 0.30 | -0.15 |
| cg11141652 | 0.15 ± 0.16 | 0.36 ± 0.23 | -0.20 |
| cg12219587 | 0.22 ± 0.20 | 0.45 ± 0.32 | -0.23 |

(continued)

| CpG ID | Average β value (cancer rectal) n=20 | Average β value (non-cancerous rectal) n=28 | Δβ value |
|---|---|---|---|
| cg12814117 | 0.37 ± 0.28 | 0.54 ± 0.16 | -0.17 |
| cg14629397 | 0.33 ± 0.21 | 0.54 ± 0.17 | -0.21 |
| cg16013720 | 0.55 ± 0.10 | 0.39 ± 0.04 | 0.16 |
| cg16776298 | 0.45 ± 0.21 | 0.61 ± 0.15 | -0.16 |
| cg17658874 | 0.38 ± 0.24 | 0.54 ± 0.18 | -0.16 |
| cg18285337 | 0.36 ± 0.25 | 0.52 ± 0.26 | -0.16 |
| cg19236675 | 0.48 ± 0.34 | 0.69 ± 0.23 | -0.20 |
| cg19631563 | 0.60 ± 0.20 | 0.76 ± 0.05 | -0.16 |
| cg19919789 | 0.60 ± 0.18 | 0.75 ± 0.06 | -0.16 |
| cg22109827 | 0.56 ± 0.27 | 0.72 ± 0.24 | -0.16 |
| cg23231631 | 0.67 ± 0.26 | 0.85 ± 0.11 | -0.17 |
| cg27351675 | 0.46 ± 0.14 | 0.28 ± 0.10 | 0.18 |

(2) Multivariate analysis of clinical samples using CpG biomarker candidates

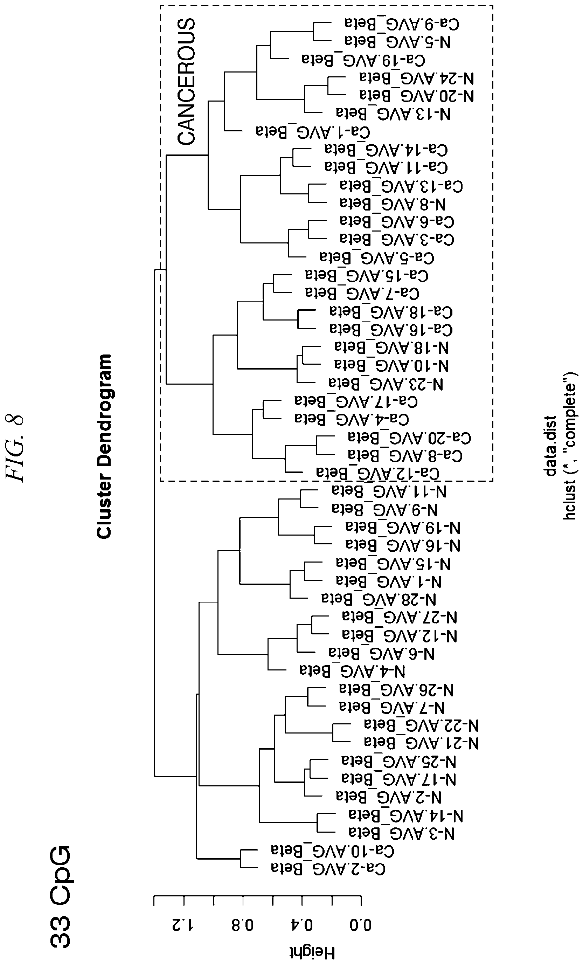

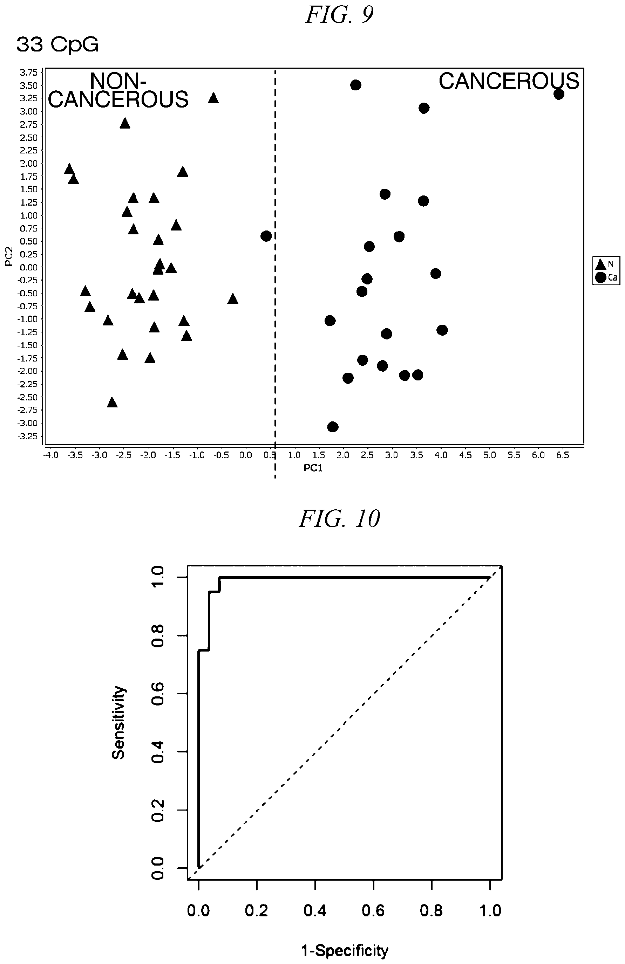

Cluster analysis and principal component analysis for all 48 samples were performed based on methylation levels of the 33 CpG sets. As a result, in the cluster analysis (FIG. 8), most colorectal cancer patient samples accumulated in the same cluster (within a frame, in the drawing). In addition, in the principal component analysis (FIG. 9, the vertical axis is a second principal component), the colorectal cancer patient samples (●) and the healthy subject samples (▲) each formed independent clusters in a first principal component (horizontal axis) direction. That is, using the 33 CpG sets, it was possible to clearly distinguish between the 20 colorectal cancer patient samples and the 28 healthy subject samples.

(3) Evaluation of the likelihood of sporadic colorectal cancer development in clinical samples using CpG biomarker candidates

Accuracy of determination of the presence or absence of sporadic colorectal cancer development was examined in a case where methylation rates of the three CpG sites of the CpG site (cg01105403) in the base sequence represented by SEQ ID NO: 57, the CpG site (cg06829686) in the base sequence represented by SEQ ID NO: 63, and the CpG site (cg14629397) in the base sequence represented by SEQ ID NO: 77 are used as markers, among the 33 CpG set.

Specifically, based on a logistic regression model using numerical values (β values) of methylation levels of the three CpG sites of specimens collected from the rectums of 20 colorectal cancer patients who had been diagnosed as having sporadic colorectal cancer and 28 healthy subjects, a discrimination expression was created to discriminate between a colorectal cancer patient and a healthy subject. As a result, sensitivity (proportion evaluated as positive among the colorectal cancer patients) was 95.0%, specificity (proportion evaluated as negative among the healthy subjects) was 96.4%, positive predictive value (proportion of colorectal cancer patients among those evaluated as positive) was 95.0%, and negative predictive value (proportion of healthy subjects among those evaluated as negative) was 96.4%, indicating that all were as high as 90% or more. In addition, FIG. 10 shows a receiver operating characteristic (ROC) curve. An AUC (area under the ROC curve) was 0.989. From these results, it was confirmed that the likelihood of sporadic colorectal cancer development can be evaluated with high sensitivity and high specificity based on methylation rates of 2 to 5 CpG sites selected from the 33 CpG sets.

[Example 3]

CpG biomarker candidates were extracted from the DNA methylation levels (β values) of rectal mucosa samples obtained in Examples 1 and 2.

(1) Extraction of CpG biomarker candidate

Specifically, firstly, in 26 colorectal cancer patient samples which had been diagnosed as sporadic colorectal cancer and 36 healthy subject samples, 42 CpG sites with an absolute value of $\Delta\beta$ higher than 0.15 were extracted from 866,895 CpG sites.

Next, the following two types of logistic regression models were created.

[Model 1] 861 logistic regression models based on all combinations of 2 CpG's selected from 42 CpG sites.
[Model 2] 11,480 logistic regression models based on all combinations of 3 CpG's selected from 42 CpG sites.

Regarding the discriminant expressions of both logistic regression models, a CpG site that satisfies each of the following two criteria was selected.

[Criterion 1] Sensitivity of higher than 90%, specificity of higher than 90%, coefficient p value of discrimination expression of lower than 0.05, and Akaike's information criterion (AIC) of lower than 30.
[Criterion 2] Sensitivity of higher than 95%, specificity of higher than 85%, coefficient p value of discrimination expression of lower than 0.05, and Akaike's information criterion (AIC) of lower than 30.

For each of the two criteria, a CpG site appearing in the discrimination expression was selected. In a case where CpG's chosen in Example 2 were excluded from the selected CpG sites, 6 CpG sites (6 CpG sets) listed in Table 16 were chosen. The results of the respective CpG sites are shown in Table 27.

[Table 27]

| CpG ID | Average $\beta$ value (cancer rectal) n=20 | Average $\beta$ value (non-cancerous rectal) n=28 | $\Delta\beta$ value |
|---|---|---|---|
| cg01561758 | $0.73 \pm 0.17$ | $0.58 \pm 0.25$ | 0.15 |
| cg06970370 | $0.41 \pm 0.13$ | $0.26 \pm 0.12$ | 0.15 |
| cg07973162 | $0.16 \pm 0.15$ | $0.36 \pm 0.30$ | -0.21 |
| cg11792281 | $0.28 \pm 0.05$ | $0.44 \pm 0.09$ | -0.16 |
| cg18500967 | $0.63 \pm 0.29$ | $0.39 \pm 0.32$ | 0.24 |
| cg23943944 | $0.76 \pm 0.19$ | $0.61 \pm 0.24$ | 0.15 |

(2) Multivariate analysis of clinical samples using CpG biomarker candidates

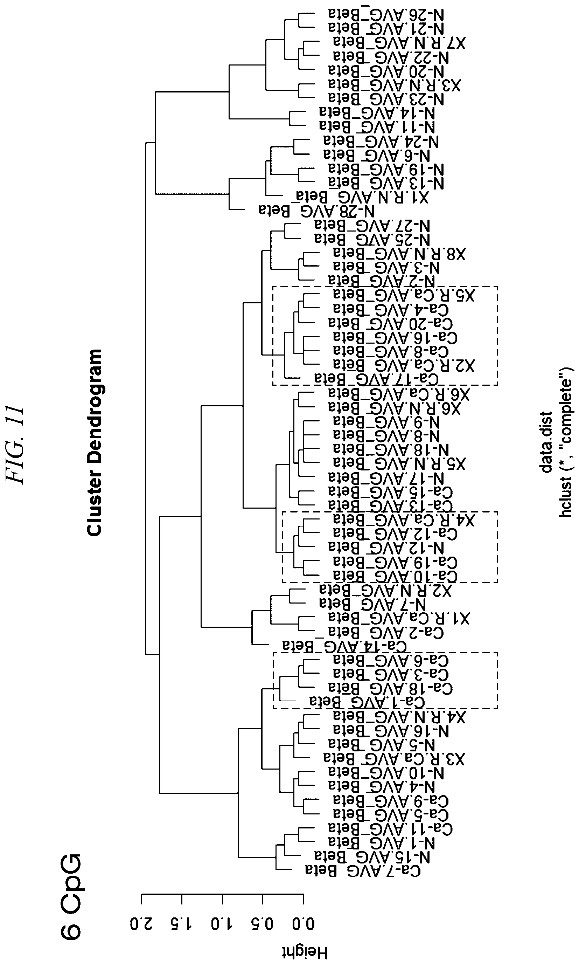

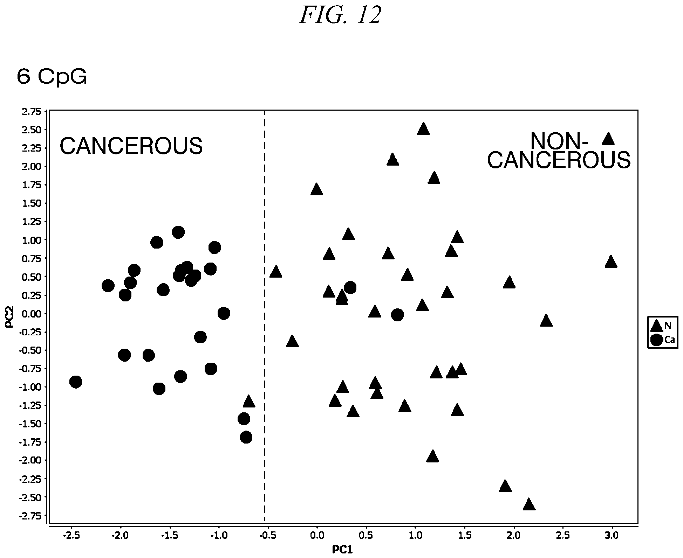

Based on the methylation levels of the 6 CpG sets, cluster analysis and principal component analysis for all 62 samples were performed. As a result, in the cluster analysis (FIG. 11), many colorectal cancer patient samples accumulated in several clusters (within a frame, in the drawing). In addition, in the principal component analysis (FIG. 12, the vertical axis is a second principal component), the colorectal cancer patient samples (●) and the healthy subject samples (▲) each formed independent clusters in a first principal component (horizontal axis) direction. That is, in the principal component analysis, using the 6 CpG sets, it was possible to clearly distinguish between the 20 colorectal cancer patient samples and the 28 healthy subject samples.

[Example 4]

DMR biomarker candidates were extracted from an average methylation rate (average $\beta$ value; additive average value of methylation levels ($\beta$ values) of CpG sites present in each DMR) of each DMR of specimens collected from the rectums of 20 colorectal cancer patients and 28 healthy subjects obtained in Example 2.

(1) Extraction of DMR biomarker candidates

Specifically, firstly, methylation data (IDAT format) of 866,895 CpG sites was input to the ChAMP pipeline (Bioinformatics, 30, 428, 2014; http://bioconductor.org/packages/release/bioc/html/ChAMP.html), and 4,232 DMR's determined as significant between the two groups of colorectal cancer patients and healthy subjects were extracted. Among these, 121 locations (DMR numbers 1 to 121) with an absolute value of $\Delta\beta$ value ([average $\beta$ value (cancerous rectum)] - [average $\beta$ value (non-cancerous rectum)]) of higher than 0.05 were set as DMR biomarker candidates. The results of the 121 DMR's (121 DMR sets) are shown in Tables 28 to 31.

[Table 28]

| | Average β value (cancer rectal) n=20 | Average β value (non-cancerous rectal) n=28 | Δβ value | 52DMR | 15DMR |
|---|---|---|---|---|---|
| 1 | 0.43±0.10 | 0.30±0.09 | 0.13 | # | # |
| 2 | 0.45±0.05 | 0.39±0.05 | 0.06 | # | # |
| 3 | 0.28±0.05 | 0.22±0.08 | 0.06 | # | # |
| 4 | 0.16±0.06 | 0.11±0.02 | 0.06 | # | # |
| 5 | 0.34±0.05 | 0.29±0.05 | 0.05 | # | # |
| 6 | 0.49±0.04 | 0.43±0.07 | 0.05 | # | # |
| 7 | 0.30±0.05 | 0.24±0.06 | 0.05 | # | # |
| 8 | 0.69±0.03 | 0.74±0.03 | -0.05 | # | # |
| 9 | 0.71±0.03 | 0.76±0.03 | -0.05 | # | # |
| 10 | 0.64±0.03 | 0.69±0.02 | -0.05 | # | # |
| 11 | 0.68±0.04 | 0.73±0.04 | -0.05 | # | # |
| 12 | 0.70±0.02 | 0.76±0.02 | -0.06 | # | # |
| 13 | 0.61±0.02 | 0.67±0.02 | -0.06 | # | # |
| 14 | 0.56±0.04 | 0.63±0.03 | -0.06 | # | # |
| 15 | 0.56±0.04 | 0.63±0.05 | -0.07 | # | # |
| 16 | 0.47±0.14 | 0.38±0.09 | 0.09 | # | |
| 17 | 0.40±0.09 | 0.31±0.12 | 0.09 | # | |
| 18 | 0.55±0.06 | 0.47±0.08 | 0.08 | # | |
| 19 | 0.39±0.06 | 0.32±0.10 | 0.06 | # | |
| 20 | 0.45±0.05 | 0.39±0.07 | 0.06 | # | |
| 21 | 0.22±0.06 | 0.16±0.05 | 0.06 | # | |
| 22 | 0.35±0.06 | 0.30±0.08 | 0.06 | # | |
| 23 | 0.32±0.05 | 0.26±0.08 | 0.06 | # | |
| 24 | 0.53±0.05 | 0.47±0.06 | 0.06 | # | |
| 25 | 0.52±0.06 | 0.46±0.06 | 0.06 | # | |
| 26 | 0.18±0.10 | 0.13±0.02 | 0.06 | # | |
| 27 | 0.30±0.06 | 0.24±0.07 | 0.06 | # | |
| 28 | 0.56±0.05 | 0.51±0.08 | 0.06 | # | |
| 29 | 0.35±0.05 | 0.29±0.06 | 0.06 | # | |
| 30 | 0.41±0.05 | 0.35±0.07 | 0.05 | # | |
| 31 | 0.45±0.05 | 0.40±0.04 | 0.05 | # | |
| 32 | 0.51±0.06 | 0.46±0.05 | 0.05 | # | |
| 33 | 0.29±0.05 | 0.24±0.08 | 0.05 | # | |
| 34 | 0.70±0.04 | 0.64±0.05 | 0.05 | # | |
| 35 | 0.70±0.05 | 0.75±0.03 | -0.05 | # | |

[Table 29]

| | Average β value (cancer rectal) n=20 | Average β value (non-cancerous rectal) n=28 | Δβ value | 52DMR | 15DMR |
|---|---|---|---|---|---|
| 36 | 0.71±0.03 | 0.76±0.02 | -0.05 | # | |
| 37 | 0.67±0.03 | 0.72±0.03 | -0.05 | # | |
| 38 | 0.70±0.06 | 0.75±0.05 | -0.05 | # | |
| 39 | 0.68±0.03 | 0.73±0.02 | -0.05 | # | |
| 40 | 0.66±0.04 | 0.71±0.03 | -0.05 | # | |
| 41 | 0.70±0.04 | 0.75±0.03 | -0.05 | # | |
| 42 | 0.73±0.05 | 0.78±0.03 | -0.05 | # | |
| 43 | 0.65±0.04 | 0.70±0.02 | -0.05 | # | |
| 44 | 0.66±0.04 | 0.71±0.03 | -0.05 | # | |
| 45 | 0.64±0.03 | 0.69±0.02 | -0.05 | # | |
| 46 | 0.52±0.03 | 0.57±0.04 | -0.05 | # | |
| 47 | 0.54±0.05 | 0.60±0.04 | -0.06 | # | |
| 48 | 0.74±0.06 | 0.80±0.03 | -0.06 | # | |
| 49 | 0.66±0.06 | 0.72±0.03 | -0.06 | # | |
| 50 | 0.66±0.04 | 0.72±0.03 | -0.06 | # | |
| 51 | 0.59±0.05 | 0.65±0.03 | -0.06 | # | |
| 52 | 0.62±0.05 | 0.68±0.03 | -0.07 | # | |
| 53 | 0.26±0.11 | 0.14±0.03 | 0.12 | | |
| 54 | 0.36±0.08 | 0.26±0.10 | 0.11 | | |
| 55 | 0.48±0.09 | 0.38±0.06 | 0.10 | | |
| 56 | 0.47±0.07 | 0.38±0.06 | 0.09 | | |
| 57 | 0.39±0.07 | 0.30±0.11 | 0.09 | | |
| 58 | 0.39±0.06 | 0.31±0.07 | 0.08 | | |
| 59 | 0.32±0.06 | 0.24±0.07 | 0.08 | | |
| 60 | 0.40±0.08 | 0.32±0.10 | 0.08 | | |
| 61 | 0.60±0.05 | 0.52±0.04 | 0.08 | | |
| 62 | 0.30±0.07 | 0.22±0.09 | 0.08 | | |
| 63 | 0.56±0.06 | 0.48±0.07 | 0.08 | | |
| 64 | 0.25±0.07 | 0.18±0.08 | 0.08 | | |
| 65 | 0.53±0.07 | 0.45±0.05 | 0.08 | | |
| 66 | 0.57±0.04 | 0.49±0.09 | 0.08 | | |
| 67 | 0.36±0.09 | 0.28±0.04 | 0.07 | | |
| 68 | 0.34±0.06 | 0.26±0.07 | 0.07 | | |
| 69 | 0.40±0.06 | 0.33±0.09 | 0.07 | | |
| 70 | 0.46±0.08 | 0.38±0.09 | 0.07 | | |

[Table 30]

|  | Average β value (cancer rectal) n=20 | Average β value (non-cancerous rectal) n=28 | Δβ value | 52DMR | 15DMR |
|---|---|---|---|---|---|
| 71 | 0.44±0.08 | 0.37±0.08 | 0.07 | | |
| 72 | 0.42±0.05 | 0.35±0.09 | 0.07 | | |
| 73 | 0.35±0.05 | 0.28±0.09 | 0.07 | | |
| 74 | 0.33±0.06 | 0.26±0.09 | 0.07 | | |
| 75 | 0.36±0.07 | 0.30±0.09 | 0.07 | | |
| 76 | 0.45±0.05 | 0.38±0.10 | 0.07 | | |
| 77 | 0.36±0.07 | 0.30±0.04 | 0.07 | | |
| 78 | 0.39±0.04 | 0.33±0.10 | 0.06 | | |
| 79 | 0.42±0.06 | 0.36±0.10 | 0.06 | | |
| 80 | 0.39±0.06 | 0.33±0.09 | 0.06 | | |
| 81 | 0.27±0.07 | 0.21±0.08 | 0.06 | | |
| 82 | 0.67±0.07 | 0.60±0.06 | 0.06 | | |
| 83 | 0.26±0.12 | 0.20±0.04 | 0.06 | | |
| 84 | 0.26±0.06 | 0.20±0.04 | 0.06 | | |
| 85 | 0.34±0.05 | 0.28±0.08 | 0.06 | | |
| 86 | 0.38±0.06 | 0.32±0.09 | 0.06 | | |
| 87 | 0.33±0.04 | 0.27±0.08 | 0.06 | | |
| 88 | 0.50±0.05 | 0.44±0.09 | 0.06 | | |
| 89 | 0.53±0.06 | 0.47±0.07 | 0.06 | | |
| 90 | 0.52±0.05 | 0.46±0.09 | 0.06 | | |
| 91 | 0.23±0.05 | 0.17±0.08 | 0.06 | | |
| 92 | 0.26±0.06 | 0.20±0.07 | 0.06 | | |
| 93 | 0.50±0.05 | 0.44±0.08 | 0.06 | | |
| 94 | 0.25±0.06 | 0.19±0.05 | 0.06 | | |
| 95 | 0.45±0.06 | 0.39±0.10 | 0.06 | | |
| 96 | 0.53±0.05 | 0.47±0.07 | 0.06 | | |
| 97 | 0.32±0.07 | 0.26±0.07 | 0.06 | | |
| 98 | 0.40±0.03 | 0.35±0.08 | 0.06 | | |
| 99 | 0.15±0.09 | 0.09±0.02 | 0.05 | | |
| 100 | 0.75±0.05 | 0.69±0.07 | 0.05 | | |
| 101 | 0.26±0.06 | 0.20±0.07 | 0.05 | | |
| 102 | 0.40±0.04 | 0.35±0.08 | 0.05 | | |
| 103 | 0.41±0.05 | 0.36±0.08 | 0.05 | | |
| 104 | 0.27±0.05 | 0.21±0.06 | 0.05 | | |
| 105 | 0.55±0.03 | 0.50±0.06 | 0.05 | | |

[Table 31]

| | Average β value (cancer rectal) n=20 | Average β value (non-cancerous rectal) n=28 | Δβ value | 52DMR | 15DMR |
|---|---|---|---|---|---|
| 106 | 0.30±0.06 | 0.25±0.07 | 0.05 | | |
| 107 | 0.34±0.05 | 0.29±0.07 | 0.05 | | |
| 108 | 0.52±0.05 | 0.47±0.08 | 0.05 | | |
| 109 | 0.32±0.04 | 027±0.08 | 0.05 | | |
| 110 | 0.44±0.04 | 0.39±0.08 | 0.05 | | |
| 111 | 0.68±0.04 | 0.73±0.04 | -0.05 | | |
| 112 | 0.49±0.06 | 0.54±0.05 | -0.05 | | |
| 113 | 0.59±0.05 | 0.65±0.03 | -0.05 | | |
| 114 | 0.60±0.04 | 0.65±0.02 | -0.05 | | |
| 115 | 0.60±0.05 | 0.65±0.03 | -0.05 | | |
| 116 | 0.61±0.03 | 0.66±0.03 | -0.05 | | |
| 117 | 0.66±0.03 | 0.72±0.02 | -0.06 | | |
| 118 | 0.61±0.04 | 0.67±0.04 | -0.06 | | |
| 119 | 0.68±0.12 | 0.74±0.12 | -0.06 | | |
| 120 | 0.74±0.07 | 0.80±0.03 | -0.06 | | |
| 121 | 0.72±0.07 | 0.78±0.06 | -0.07 | | |

Next, using the glm function of R software, 287,980 logistic regression models based on combinations of all three DMR's selected from the 121 DMR sets were created. Regarding the obtained discrimination expression, 47 discrimination expressions with sensitivity of higher than 95% and with three or more coefficients having a p value of less than 0.05 among four coefficients were selected, in which 52 DMR's appeared (52 DMR's in the tables). Furthermore, a frequency of DMR's appearing in the 47 discrimination expressions was obtained, and 15 DMR's appeared three times or more (15 DMR's, in the tables).

(2) Multivariate analysis of clinical samples using DMR biomarker candidates

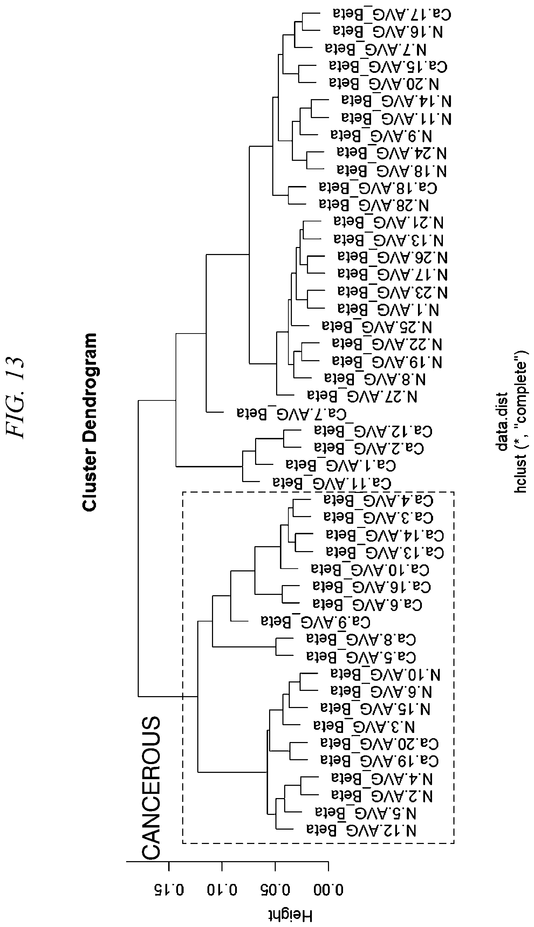

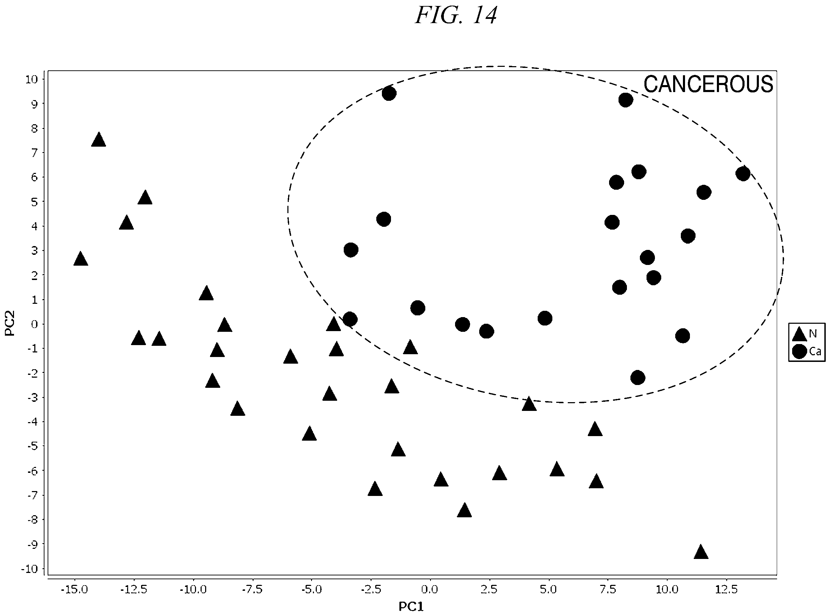

Cluster analysis and principal component analysis for all 48 samples of Example 2 were performed based on the methylation rates of the 121 DMR sets. As a result, in cluster analysis, a majority of colorectal cancer patient samples accumulated in the same cluster (within a frame, in FIG. 13). In addition, in the principal component analysis (FIG. 14), the colorectal cancer patient samples (●) and the healthy subject samples (▲) each formed independent clusters in a first principal component (horizontal axis) direction.

(3) Evaluation of the likelihood of sporadic colorectal cancer development in clinical samples using DMR biomarker candidates

Accuracy of determination of the presence or absence of sporadic colorectal cancer development was examined in a case where methylation rates in regions of DMR numbers 11, 24, and 42 among the 121 DMR sets are used as markers.

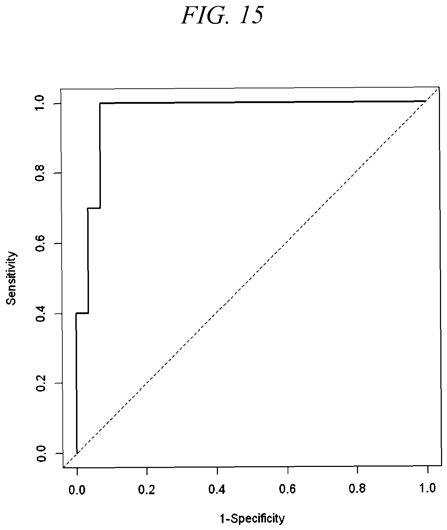

Specifically, based on a logistic regression model using numerical values (β values) of methylation levels of the three DMR's of specimens collected from the rectum of 20 colorectal cancer patients and 28 healthy subjects, a discrimination expression was created to discriminate between a colorectal cancer patient and a healthy subject. As a result, sensitivity (proportion of patients evaluated as positive among the colorectal cancer patients) was 100%, specificity (proportion of subjects evaluated as negative among the healthy subjects) was 92.9%, positive predictive value (proportion of colorectal cancer patients among those evaluated as positive) was 90.9%, and negative predictive value (proportion of healthy subjects among those evaluated as negative) was 100%, indicating that all were as high as 90% or more. FIG. 15 shows a ROC curve. As a result, an AUC (area under the ROC curve) was 0.968. From these results, it was confirmed that the likelihood of sporadic colorectal cancer development can be evaluated with high sensitivity and high specificity based on methylation rates of several DMR's selected from the 121 DMR sets.

Reference Signs List

1: kit for collecting large intestinal mucosa
2: collection tool
3a: first clamping piece
3b: second clamping piece
31, 31a, 31b: clamping portion
32, 32a, 32b: gripping portion
33, 33a, 33b: spring portion
34, 34a, 34b: fixing portion
35, 35a, 35b: clamping surface
11: collection auxiliary tool
12: collection tool introduction portion
13: slit
14: gripping portion
15: tip end side edge portion
16: proximal side edge portion

**Claims**

1. A method for determining the likelihood of sporadic colorectal cancer development, the method comprising:

a measurement step of measuring methylation rates of one or more CpG sites present in respective differentially methylated regions represented by differentially methylated region numbers 1 to 121 listed in Tables 1 to 7, in DNA recovered from a biological sample collected from a human subject; and
a determination step of determining the likelihood of sporadic colorectal cancer development in the human subject, based on average methylation rates of the differentially methylated regions which are calculated based on the methylation rates measured in the measurement step and a preset reference value or a preset multivariate discrimination expression,
wherein the average methylation rate of the differentially methylated region is an average value of methylation rates of all CpG sites, for which the methylation rate is measured in the measurement step, among the CpG sites in the differentially methylated region,
the reference value is a value for identifying a sporadic colorectal cancer patient and a non-sporadic colorectal cancer patient, which is set for the average methylation rate of each differentially methylated region, and
the multivariate discrimination expression includes, as variables, average methylation rates of one or more differentially methylated regions among the differentially methylated regions represented by the differentially methylated region numbers 1 to 121.

[Table 1]

| DMR no. | Gene Symbol | Ensembl ID | Chromosome no. | DMR start | DMR end | Width | +l |
|---|---|---|---|---|---|---|---|
| 1 | | | 17 | 46827397 | 46827628 | 232 | + |
| 2 | | ENST00000561259.1 | 15 | 37180595 | 37181182 | 588 | + |
| 3 | FADS2 | | 11 | 61596200 | 61596511 | 312 | + |
| 4 | SHF | ENST00000560734.1;ENST00000560471.1; ENST00000560540.1;ENST00000561091.1; ENST00000560034.1 | 15 | 45479648 | 45479861 | 214 | + |
| 5 | TDH | ENST00000525867.1;ENST00000534302.1 | 8 | 11203722 | 11205353 | 1632 | + |
| 6 | MYF6 | ENST00000228641.3 | 12 | 81102475 | 81103021 | 547 | + |
| 7 | SOX21; SOX21-AS1 | ENST00000438290.1; ENST00000376945.2 | 13 | 95364512 | 95364619 | 108 | + |
| 8 | RANBP9 | ENST00000469916.1 | 6 | 13633257 | 13635423 | 2167 | − |
| 9 | | ENST00000390750.1 | 1 | 97366188 | 97369696 | 3509 | − |
| 10 | EHBP1 | ENST00000516627.1 | 2 | 62953601 | 62956283 | 2683 | − |
| 11 | HECTD1 | ENST00000384709.1 | 14 | 31610929 | 31613066 | 2138 | − |
| 12 | | ENST00000440936.1 | 11 | 27911088 | 27914543 | 3456 | − |
| 13 | ASH1L | ENST00000384405.1 | 1 | 155327687 | 155330111 | 2425 | − |
| 14 | | ENST00000401135.1 | 11 | 112115998 | 112119870 | 3873 | − |
| 15 | | ENST00000562976.1 | 16 | 32609347 | 32612783 | 3437 | − |
| 16 | HOXA2 | ENST00000222718.5 | 7 | 27142503 | 27143294 | 792 | + |
| 17 | GNAL | ENST00000535121.1;ENST00000269162.4; ENST00000423027.2;ENST00000540217.1 | 18 | 11751996 | 11752178 | 183 | + |
| 18 | ARHGEF4 | ENST00000428230.2;ENST00000525839.1; ENST00000326016.5 | 2 | 131674106 | 131674191 | 86 | + |
| 19 | PCDHA7; PCDHA12; PCDHA6; PCDHAC1; PCDHA10; PCDHA4; PCDHA11; PCDHA8; PCDHA1; PCDHA2; PCDHA9; PCDHA13; PCDHA5; PCDHA3 | ENST00000253807.2; ENST00000409700.3 | 5 | 140306074 | 140306355 | 282 | + |
| 20 | FLJ45983 | ENST00000458727.1;ENST00000355358.1; ENST00000418270.1 | 10 | 8094324 | 8094640 | 317 | + |

[Table 2]

| DMR no. | Gene Symbol | Ensembl ID | Chromosome no. | DMR start | DMR end | Width | +ǀ |
|---|---|---|---|---|---|---|---|
| 21 | ATF7IP2 | ENST00000396559.1;ENST00000561932.1;ENST00000543967.1 | 16 | 10479725 | 10480582 | 858 | + |
| 22 | | | 11 | 20617680 | 20618294 | 615 | + |
| 23 | DMRTA2 | ENST00000418121.1 | 1 | 50886813 | 50887075 | 263 | + |
| 24 | SEPT9 | ENST00000363781.1;ENST00000397613.4 | 17 | 75436513 | 75439186 | 2674 | + |
| 25 | TNFRSF25; PLEKHG5 | ENST00000348333.3;ENST00000377782.3;ENST00000356876.3;ENST00000400913.1;ENST00000489097.1 | 1 | 6525942 | 6526668 | 727 | + |
| 26 | FLJ32063 | ENST00000450728.1;ENST00000416200.1;ENST00000446911.1;ENST00000457245.1;ENST00000441234.1 | 2 | 200334170 | 200335332 | 1163 | + |
| 27 | DTX1 | ENST00000257600.3 | 12 | 113494374 | 113494471 | 98 | + |
| 28 | LYNX1 | ENST00000522906.1;ENST00000398906.1;ENST00000395192.2;ENST00000335822.5;ENST00000523332.1;ENST00000345173.6 | 8 | 143858547 | 143858706 | 160 | + |
| 29 | IZUMO1 | ENST00000332955.2 | 19 | 49250305 | 49250694 | 390 | + |
| 30 | | | 18 | 55095061 | 55095364 | 304 | + |
| 31 | AEBP2 | ENST00000360995.4;ENST00000541908.1 | 12 | 19593346 | 19593565 | 220 | + |
| 32 | | ENST00000406197.1 | 7 | 155284154 | 155284741 | 588 | + |
| 33 | ZNF542 | ENST00000490123.1 | 19 | 56879271 | 56879751 | 481 | + |
| 34 | LRRC43 | | 12 | 122651566 | 122651863 | 298 | + |
| 35 | ERCC6 | ENST00000374129.3;ENST00000539110.1;ENST00000542458.1 | 10 | 50696150 | 50698147 | 1998 | — |
| 36 | ACSM3 | ENST00000289416.5;ENST00000440284.2;ENST00000565498.1 | 16 | 20777186 | 20779229 | 2044 | — |
| 37 | WAPAL | ENST00000372075.1;ENST00000263070.7 | 10 | 88226215 | 88229444 | 3230 | — |
| 38 | HLA-E | ENST00000376630.4 | 6 | 30455709 | 30456000 | 292 | — |
| 39 | | ENST00000459557.1 | 6 | 114159118 | 114163406 | 4289 | — |
| 40 | | ENST00000486767.1 | 3 | 164402447 | 164406668 | 4222 | — |

[Table 3]

| DMR no. | Gene Symbol | Ensembl ID | Chromosome no. | DMR start | DMR end | Width | ± |
|---|---|---|---|---|---|---|---|
| 41 | BET1 | ENST00000471446.1;ENST00000426193.2; ENST00000426634.1 | 7 | 93625930 | 93628057 | 2128 | − |
| 42 | | | 6 | 14406829 | 14409842 | 3014 | − |
| 43 | ZNF323; ZKSCAN3 | ENST00000252211.2;ENST00000341464.5; ENST00000396838.2;ENST00000414429.1 | 6 | 28320486 | 28323328 | 2843 | − |
| 44 | MTMR3 | ENST00000384724.1;ENST00000401950.2; ENST00000333027.3;ENST00000323630.5; ENST00000351488.3;ENST00000415511.1 | 22 | 30295038 | 30296772 | 1735 | − |
| 45 | SH3YL1 | ENST00000403657.1;ENST00000468321.1; ENST00000403658.1 | 2 | 252349 | 255227 | 2879 | − |
| 46 | | ENST00000455502.1 | 7 | 93472562 | 93475664 | 3103 | − |
| 47 | | ENST00000555070.1 | 14 | 90167165 | 90167752 | 588 | − |
| 48 | | | 8 | 1404844 | 1405431 | 588 | − |
| 49 | TFDP2 | ENST00000383877.1;ENST00000489671.1; ENST00000464782.1;ENST00000317104.7; ENST00000467072.1;ENST00000499676.2 | 3 | 141863017 | 141865101 | 2085 | − |
| 50 | TMEM106B | | 7 | 12268344 | 12270783 | 2440 | − |
| 51 | | ENST00000364882.1 | 4 | 117758275 | 117761934 | 3660 | − |
| 52 | SLC20A2 | ENST00000520262.1;ENST00000520179.1; ENST00000342228.3 | 8 | 42357666 | 42360957 | 3292 | − |
| 53 | | | 1 | 47910065 | 47911801 | 1737 | + |
| 54 | STK32B | ENST00000282908.5 | 4 | 5053444 | 5053551 | 108 | + |
| 55 | SOX2OT; SOX2 | ENST00000498731.1;ENST00000431565.2; ENST00000325404.1 | 3 | 181427354 | 181428928 | 1575 | + |
| 56 | SOX2OT | ENST00000498731.1 | 3 | 181437890 | 181438559 | 670 | + |
| 57 | CLIP4 | ENST00000320081.5;ENST00000379543.5; ENST00000401605.1;ENST00000401617.2; ENST00000404424.1 | 2 | 29337848 | 29338142 | 295 | + |

[Table 4]

| DMR no. | Gene Symbol | Ensembl ID | Chromosome no. | DMR start | DMR end | Width | + |
|---|---|---|---|---|---|---|---|
| 58 | | | 5 | 2038695 | 2039282 | 588 | + |
| 59 | SHISA9 | ENST00000423335.2;ENST00000482916.1;ENST00000558318.1;ENST00000424107.3 | 16 | 12995279 | 12995656 | 378 | + |
| 60 | | ENST00000364275.1 | 4 | 190938593 | 190938935 | 343 | + |
| 61 | | | 16 | 73096548 | 73097135 | 588 | + |
| 62 | TTYH1 | ENST00000391739.3;ENST00000376531.3;ENST00000301194.4;ENST00000376530.3 | 19 | 54926333 | 54927197 | 865 | + |
| 63 | PHACTR1 | ENST00000379350.1;ENST00000399446.2;ENST00000334971.6 | 6 | 13273152 | 13275352 | 2201 | + |
| 64 | DAB1 | ENST00000371236.1;ENST00000371234.4;ENST00000485760.1 | 1 | 58715419 | 58715632 | 214 | + |
| 65 | | ENST00000558382.1;ENST00000558499.1 | 15 | 96905928 | 96910011 | 4084 | + |
| 66 | ZNF382;ZNF529 | ENST00000423582.1;ENST00000460670.1;ENST00000292928.2;ENST00000439428.1 | 19 | 37096052 | 37096201 | 150 | + |
| 67 | SOX2OT;SOX2-OT | ENST00000498731.1 | 3 | 181440653 | 181444202 | 3550 | + |
| 68 | CPEB1;CPEB1-AS1 | ENST00000560650.1;ENST00000450751.2;ENST00000568757.1;ENST00000563519.1 | 15 | 83316116 | 83316484 | 369 | + |
| 69 | EVC2 | ENST00000344938.1;ENST00000310917.2 | 4 | 5710239 | 5710490 | 252 | + |
| 70 | C2orf74 | ENST00000426997.1;ENST00000420918.1 | 2 | 61372150 | 61372361 | 212 | + |
| 71 | DPYSL3 | ENST00000343218.5;ENST00000504965.1 | 5 | 146889149 | 146889390 | 242 | + |
| 72 | PENK;LOC101929415 | ENST00000518662.1;ENST00000523274.1;ENST00000523051.1;ENST00000518770.1;ENST00000539312.1;ENST00000451791.2;ENST00000314922.3 | 8 | 57358624 | 57358800 | 177 | + |

[Table 5]

| DMR no. | Gene Symbol | Ensembl ID | Chromosome no. | DMR start | DMR end | Width | +I |
|---|---|---|---|---|---|---|---|
| 73 | GJD2; LOC101928174 | ENST00000503496.1;ENST00000290374.4 | 15 | 35047146 | 35047453 | 308 | + |
| 74 | ADAMTS16 | ENST00000512155.1;ENST00000511368.1 | 5 | 5139810 | 5139920 | 111 | + |
| 75 | FAM159B | ENST00000512767.1 | 5 | 63986626 | 63986899 | 274 | + |
| 76 | KCNA4 | ENST00000526518.1;ENST00000328224.6 | 11 | 30038649 | 30038734 | 86 | + |
| 77 | IRX5 | ENST00000447390.2;ENST00000560487.1; ENST00000560154.1;ENST00000558597.1; ENST00000394636.4 | 16 | 54967579 | 54969439 | 1861 | + |
| 78 | BCAT1 | ENST00000538118.1;ENST00000544418.1; ENST00000539282.1 | 12 | 25055964 | 25056233 | 270 | + |
| 79 | SOX11 | ENST00000322002.3;ENST00000455579.1 | 2 | 5836177 | 5836284 | 108 | + |
| 80 | CHL1 | ENST00000452919.1;ENST00000444879.1; ENST00000489224.1;ENST00000256509.2; ENST00000397491.2 | 3 | 239108 | 239308 | 201 | + |
| 81 | FAM115A; TCAF1 | ENST00000392900.3;ENST00000355951.2; ENST00000479870.1 | 7 | 143578766 | 143581048 | 2283 | + |
| 82 | | ENST00000551875.1 | 12 | 115172454 | 115173299 | 846 | + |
| 83 | | | 17 | 46831196 | 46831783 | 588 | + |
| 84 | NR5A2 | | 1 | 200003863 | 200004690 | 828 | + |
| 85 | UTF1 | ENST00000304477.2 | 10 | 135043449 | 135043550 | 102 | + |
| 86 | ATP10A | ENST00000553577.1;ENST00000356865.6 | 15 | 26107150 | 26108725 | 1576 | + |
| 87 | LOC283999; TMEM235 | ENST00000374946.3;ENST00000550981.2 | 17 | 76227764 | 76228227 | 464 | + |
| 88 | ZNF177 | ENST00000343499.3;ENST00000541595.1; ENST00000446085.2 | 19 | 9473642 | 9473768 | 127 | + |
| 89 | | | 6 | 107809023 | 107809834 | 812 | + |
| 90 | NR2E1 | ENST00000368986.4 | 6 | 108492410 | 108493000 | 591 | + |
| 91 | CDO1 | ENST00000250535.4;ENST00000502631.1 | 5 | 115152332 | 115152439 | 108 | + |
| 92 | CASR | ENST00000498619.1;ENST00000490131.1 | 3 | 121902936 | 121903190 | 255 | + |

[Table 6]

| DMR no. | Gene Symbol | Ensembl ID | Chromosome no. | DMR start | DMR end | Width | ± |
|---|---|---|---|---|---|---|---|
| 93 | PCDHGA4; PCDHGA11; PCDHGA9; PCDHGA1; PCDHGB1; PCDHGB6; PCDHGA12; PCDHGB3; PCDHGB7; PCDHGA6; PCDHGA8; PCDHGA10; PCDHGA5; PCDHGB4; PCDHGA3; PCDHGA2; PCDHGB2; PCDHGA7; PCDHGB5 | ENST00000252085.3 | 5 | 140809819 | 140810664 | 846 | + |
| 94 | OCA2 | ENST00000353809.5;ENST00000354638.3 | 15 | 28344617 | 28344827 | 211 | + |
| 95 | LINC01248; SOX11 | ENST00000420221.1;ENST00000453678.1; ENST00000458264.1;ENST00000322002.3 | 2 | 5830853 | 5831440 | 588 | + |
| 96 | GDF7 | ENST00000272224.3 | 2 | 20871066 | 20871694 | 629 | + |
| 97 | SOX8 | ENST00000562570.1;ENST00000568394.1; ENST00000565467.1;ENST00000563863.1; ENST00000565069.1;ENST00000563837.1; ENST00000293894.3 | 16 | 1030543 | 1030628 | 86 | + |
| 98 | NEFM | ENST00000221166.5;ENST00000433454.2; ENST00000518131.1;ENST00000521540.1 | 8 | 24771213 | 24771326 | 114 | + |
| 99 | | ENST00000560487.1 | 16 | 54970835 | 54971133 | 299 | + |
| 100 | PTGFRN | ENST00000544471.1;ENST00000393203.2 | 1 | 117528415 | 117531212 | 2798 | + |
| 101 | STAC | ENST00000273183.3;ENST00000457375.2; ENST00000476388.1;ENST00000544687.1 | 3 | 36422165 | 36422637 | 473 | + |
| 102 | | | 12 | 81106709 | 81109314 | 2606 | + |
| 103 | HBQ1 | ENST00000199708.2 | 16 | 230287 | 230396 | 110 | + |
| 104 | | | 6 | 85484569 | 85485156 | 588 | + |

[Table 7]

| DMR no. | Gene Symbol | Ensembl ID | Chromosome no. | DMR start | DMR end | Width | +l |
|---|---|---|---|---|---|---|---|
| 105 | NPR3 | ENST00000434067.2;ENST00000415685.2 | 5 | 32708777 | 32709689 | 913 | + |
| 106 | NMBR | ENST00000258042.1;ENST00000454401.1 | 6 | 142410081 | 142410276 | 196 | + |
| 107 | KCNIP1 | ENST00000411494.1;ENST00000328939.4; ENST00000390656.4;ENST00000520740.1 | 5 | 169931309 | 169931416 | 108 | + |
| 108 | ZNF835 | ENST00000537055.1 | 19 | 57183011 | 57183374 | 364 | + |
| 109 | SALL3 | ENST00000575722.1;ENST00000573860.1; ENST00000537592.2 | 18 | 76740075 | 76740337 | 263 | + |
| 110 | CCNA1 | ENST00000418263.1;ENST00000255465.4; ENST00000440264.1 | 13 | 37006053 | 37006793 | 741 | + |
| 111 | NR3C1 | ENST00000504336.1;ENST00000416954.2 | 5 | 142768792 | 142771780 | 2989 | — |
| 112 | STX19; ARL13B | ENST00000315099.2;ENST00000539730.1; ENST00000486562.1 | 3 | 93746411 | 93748870 | 2460 | — |
| 113 | NFIB | ENST00000493697.1 | 9 | 14307151 | 14309148 | 1998 | — |
| 114 | | ENST00000510419.1 | 4 | 75513579 | 75517080 | 3502 | — |
| 115 | TRIM9 | ENST00000554475.1 | 14 | 51554159 | 51556518 | 2360 | — |
| 116 | PIBF1 | ENST00000362511.1 | 13 | 73455494 | 73457491 | 1998 | — |
| 117 | | ENST00000468232.1 | 3 | 170126475 | 170129488 | 3014 | — |
| 118 | LOC101060498 | ENST00000510551.1 | 4 | 40316101 | 40318304 | 2204 | — |
| 119 | RNU6-2 | ENST00000384716.1 | 10 | 13257430 | 13260736 | 3307 | — |
| 120 | EFNB2 | | 13 | 107181847 | 107183783 | 1937 | — |
| 121 | ARG1 | ENST00000368087.3;ENST00000356962.2; ENST00000476845.1;ENST00000489091.1 | 6 | 131893339 | 131893636 | 298 | — |

2. The method for determining the likelihood of sporadic colorectal cancer development according to Claim 1, wherein in the measurement step, in a case where one or more among the differentially methylated regions represented by differentially methylated region numbers 8 to 15, 35 to 52, and 111 to 121 have an average methylation rate of equal to or lower than the preset reference value, or one or more among the differentially methylated regions represented by differentially methylated region numbers 1 to 7, 16 to 34, and 53 to 110 have an average methylation rate of equal to or higher than the preset reference value, it is determined that there is a high likelihood of sporadic colorectal cancer development in the human subject.

3. The method for determining the likelihood of sporadic colorectal cancer development according to Claim 1, wherein in the measurement step, the methylation rates of the one or more CpG sites present in the differentially methylated region, of which an average methylation rate is included as a variable in the multivariate discrimination expression, are measured, and
in the determination step, in a case where based on the average methylation rate of the differentially methylated region calculated based on the methylation rates measured in the measurement step, and the multivariate discrimination expression, a discrimination value which is a value of the multivariate discrimination expression is calculated, and the discrimination value is equal to or higher than a preset reference discrimination value, it is determined that there is a high likelihood of sporadic colorectal cancer development in the human subject.

4. The method for determining the likelihood of sporadic colorectal cancer development according to Claim 3, wherein the multivariate discrimination expression includes, as variables, average methylation rates of two or more differentially methylated regions selected from the differentially methylated regions represented by the differentially methylated region numbers 1 to 121.

5. The method for determining the likelihood of sporadic colorectal cancer development according to Claim 3, wherein the multivariate discrimination expression includes, as variables, average methylation rates of three or more differentially methylated regions selected from the differentially methylated regions represented by the differentially methylated region numbers 1 to 121.

6. The method for determining the likelihood of sporadic colorectal cancer development according to Claim 3, wherein the multivariate discrimination expression includes, as variables, average methylation rates of one or more differentially methylated regions selected from the group consisting of the differentially methylated regions represented by the differentially methylated region numbers 1 to 52.

7. The method for determining the likelihood of sporadic colorectal cancer development according to Claim 3, wherein the multivariate discrimination expression includes, as variables, average methylation rates of one or more differentially methylated regions selected from the group consisting of the differentially methylated regions represented by the differentially methylated region numbers 1 to 15.

8. A method for determining the likelihood of sporadic colorectal cancer development, the method comprising:

a measurement step of measuring methylation rates of one or more CpG sites selected from the group consisting of CpG sites in base sequences represented by SEQ ID NOs: 1 to 93, in DNA recovered from a biological sample collected from a human subject; and
a determination step of determining the likelihood of sporadic colorectal cancer development in the human subject, based on the methylation rates measured in the measurement step and a preset reference value or a preset multivariate discrimination expression,
wherein the reference value is a value for identifying a sporadic colorectal cancer patient and a non-sporadic colorectal cancer patient, which is set for the methylation rate of each CpG site, and
the multivariate discrimination expression includes, as variables, methylation rates of one or more CpG sites among the CpG sites in the base sequences represented by SEQ ID NOs: 1 to 93.

9. The method for determining the likelihood of sporadic colorectal cancer development according to Claim 8, wherein in the measurement step, methylation rates of 2 to 10 CpG sites are measured.

10. The method for determining the likelihood of sporadic colorectal cancer development according to Claim 8 or 9, wherein in the determination step, in a case where at least one among CpG sites in the base sequences represented by SEQ ID NOs: 1, 4, 6, 10, 11, 13, 14, 17 to 20, 23 to 27, 29, 30, 32, 33, 35, 36, 39, 41 to 48, 50 to 54, 59, 65 to 68, 70 to 77, 79 to 86, 90, and 91 has a methylation rate of equal to or lower than the preset reference value, or at least one among CpG sites in the base sequences represented by SEQ ID NOs: 2, 3, 5, 7 to 9, 12, 15, 16, 21, 22, 28, 31, 34, 37, 38, 40, 49, 55 to 58, 60 to 64, 69, 78, 87 to 89, 92, and 93 has a methylation rate of equal to or higher than the preset reference value, it is determined that there is a high likelihood of sporadic colorectal cancer development in the human subject.

11. The method for determining the likelihood of sporadic colorectal cancer development according to any one of Claims 8 to 10,
wherein in the measurement step, methylation rates of CpG sites in the base sequences represented by SEQ ID NOs: 1 to 54 are measured, and
in the determination step, in a case where at least one among CpG sites in the base sequences represented by SEQ ID NOs: 1, 4, 6, 10, 11, 13, 14, 17 to 20, 23 to 27, 29, 30, 32, 33, 35, 36, 39, 41 to 48, and 50 to 54 has a methylation rate of equal to or lower than the preset reference value, or at least one among CpG sites in the base sequences represented by SEQ ID NOs: 2, 3, 5, 7 to 9, 12, 15, 16, 21, 22, 28, 31, 34, 37, 38, 40, and 49 has a methylation rate of equal to or higher than the preset reference value, it is determined that there is a high likelihood of sporadic colorectal cancer development in the human subject.

12. The method for determining the likelihood of sporadic colorectal cancer development according to any one of Claims 8 to 11,
wherein in the determination step, in a case where a sum of the number of CpG sites having a methylation rate equal to or lower than the preset reference value among CpG sites in the base sequences represented by SEQ ID NOs: 1, 4, 6, 10, 11, 13, 14, 17 to 20, 23 to 27, 29, 30, 32, 33, 35, 36, 39, 41 to 48, and 50 to 54, and the number of CpG sites having a methylation rate equal to or higher than the preset reference value among CpG sites in the base sequences represented by SEQ ID NOs: 2, 3, 5, 7 to 9, 12, 15, 16, 21, 22, 28, 31, 34, 37, 38, 40, and 49 is three or more, it is determined that there is a high likelihood of sporadic colorectal cancer development in the human subject.

13. The method for determining the likelihood of sporadic colorectal cancer development according to any one of Claims 8 to 10,
wherein in the measurement step, methylation rates of CpG sites in the base sequences represented by SEQ ID NOs: 1 to 8 are measured, and
in the determination step, in a case where at least one among CpG sites in the base sequences represented by SEQ ID NOs: 1, 4, and 6 has a methylation rate of equal to or lower than the preset reference value, or at least one among CpG sites in the base sequences represented by SEQ ID NOs: 2, 3, 5, 7, and 8 has a methylation rate of equal to or higher than the preset reference value, it is determined that there is a high likelihood of sporadic colorectal cancer development in the human subject.

14. The method for determining the likelihood of sporadic colorectal cancer development according to any one of Claims 8 to 10, and 13,
wherein in the determination step, in a case where a sum of the number of CpG sites having a methylation rate equal to or lower than the preset reference value among CpG sites in the base sequences represented by SEQ ID NOs: 1, 4, and 6, and the number of CpG sites having a methylation rate equal to or higher than the preset reference value among CpG sites in the base sequences represented by SEQ ID NOs: 2, 3, 5, 7, and 8 is three or more, it is determined that there is a high likelihood of sporadic colorectal cancer development in the human subject.

15. The method for determining the likelihood of colorectal cancer development according to any one of Claims 8 to 10,
wherein in the measurement step, methylation rates of CpG sites in the base sequences represented by SEQ ID NOs: 55 to 87 are measured, and
in the determination step, in a case where at least one among CpG sites in the base sequences represented by SEQ ID NOs: 59, 65 to 68, 70 to 77, and 79 to 86 has a methylation rate of equal to or lower than the preset reference value, or at least one among CpG sites in the base sequences represented by SEQ ID NOs: 55 to 58, 60 to 64, 69, 78, and 87 has a methylation rate of equal to or higher than the preset reference value, it is determined that there is a high likelihood of sporadic colorectal cancer development in the human subject.

16. The method for determining the likelihood of sporadic colorectal cancer development according to any one of Claims 8 to 10, and 15,
wherein in the determination step, in a case where a sum of the number of CpG sites having a methylation rate equal to or lower than the preset reference value among CpG sites in the base sequences represented by SEQ ID NOs: 59, 65 to 68, 70 to 77, and 79 to 86, and the number of CpG sites having a methylation rate equal to or higher than the preset reference value among CpG sites in the base sequences represented by SEQ ID NOs: 55 to 58, 60 to 64, 69, 78, and 87 is two or more, it is determined that there is a high likelihood of sporadic colorectal cancer development in the human subject.

17. The method for determining the likelihood of sporadic colorectal cancer development according to any one of Claims 8 to 10,
wherein in the measurement step, methylation rates of CpG sites in the base sequences represented by SEQ ID NOs: 88 to 93 are measured, and
in the determination step, in a case where at least one among CpG sites in the base sequences represented by SEQ ID NOs: 90 and 91 has a methylation rate of equal to or lower than the preset reference value, or at least one among CpG sites in the base sequences represented by SEQ ID NOs: 88, 89, 92, and 93 has a methylation rate of equal to or higher than the preset reference value, it is determined that there is a high likelihood of sporadic colorectal cancer development in the human subject.

18. The method for determining the likelihood of sporadic colorectal cancer development according to any one of Claims 8 to 10, and 17,
wherein in the determination step, in a case where a sum of the number of CpG sites having a methylation rate equal to or lower than the preset reference value among CpG sites in the base sequences represented by SEQ ID NOs: 90 and 91, and the number of CpG sites having a methylation rate equal to or higher than the preset reference value among CpG sites in the base sequences represented by SEQ ID NOs: 88, 89, 92, and 93 is two or more, it is determined that there is a high likelihood of sporadic colorectal cancer development in the human subject.

19. The method for determining the likelihood of sporadic colorectal cancer development according to Claim 12, 14, 16, or 18,
wherein in a case where the sum is five or more, it is determined that there is a high likelihood of sporadic colorectal cancer development in the human subject.

20. The method for determining the likelihood of sporadic colorectal cancer development according to Claim 8 or 9, wherein the multivariate discrimination expression includes, as variables, methylation rates of one or more CpG sites selected from the group consisting of CpG sites in the base sequences represented by SEQ ID NOs: 55 to 87, in the measurement step, a methylation rate of the CpG site which is included as a variable in the multivariate discrimination expression is measured, and

in the determination step, in a case where based on the methylation rate measured in the measurement step, and the multivariate discrimination expression, a discrimination value which is a value of the multivariate discrimination expression is calculated, and the discrimination value is equal to or higher than a preset reference discrimination value, it is determined that there is a high likelihood of colorectal cancer development in the human subject.

21. The method for determining the likelihood of sporadic colorectal cancer development according to Claim 8 or 9, wherein the multivariate discrimination expression includes, as variables, methylation rates of one or more CpG sites selected from the group consisting of CpG sites in the base sequences represented by SEQ ID NOs: 88 to 93, in the measurement step, a methylation rate of the CpG site which is included as a variable in the multivariate discrimination expression is measured, and

in the determination step, in a case where based on the methylation rate measured in the measurement step, and the multivariate discrimination expression, a discrimination value which is a value of the multivariate discrimination expression is calculated, and the discrimination value is equal to or higher than a preset reference discrimination value, it is determined that there is a high likelihood of sporadic colorectal cancer development in the human subject.

22. The method for determining the likelihood of sporadic colorectal cancer development according to any one of Claims 8 to 21,
wherein the multivariate discrimination expression is a logistic regression expression, a linear discrimination expression, an expression created by Naive Bayes classifier, or an expression created by Support Vector Machine.

23. The method for determining the likelihood of sporadic colorectal cancer development according to any one of Claims 8 to 22,
wherein the biological sample is intestinal tract tissue.

24. The method for determining the likelihood of sporadic colorectal cancer development according to any one of Claims 8 to 23,
wherein the biological sample is rectal mucosal tissue.

25. The method for determining the likelihood of sporadic colorectal cancer development according to Claim 24, wherein the rectal mucosal tissue is collected by a kit for collecting large intestinal mucosa which includes a collection tool and a collection auxiliary tool,
the collection tool includes a first clamping piece and a second clamping piece which are a pair of plate-like bodies, each of the first clamping piece and the second clamping piece is configured to have a clamping portion, a gripping portion, a spring portion, and a fixing portion, and
the collection auxiliary tool has

a truncated cone-shaped collection tool introduction portion having a slit on a side wall, and
a rod-like gripping portion,

one end of the gripping portion is connected in the vicinity of a side edge portion having a larger outer diameter of the collection tool introduction portion,
the slit is provided from a side edge portion having a smaller outer diameter of the collection tool introduction portion toward the side edge portion having a larger outer diameter,
a width of the slit is wider than a width in a state in which the first clamping piece and the second clamping piece are bonded to each other at end portions on a side of the clamping portions, and
the collection tool introduction portion has a larger outer diameter of 30 to 70 mm and a length in a rotation axis direction of 50 to 150 mm.

26. The method for determining the likelihood of sporadic colorectal cancer development according to Claim 25,

wherein a recess is provided on at least one of an end portion of a surface, in the clamping portion of the first clamping piece, opposed to the second clamping piece, and an end portion of a surface, in the clamping portion of the second clamping piece, opposed to the first clamping piece.

27. A kit for collecting large intestinal mucosa, comprising:

a collection tool; and
a collection auxiliary tool,
wherein the collection tool includes
a first clamping piece and a second clamping piece which are a pair of plate-like bodies,
each of the first clamping piece and the second clamping piece is configured to have a clamping portion, a gripping portion, a spring portion, and a fixing portion, and
the collection auxiliary tool has

a truncated cone-shaped collection tool introduction portion having a slit on a side wall, and
a rod-like gripping portion,

one end of the gripping portion is connected in the vicinity of a side edge portion having a larger outer diameter of the collection tool introduction portion,
the slit is provided from a side edge portion having a smaller outer diameter of the collection tool introduction portion toward the side edge portion having a larger outer diameter,
a width of the slit is wider than a width in a state in which the first clamping piece and the second clamping piece are bonded to each other at end portions on a side of the clamping portions, and
the collection tool introduction portion has a larger outer diameter of 30 to 70 mm and a length in a rotation axis direction of 50 to 150 mm.

28. The kit for collecting large intestinal mucosa according to Claim 27, wherein a recess is provided on at least one of an end portion of a surface, in the clamping portion of the first clamping piece, opposed to the second clamping piece, and an end portion of a surface, in the clamping portion of the second clamping piece, opposed to the first clamping piece.

29. A marker for analyzing a DNA methylation rate, comprising:

a DNA fragment having a partial base sequence containing one or more CpG sites selected from the group consisting of CpG sites in base sequences represented by SEQ ID NOs: 1 to 93,
wherein the marker is used to determine the likelihood of sporadic colorectal cancer development in a human subject.

*FIG. 1*

(A)

2

3

4

35

31

32

33

34

(B)

2

3b

4

35b

35a

31

32

33

34

3a

(C)

4

3b

2

34

33

3a

32

35b

35a

31

*FIG. 2*

*FIG. 3*

FIG. 4

54 CpG

**Cluster Dendrogram**

CANCEROUS

data.dist
hclust (*, "complete")

*FIG. 5*

*FIG. 6*

54 CpG

*FIG. 7*

8 CpG

*FIG. 8*

## 33 CpG

**Cluster Dendrogram**

CANCEROUS

data.dist
hclust (*, "complete")

*FIG. 9*

*FIG. 10*

*FIG. 11*

6 CpG

**Cluster Dendrogram**

data.dist
hclust (*, "complete")

*FIG. 12*

6 CpG

FIG. 13

FIG. 14

*FIG. 15*

EP 3 521 448 A1

INTERNATIONAL SEARCH REPORT

International application No.

PCT/JP2017/035137

A.  CLASSIFICATION OF SUBJECT MATTER
Int.Cl.  C12Q1/68(2006.01)i,  C12N15/09(2006.01)n

According to International Patent Classification (IPC) or to both national classification and IPC

B.  FIELDS SEARCHED

Minimum documentation searched (classification system followed by classification symbols)
Int.Cl.  C12Q1/68, C12N15/09

Documentation searched other than minimum documentation to the extent that such documents are included in the fields searched
Published examined utility model applications of Japan          1922-1996
Published unexamined utility model applications of Japan        1971-2017
Registered utility model specifications of Japan                1996-2017
Published registered utility model applications of Japan        1994-2017

Electronic data base consulted during the international search (name of data base and, where practicable, search terms used)
CAplus/MEDLINE/EMBASE/BIOSIS(STN)  JSTPlus/JMEDPlus/JST7580(JDreamIII)
GenBank/EMBL/DDBJ/GeneSeq

C.  DOCUMENTS CONSIDERED TO BE RELEVANT

| Category* | Citation of document, with indication, where appropriate, of the relevant passages | Relevant to claim No. |
|---|---|---|
| Y | GONZALO, V. et al., Multiple sporadic colorectal cancers display a unique methylation phenotype., PLoS One, 2014, vol. 9, no. 3, p. e91033, DOI: 10.1371/journal.pone.0091033, abstract | 1-26, 29 |
| Y | GOEL, A. et al., The CpG island methylator phenotype and chromosomal instability are inversely correlated in sporadic, Gastroenterology, 2007, vol. 132, pp. 127-138, abstract, DOI : 10.1053/j.gastro.2006.09.18 | 1-26, 29 |

☒   Further documents are listed in the continuation of Box C.          ☐   See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
08 December 2017

Date of mailing of the international search report
19 December 2017

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

**INTERNATIONAL SEARCH REPORT**

International application No.

PCT/JP2017/035137

C (Continuation).　DOCUMENTS CONSIDERED TO BE RELEVANT

| Category* | Citation of document, with indication, where appropriate, of the relevant passages | Relevant to claim No. |
|---|---|---|
| Y | WO 2005/021743 A1 (MATSUBARA, Nagahide et al.) 10 March 2005, claims<br>& CN 1845991 A | 1-26, 29 |
| Y | JP 2015-006164 A (SYSMEX CORPORATION) 15 January 2015, claims<br>& US 2014/0356866 A1, claims<br>& EP 2808397 A2 | 1-26, 29 |
| Y | KIBRIYA, M. G. et al., A genome-wide DNA methylation study in colorectal carcinoma, BMC Medical Genomics, 2011, 4:50, pp. 1-16, abstract, DOI: 10.1186/1755-8794-4-50 | 1-26, 29 |
| Y | JP 2003-235799 A (OKUMURA, Yukiyasu) 26 August 2003, fig. 3, paragraph [0013] (Family: none) | 25-28 |
| Y | JP 2012-090919 A (NAKAMURA, Shouichi) 17 May 2012, fig. 1, claim 1<br>& US 2012/0303049 A1, claim 1, fig. 1<br>& EP 2537476 A1 | 25-28 |
| Y | JP 2009-119219 A (KOHASE, Momoe et al.) 24 June 2009, fig. 1, claim 1 (Family: none) | 25-28 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016078810 W
- JP 2017072674 A

- WO 2014151551 A

**Non-patent literature cited in the description**

- *Cellular and Molecular Life Sciences,* 2014, vol. 71 (18), 3523-3535
- *Cancer Letters,* 2014, vol. 345, 235-241
- *BMC Med genomics,* 2011, vol. 4, 50

- *Sex Dev,* 2011, vol. 5, 70
- *Bioinformatics,* 2014, vol. 30, 428, http://bioconductor.org/packages/release/bioc/html/ChAMP.html